# EUROPEAN PATENT APPLICATION

(11) **EP 3 505 618 A1**
(43) Date of publication of application: **03.07.2019**
(21) Application number: 17864886.1
(22) Date of filing: 25.10.2017
(51) Int. Cl.: C12N 1/21, C12P 5/00, C12N 15/09

(54) **RECOMBINANT CELLS AND METHOD FOR PRODUCING ISOPRENE OR TERPENE**

(30) Priority: 28.10.2016 JP 2016211836
(71) Applicant: Sekisui Chemical Co., Ltd., Osaka-shi, Osaka 530-8565 (JP)
(72) Inventor: FURUTANI Masahiro, Tsukuba-shi Ibaraki 300-4292 (JP); MATSUSHIMA Kana, Tsukuba-shi Ibaraki 300-4292 (JP); KAWABATA Kazufumi, Tsukuba-shi Ibaraki 300-4292 (JP)
(74) Representative: Ter Meer Steinmeister & Partner
(86) International application number: PCT/JP2017/038551
(87) International publication number: WO 2018/079619

(57) **Abstract**

To provide a recombinant cell being an anaerobic archaeon, including a gene encoding isoprene synthase, a gene encoding monoterpene synthase, a gene encoding sesquiterpene synthase, a gene encoding diterpene synthase, a gene encoding squalene synthase, or a gene encoding phytoene synthase as a first foreign gene, wherein the first foreign gene is expressed, and the recombinant cell is capable of producing isoprene or terpene having 10, 15, 20, 30, or 40 carbon atoms.

SEQUENCE LISTING

<110>  Sekisui Chemical Co., Ltd.

<120>

<130>  17P01130

<150>  JP 2016-211836
<151>  2016-10-28

<160>  16

<170>  PatentIn version 3.1

<210>  1
<211>  595
<212>  PRT
<213>  Populus canescens

<400>  1

Met Ala Thr Glu Leu Leu Cys Leu His Arg Pro Ile Ser Leu Thr His
1               5                   10                  15

Lys Leu Phe Arg Asn Pro Leu Pro Lys Val Ile Gln Ala Thr Pro Leu
            20                  25                  30

Thr Leu Lys Leu Arg Cys Ser Val Ser Thr Glu Asn Val Ser Phe Thr
        35                  40                  45

Glu Thr Glu Thr Glu Ala Arg Arg Ser Ala Asn Tyr Glu Pro Asn Ser
    50                  55                  60

Trp Asp Tyr Asp Phe Leu Leu Ser Ser Asp Thr Asp Glu Ser Ile Glu
65                  70                  75                  80

Val Tyr Lys Asp Lys Ala Lys Lys Leu Glu Ala Glu Val Arg Arg Glu
                85                  90                  95

Ile Asn Asn Glu Lys Ala Glu Phe Leu Thr Leu Leu Glu Leu Ile Asp
            100                 105                 110

Asn Val Gln Arg Leu Gly Leu Gly Tyr Arg Phe Glu Ser Asp Ile Arg
        115                 120                 125

Arg Ala Leu Asp Arg Phe Val Ser Ser Gly Gly Phe Asp Gly Val Thr
    130                 135                 140

Lys Thr Ser Leu His Ala Thr Ala Leu Ser Phe Arg Leu Leu Arg Gln
145                 150                 155                 160

His Gly Phe Glu Val Ser Gln Glu Ala Phe Ser Gly Phe Lys Asp Gln

                165                 170                 175

Asn Gly Asn Phe Leu Glu Asn Leu Lys Glu Asp Thr Lys Ala Ile Leu
            180                 185                 190

Ser Leu Tyr Glu Ala Ser Phe Leu Ala Leu Glu Gly Glu Asn Ile Leu
        195                 200                 205

Asp Glu Ala Arg Val Phe Ala Ile Ser His Leu Lys Glu Leu Ser Glu
    210                 215                 220

Glu Lys Ile Gly Lys Glu Leu Ala Glu Gln Val Asn His Ala Leu Glu
225                 230                 235                 240

Leu Pro Leu His Arg Arg Thr Gln Arg Leu Glu Ala Val Trp Ser Ile
                245                 250                 255

Glu Ala Tyr Arg Lys Lys Glu Asp Ala Asn Gln Val Leu Leu Glu Leu
            260                 265                 270

Ala Ile Leu Asp Tyr Asn Met Ile Gln Ser Val Tyr Gln Arg Asp Leu
        275                 280                 285

Arg Glu Thr Ser Arg Trp Trp Arg Arg Val Gly Leu Ala Thr Lys Leu
    290                 295                 300

His Phe Ala Lys Asp Arg Leu Ile Glu Ser Phe Tyr Trp Ala Val Gly
305                 310                 315                 320

Val Ala Phe Glu Pro Gln Tyr Ser Asp Cys Arg Asn Ser Val Ala Lys
                325                 330                 335

Met Phe Ser Phe Val Thr Ile Ile Asp Asp Ile Tyr Asp Val Tyr Gly
            340                 345                 350

Thr Leu Asp Glu Leu Glu Leu Phe Thr Asp Ala Val Glu Arg Trp Asp
        355                 360                 365

Val Asn Ala Ile Asn Asp Leu Pro Asp Tyr Met Lys Leu Cys Phe Leu
    370                 375                 380

Ala Leu Tyr Asn Thr Ile Asn Glu Ile Ala Tyr Asp Asn Leu Lys Asp
385                 390                 395                 400

Lys Gly Glu Asn Ile Leu Pro Tyr Leu Thr Lys Ala Trp Ala Asp Leu
                405                 410                 415

Cys Asn Ala Phe Leu Gln Glu Ala Lys Trp Leu Tyr Asn Lys Ser Thr
            420                 425                 430

Pro Thr Phe Asp Asp Tyr Phe Gly Asn Ala Trp Lys Ser Ser Ser Gly
        435                 440                 445

Pro Leu Gln Leu Ile Phe Ala Tyr Phe Ala Val Val Gln Asn Ile Lys
    450                 455                 460

Lys Glu Glu Ile Glu Asn Leu Gln Lys Tyr His Asp Ile Ile Ser Arg
465                 470                 475                 480

Pro Ser His Ile Phe Arg Leu Cys Asn Asp Leu Ala Ser Ala Ser Ala
                485                 490                 495

Glu Ile Ala Arg Gly Glu Thr Ala Asn Ser Val Ser Cys Tyr Met Arg
            500                 505                 510

Thr Lys Gly Ile Ser Glu Glu Leu Ala Thr Glu Ser Val Met Asn Leu
        515                 520                 525

Ile Asp Glu Thr Cys Lys Lys Met Asn Lys Glu Lys Leu Gly Gly Ser
    530                 535                 540

Leu Phe Ala Lys Pro Phe Val Glu Thr Ala Ile Asn Leu Ala Arg Gln
545                 550                 555                 560

Ser His Cys Thr Tyr His Asn Gly Asp Ala His Thr Ser Pro Asp Glu
                565                 570                 575

Leu Thr Arg Lys Arg Val Leu Ser Val Ile Thr Glu Pro Ile Leu Pro
            580                 585                 590

Phe Glu Arg
        595

<210> 2
<211> 778
<212> PRT
<213> Solanum pennellii

<400> 2

Met Ile Val Gly Tyr Arg Ser Thr Ile Ile Thr Leu Ser His Pro Lys
1               5                   10                  15

Leu Gly Asn Gly Lys Thr Ile Ser Ser Asn Ala Ile Phe Gln Arg Ser
            20                  25                  30

Cys Arg Val Arg Cys Ser His Ser Thr Thr Ser Ser Met Asn Gly Phe
        35                  40                  45

Glu Asp Ala Arg Asp Arg Ile Arg Glu Ser Phe Gly Lys Leu Glu Leu
    50                  55                  60

Ser Pro Ser Ser Tyr Asp Thr Ala Trp Val Ala Met Val Pro Ser Lys
65                  70                  75                  80

His Ser Leu Asn Glu Pro Cys Phe Pro Gln Cys Leu Asp Trp Ile Ile
                85                  90                  95

Glu Asn Gln Arg Glu Asp Gly Ser Trp Gly Leu Asn Pro Thr His Pro
            100                 105                 110

Leu Leu Leu Lys Asp Ser Leu Ser Ser Thr Leu Ala Cys Leu Leu Ala
        115                 120                 125

Leu Thr Lys Trp Arg Val Gly Asp Glu Gln Ile Lys Arg Gly Leu Gly
    130                 135                 140

Phe Ile Glu Thr Tyr Gly Trp Ala Val Asp Asn Lys Asp Gln Ile Ser
145                 150                 155                 160

Pro Leu Gly Phe Glu Val Ile Phe Ser Ser Met Ile Lys Ser Ala Glu
                165                 170                 175

Lys Leu Asp Leu Asn Leu Pro Leu Asn Leu His Leu Val Asn Leu Val
            180                 185                 190

Asn Cys Lys Arg Asp Ser Thr Ile Lys Arg Asn Val Glu Tyr Met Gly
        195                 200                 205

Glu Gly Val Gly Glu Leu Cys Asp Trp Lys Glu Ile Ile Lys Leu His
    210                 215                 220

Gln Arg Gln Asn Gly Ser Leu Phe Asp Ser Pro Ala Thr Thr Ala Ala
225                 230                 235                 240

Ala Leu Ile Tyr His Gln His Asp Gln Lys Cys Asn Gln Tyr Leu Asn
                245                 250                 255

Ser Ile Leu Lys Gln His Lys Asn Trp Val Pro Thr Met Tyr Pro Thr
            260                 265                 270

Lys Ile His Ser Leu Leu Cys Leu Val Asp Thr Leu Gln Asn Leu Gly
        275                 280                 285

Val His Arg His Phe Lys Ser Glu Ile Lys Lys Ala Leu Asp Glu Ile
    290                 295                 300

Tyr Arg Leu Trp Gln Gln Lys Asn Glu Gln Ile Phe Ser Asn Val Thr
305                 310                 315                 320

His Cys Ala Met Ala Phe Arg Leu Leu Arg Met Ser Tyr Tyr Asp Val
                325                 330                 335

Ser Ser Asp Glu Leu Ala Glu Phe Val Asp Glu Glu His Phe Phe Thr
            340                 345                 350

Thr Ser Gly Lys Tyr Thr Ser His Val Glu Ile Leu Glu Leu His Lys
        355                 360                 365

Ala Ser Gln Leu Ala Ile Asp His Glu Lys Asp Asp Ile Leu Asp Lys
    370                 375                 380

Ile Asn Asn Trp Thr Arg Thr Phe Met Glu Gln Lys Leu Leu Asn Asn
385                 390                 395                 400

Gly Phe Ile Asp Arg Met Ser Lys Lys Glu Val Glu Leu Ala Leu Lys
                405                 410                 415

Lys Phe Tyr Thr Thr Ser Asp Leu Ala Glu Asn Arg Arg Tyr Ile Lys
            420                 425                 430

Ser Tyr Glu Glu Asn Asn Phe Lys Ile Leu Lys Ala Ala Tyr Arg Ser
        435                 440                 445

Pro Asn Ile Asn Asn Lys Asp Leu Leu Ala Phe Ser Ile His Glu Phe
    450                 455                 460

Glu Leu Cys Gln Ala Gln His Arg Glu Glu Leu Gln Gln Leu Arg Arg
465                 470                 475                 480

Trp Phe Glu Asp Tyr Arg Leu Asp Gln Leu Gly Leu Ala Glu Arg Tyr
                485                 490                 495

Ile His Ala Thr Tyr Leu Phe Gly Val Thr Ile Ile Pro Glu Pro Glu
            500                 505                 510

Leu Ser Asp Ala Arg Leu Met Asn Ala Lys Tyr Ile Met Leu Leu Thr
        515                 520                 525

Ile Val Asp Glu Tyr Phe Glu Ser Phe Ala Ser Lys Asp Glu Cys Leu
    530                 535                 540

Asn Ile Ile Glu Leu Val Glu Arg Trp Asp Asp Tyr Ala Ser Val Gly
545                 550                 555                 560

Tyr Lys Ser Glu Lys Val Lys Val Phe Phe Ser Thr Phe Tyr Lys Ser
                565                 570                 575

Ile Glu Glu Leu Ala Thr Ile Ala Glu Ile Lys Gln Gly Arg Ser Val
            580                 585                 590

Lys Asn His Leu Ile Asn Leu Trp Leu Glu Val Met Lys Leu Met Leu
        595                 600                 605

Met Glu Gln Val Glu Trp Trp Thr Ser Lys Thr Ile Pro Ser Ile Glu
    610                 615                 620

Glu Tyr Leu Cys Val Thr Ser Ile Thr Phe Gly Ser Arg Leu Leu Leu
625                 630                 635                 640

Leu Thr Ile Gln Tyr Phe Leu Gly Ile Lys Ile Ser Lys Asp Leu Leu
                645                 650                 655

Glu Ser Asp Glu Ile Cys Gly Leu Cys Asn Cys Thr Gly Arg Val Met
            660                 665                 670

Arg Ile Leu Asn Asp Leu Gln Asp Ser Lys Arg Glu Gln Lys Gly Val
        675                 680                 685

Ser Ile Asn Leu Val Thr Leu Leu Met Lys Ser Ile Ser Glu Glu Glu
    690                 695                 700

Ala Ile Met Lys Met Lys Glu Ile Leu Glu Met Asn Arg Arg Glu Leu
705                 710                 715                 720

Leu Lys Met Val Leu Val Gln Lys Lys Gly Ser Gln Leu Pro Gln Leu
                725                 730                 735

Cys Lys Asp Ile Phe Trp Arg Thr Ser Lys Trp Thr His Phe Thr Tyr
            740                 745                 750

Ser Gln Thr Asp Gly Phe Arg Ile Glu Glu Glu Met Lys Asn His Ile
        755                 760                 765

Asp Glu Val Phe Tyr Lys Pro Leu Asn His
    770                 775

<210>  3

<211>  630
<212>  PRT
<213>  Abies grandis

<400>  3

Met Ala Leu Val Ser Ser Ala Pro Lys Ser Cys Leu His Lys Ser Leu
1               5                   10                  15

Ile Arg Ser Thr His His Glu Leu Lys Pro Leu Arg Arg Thr Ile Pro
            20                  25                  30

Thr Leu Gly Met Cys Arg Arg Gly Lys Ser Phe Thr Pro Ser Val Ser
        35                  40                  45

Met Ser Leu Thr Thr Ala Val Ser Asp Asp Gly Leu Gln Arg Arg Ile
    50                  55                  60

Gly Asp Tyr His Ser Asn Leu Trp Asp Asp Asp Phe Ile Gln Ser Leu
65                  70                  75                  80

Ser Thr Pro Tyr Gly Glu Pro Ser Tyr Arg Glu Arg Ala Glu Lys Leu
                85                  90                  95

Ile Gly Glu Val Lys Glu Met Phe Asn Ser Met Pro Ser Glu Asp Gly
            100                 105                 110

Glu Ser Met Ser Pro Leu Asn Asp Leu Ile Glu Arg Leu Trp Met Val
        115                 120                 125

Asp Ser Val Glu Arg Leu Gly Ile Asp Arg His Phe Lys Lys Glu Ile
    130                 135                 140

Lys Ser Ala Leu Asp Tyr Val Tyr Ser Tyr Trp Asn Glu Lys Gly Ile
145                 150                 155                 160

Gly Cys Gly Arg Asp Ser Val Phe Pro Asp Val Asn Ser Thr Ala Ser
                165                 170                 175

Gly Phe Arg Thr Leu Arg Leu His Gly Tyr Ser Val Ser Ser Glu Val
            180                 185                 190

Leu Lys Val Phe Gln Asp Gln Asn Gly Gln Phe Ala Phe Ser Pro Ser
        195                 200                 205

Thr Lys Glu Arg Asp Ile Arg Thr Val Leu Asn Leu Tyr Arg Ala Ser
    210                 215                 220

Phe Ile Ala Phe Pro Gly Glu Lys Val Met Glu Glu Ala Glu Ile Phe

225                 230                 235                 240

Ser Ser Arg Tyr Leu Lys Glu Ala Val Gln Lys Ile Pro Val Ser Ser
                245                 250                 255

Leu Ser Gln Glu Ile Asp Tyr Thr Leu Glu Tyr Gly Trp His Thr Asn
            260                 265                 270

Met Pro Arg Leu Glu Thr Arg Asn Tyr Leu Asp Val Phe Gly His Pro
        275                 280                 285

Thr Ser Pro Trp Leu Lys Lys Lys Arg Thr Gln Tyr Leu Asp Ser Glu
    290                 295                 300

Lys Leu Leu Glu Leu Ala Lys Leu Glu Phe Asn Ile Phe His Ser Leu
305                 310                 315                 320

Gln Gln Lys Glu Leu Gln Tyr Leu Ser Arg Trp Trp Ile His Ser Gly
                325                 330                 335

Leu Pro Glu Leu Thr Phe Gly Arg His Arg His Val Glu Tyr Tyr Thr
            340                 345                 350

Leu Ser Ser Cys Ile Ala Thr Glu Pro Lys His Ser Ala Phe Arg Leu
        355                 360                 365

Gly Phe Ala Lys Thr Cys His Leu Ile Thr Val Leu Asp Asp Ile Tyr
    370                 375                 380

Asp Thr Phe Gly Thr Met Asp Glu Ile Glu Leu Phe Asn Glu Ala Val
385                 390                 395                 400

Arg Arg Trp Asn Pro Ser Glu Lys Glu Arg Leu Pro Glu Tyr Met Lys
                405                 410                 415

Glu Ile Tyr Met Ala Leu Tyr Glu Ala Leu Thr Asp Met Ala Arg Glu
            420                 425                 430

Ala Glu Lys Thr Gln Gly Arg Asp Thr Leu Asn Tyr Ala Arg Lys Ala
        435                 440                 445

Trp Glu Val Tyr Leu Asp Ser Tyr Thr Gln Glu Ala Lys Trp Ile Ala
    450                 455                 460

Ser Gly Tyr Leu Pro Thr Phe Glu Glu Tyr Leu Glu Asn Ala Lys Val
465                 470                 475                 480

Ser Ser Gly His Arg Ala Ala Ala Leu Thr Pro Leu Leu Thr Leu Asp
                485                 490                 495

Val Pro Leu Pro Asp Asp Val Leu Lys Gly Ile Asp Phe Pro Ser Arg
            500                 505                 510

Phe Asn Asp Leu Ala Ser Ser Phe Leu Arg Leu Arg Gly Asp Thr Arg
        515                 520                 525

Cys Tyr Lys Ala Asp Arg Asp Arg Gly Glu Glu Ala Ser Ser Ile Ser
    530                 535                 540

Cys Tyr Met Lys Asp Asn Pro Gly Leu Thr Glu Glu Asp Ala Leu Asn
545                 550                 555                 560

His Ile Asn Ala Met Ile Asn Asp Ile Ile Lys Glu Leu Asn Trp Glu
                565                 570                 575

Leu Leu Lys Pro Asp Ser Asn Ile Pro Met Thr Ala Arg Lys His Ala
            580                 585                 590

Tyr Glu Ile Thr Arg Ala Phe His Gln Leu Tyr Lys Tyr Arg Asp Gly
        595                 600                 605

Phe Ser Val Ala Thr Gln Glu Thr Lys Ser Leu Val Arg Arg Thr Val
    610                 615                 620

Leu Glu Pro Val Pro Leu
625                 630

<210>  4
<211>  574
<212>  PRT
<213>  Artemisia annua

<400>  4

Met Ser Thr Leu Pro Ile Ser Ser Val Ser Phe Ser Ser Ser Thr Ser
1               5                   10                  15

Pro Leu Val Val Asp Asp Lys Val Ser Thr Lys Pro Asp Val Ile Arg
            20                  25                  30

His Thr Met Asn Phe Asn Ala Ser Ile Trp Gly Asp Gln Phe Leu Thr
        35                  40                  45

Tyr Asp Glu Pro Glu Asp Leu Val Met Lys Lys Gln Leu Val Glu Glu
    50                  55                  60

Leu Lys Glu Glu Val Lys Lys Glu Leu Ile Thr Ile Lys Gly Ser Asn
65                  70                  75                  80

Glu Pro Met Gln His Val Lys Leu Ile Glu Leu Ile Asp Ala Val Gln
                85                  90                  95

Arg Leu Gly Ile Ala Tyr His Phe Glu Glu Glu Ile Glu Glu Ala Leu
            100                 105                 110

Gln His Ile His Val Thr Tyr Gly Glu Gln Trp Val Asp Lys Glu Asn
        115                 120                 125

Leu Gln Ser Ile Ser Leu Trp Phe Arg Leu Leu Arg Gln Gln Gly Phe
    130                 135                 140

Asn Val Ser Ser Gly Val Phe Lys Asp Phe Met Asp Glu Lys Gly Lys
145                 150                 155                 160

Phe Lys Glu Ser Leu Cys Asn Asp Ala Gln Gly Ile Leu Ala Leu Tyr
                165                 170                 175

Glu Ala Ala Phe Met Arg Val Glu Asp Glu Thr Ile Leu Asp Asn Ala
            180                 185                 190

Leu Glu Phe Thr Lys Val His Leu Asp Ile Ile Ala Lys Asp Pro Ser
        195                 200                 205

Cys Asp Ser Ser Leu Arg Thr Gln Ile His Gln Ala Leu Lys Gln Pro
    210                 215                 220

Leu Arg Arg Arg Leu Ala Arg Ile Glu Ala Leu His Tyr Met Pro Ile
225                 230                 235                 240

Tyr Gln Gln Glu Thr Ser His Asp Glu Val Leu Leu Lys Leu Ala Lys
                245                 250                 255

Leu Asp Phe Ser Val Leu Gln Ser Met His Lys Lys Glu Leu Ser His
            260                 265                 270

Ile Cys Lys Trp Trp Lys Asp Leu Asp Leu Gln Asn Lys Leu Pro Tyr
        275                 280                 285

Val Arg Asp Arg Val Val Glu Gly Tyr Phe Trp Ile Leu Ser Ile Tyr
    290                 295                 300

Tyr Glu Pro Gln His Ala Arg Thr Arg Met Phe Leu Met Lys Thr Cys
305                 310                 315                 320

Met Trp Leu Val Val Leu Asp Asp Thr Phe Asp Asn Tyr Gly Thr Tyr
                325                 330                 335
Glu Glu Leu Glu Ile Phe Thr Gln Ala Val Glu Arg Trp Ser Ile Ser
            340                 345                 350
Cys Leu Asp Met Leu Pro Glu Tyr Met Lys Leu Ile Tyr Gln Glu Leu
        355                 360                 365
Val Asn Leu His Val Glu Met Glu Glu Ser Leu Glu Lys Glu Gly Lys
    370                 375                 380
Thr Tyr Gln Ile His Tyr Val Lys Glu Met Ala Lys Glu Leu Val Arg
385                 390                 395                 400
Asn Tyr Leu Val Glu Ala Arg Trp Leu Lys Glu Gly Tyr Met Pro Thr
                405                 410                 415
Leu Glu Glu Tyr Met Ser Val Ser Met Val Thr Gly Thr Tyr Gly Leu
            420                 425                 430
Met Ile Ala Arg Ser Tyr Val Gly Arg Gly Asp Ile Val Thr Glu Asp
        435                 440                 445
Thr Phe Lys Trp Val Ser Ser Tyr Pro Pro Ile Ile Lys Ala Ser Cys
    450                 455                 460
Val Ile Val Arg Leu Met Asp Asp Ile Val Ser His Lys Glu Glu Gln
465                 470                 475                 480
Glu Arg Gly His Val Ala Ser Ser Ile Glu Cys Tyr Ser Lys Glu Ser
                485                 490                 495
Gly Ala Ser Glu Glu Glu Ala Cys Glu Tyr Ile Ser Arg Lys Val Glu
            500                 505                 510
Asp Ala Trp Lys Val Ile Asn Arg Glu Ser Leu Arg Pro Thr Ala Val
        515                 520                 525
Pro Phe Pro Leu Leu Met Pro Ala Ile Asn Leu Ala Arg Met Cys Glu
    530                 535                 540
Val Leu Tyr Ser Val Asn Asp Gly Phe Thr His Ala Glu Gly Asp Met
545                 550                 555                 560
Lys Ser Tyr Met Lys Ser Phe Phe Val His Pro Met Val Val
                565                 570

<210>  5
<211>  99
<212>  DNA
<213>  Artificial

<220>
<223>  Linker

<400>  5
agctggcatg cagagtttaa acccagggcg cgccacgcgt ccttcaattg ccgcgatcgt      60

taattaaccg ccacctaggc ctctgcggcc gcactagtc                             99


<210>  6
<211>  99
<212>  DNA
<213>  Artificial

<220>
<223>  Linker

<400>  6
gatcgactag tgcggccgca gaggcctagg tggcggttaa ttaacgatcg cggcaattga      60

aggacgcgtg gcgcgccctg ggtttaaact ctgcatgcc                             99


<210>  7
<211>  964
<212>  DNA
<213>  Unknown

<220>
<223>  DNA fragment for Puromycin resistance

<400>  7
gcatgcatct tctattttca tcttaatttt atatattaaa ggctaatttt tcaatatttt      60

atcgcaaact attgtgttaa ttacatcaaa tatataaact tttctattta aagctaatga     120

aaaagtgaat atatacacat agagtaatgt tatgatgtat atatcaaaaa aataggagtg     180

attcctatga ccgagtacaa gcccacggtg cgcctcgcca cccgcgacga cgtcccccgg     240

gccgtacgca ccctcgccgc cgcgttcgcc gactaccccg ccacgcgcca caccgtcgac     300

ccggaccgcc acatcgagcg ggtcaccgag ctgcaagaac tcttcctcac gcgcgtcggg     360

ctcgacatcg gcaaggtgtg ggtcgcggac gacggcgccg cggtggcggt ctggaccacg     420

ccggagagcg tcgaagcggg ggcggtgttc gccgagatcg gcccgcgcat ggccgagttg     480

agcggttccc ggctggccgc gcagcaacag atggaaggcc tcctggcgcc gcaccggccc     540

aaggagcccg cgtggttcct ggccaccgtc ggcgtctcgc ccgaccacca gggcaagggt     600

ctgggcagcg ccgtcgtgct ccccggagtg gaggcggccg agcgcgccgg ggtgcccgcc     660

ttcctggaga cctccgcgcc ccgcaacctc cccttctacg agcggctcgg cttcaccgtc     720

accgccgacg tcgaggtgcc cgaaggaccg cgcacctggt gcatgacccg caagcccggt 780

gcctgagtta attactaatt tattattaat ttattattag attgggcaaa atagtaaaag 840

aaaactaaag gaaacctaat atggtttcct ttttttatat atttttaaaa attgattata 900

ttatatgtaa acaattataa ttaatgttaa tcgaattcaa tttttattta ataaacggcg 960

cgcc 964

<210> 8
<211> 2918
<212> DNA
<213> Artificial

<220>
<223> DNA fragment encoding IDI-IspS

<400> 8
ttaattaaat gatttttaaa aaaatacatg aattcatcta gcggagaaca caaaagattt 60

aagtaccttc taaacgaatg agatttcatt gggaaagtgg acacttaaga acgacgcggt 120

acttgattta ttgagtgcaa aagcactcga ttaggtgacc agtcccaaag tgattttaat 180

aaattaagga ggaaattaaa atgactgccg acaacaatag tatgccccat ggtgcagtat 240

ctagttacgc caaattagtg caaaaccaaa cacctgaaga cattttggaa gagtttcctg 300

aaattattcc attacaacaa agacctaata cccgatctag tgagacgtca aatgacgaaa 360

gcggagaaac atgtttttct ggtcatgatg aggagcaaat taagttaatg aatgaaaatt 420

gtattgtttt ggattgggac gataatgcta ttggtgccgg taccaagaaa gtttgtcatt 480

taatggaaaa tattgaaaag ggtttactac atcgtgcatt ctccgtcttt attttcaatg 540

aacaaggtga attactttta caacaaagag ccactgaaaa aataactttc cctgatcttt 600

ggactaacac atgctgctct catccactat gtattgatga cgaattaggt ttgaagggta 660

agctagacga taagattaag ggcgctatta ctgcggcggt gagaaaacta gatcatgaat 720

taggtattcc agaagatgaa actaagacaa ggggtaagtt tcacttttta aacagaatcc 780

attacatggc accaagcaat gaaccatggg gtgaacatga aattgattac atcctatttt 840

ataagatcaa cgctaaagaa aacttgactg tcaacccaaa cgtcaatgaa gttagagact 900

tcaaatgggt ttcaccaaat gatttgaaaa ctatgtttgc tgacccaagt tacaagttta 960

cgccttggtt taagattatt tgcgagaatt acttattcaa ctggtgggag caattagatg 1020

acctttctga agtggaaaat gacaggcaaa ttcatagaat gctataaaca aattagttaa 1080

aaggtctaaa gatggaagcc agacggtctg ccaattatga accaaatagc tgggattatg 1140

attttttgct gtcttcagac actgacgaat cgattgaagt atacaaagac aaggccaaaa 1200

agctggaggc tgaggtgaga agagagatta acaatgaaaa ggcagagttt ttgactctgc 1260

ttgaactgat agataatgtc caaaggttag gattgggtta ccggttcgag agtgacataa 1320

ggagagccct cgacagattt gtttcttcag gaggatttga tggtgttaca aaaactagcc 1380

ttcatgctac tgctcttagc ttcaggcttc tcagacagca tggctttgag gtctctcaag 1440

aagcgttcag tggattcaag gatcaaaatg gcaatttctt ggaaaacctt aaggaggaca 1500

ccaaggcaat actaagccta tatgaagctt catttcttgc attagaagga gaaaatatct 1560

tggatgaggc cagggtgttt gcaatatcac atctaaaaga gctcagcgaa gaaaagattg 1620

gaaaagagct ggccgaacag gtgaatcatg cattggagct tccattgcat cgcaggacgc 1680

aaagactaga agctgtttgg agtattgaag cataccgtaa aaaggaagat gcaaatcaag 1740

tactgctaga acttgctata ttggactaca acatgattca atcagtatac caaagagatc 1800

ttcgcgagac atcaaggtgg tggaggcgag tgggtcttgc aacaaagttg cattttgcta 1860

aagacaggtt aattgaaagc ttttactggg cagttggagt tgcgttcgaa cctcaataca 1920

gtgattgccg taattcagta gcaaaaatgt tttcatttgt aacaatcatt gatgatatct 1980

atgatgttta tggtactctg gatgagctgg agctatttac agatgctgtt gagagatggg 2040

atgttaacgc catcaatgat cttccggatt atatgaagct ctgcttccta gctctctaca 2100

acactatcaa tgagatagct tatgacaatc tgaaggacaa gggggaaaac attcttccat 2160

acctaacaaa agcgtgggca gatttatgca atgcattcct acaagaagca aaatggctgt 2220

acaataagtc cacaccaaca tttgatgact atttcggaaa tgcatggaaa tcatcctcag 2280

ggcctcttca actaattttt gcctactttg ccgtggttca aaacatcaag aaagaggaaa 2340

ttgaaaactt acaaaagtat catgatatca tcagtaggcc ttcccacatc tttcgtcttt 2400

gcaacgacct ggcttcagca tcggctgaga tagcgagagg tgaaactgcg aattccgtat 2460

cctgctacat gcgtacaaaa ggcatttctg aggaacttgc tactgaatcc gtaatgaatt 2520

tgatcgacga aacctgtaaa aagatgaaca aagaaaagct tggtggctct ttgtttgcaa 2580

aaccttttgt cgaaacagct attaaccttg cacggcaatc ccattgcact tatcataacg 2640

gagatgcgca tacttcacca gacgagctaa ctaggaaacg tgtcctgtca gtaatcacag 2700

agcctattct accctttgag agataaactc aataaattaa acaacttaaa acagagcagg 2760

ccttcgggcc tgctttttct ttctttcccg agtcccgcct cgggagggcg gtgtcctttc 2820

cgccccgaat tgcgcatcgg gagcacgcgg tccttttcgc tcgctttgct ctctcaagag 2880

gactaattta caaaattaca tattgagagc gcggccgc 2918

<210> 9
<211> 11613
<212> DNA
<213> Artificial

<220>
<223> Vector pAC:Pmcr-IDI-IspS

<400> 9
gaattccgga tgagcattca tcaggcgggc aagaatgtga ataaaggccg gataaaactt 60
gtgcttattt ttctttacgg tctttaaaaa ggccgtaata tccagctgaa cggtctggtt 120
ataggtacat tgagcaactg actgaaatgc ctcaaaatgt tctttacgat gccattggga 180
tatatcaacg gtggtatatc cagtgatttt tttctccatt ttagcttcct tagctcctga 240
aaatctcgat aactcaaaaa atacgcccgg tagtgatctt atttcattat ggtgaaagtt 300
ggaacctctt acgtgccgat caacgtctca ttttcgccaa aagttggccc agggcttccc 360
ggtatcaaca gggacaccag gatttattta ttctgcgaag tgatcttccg tcacaggtat 420
ttattcggcg caaagtgcgt cgggtgatgc tgccaactta ctgatttagt gtatgatggt 480
gtttttgagg tgctccagtg gcttctgttt ctatcagctg tccctcctgt tcagctactg 540
acggggtggt gcgtaacggc aaaagcaccg ccggacatca gcgctagcgg agtgtatact 600
ggcttactat gttggcactg atgagggtgt cagtgaagtg cttcatgtgg caggagaaaa 660
aaggctgcac cggtgcgtca gcagaatatg tgatacagga tatattccgc ttcctcgctc 720
actgactcgc tacgctcggt cgttcgactg cggcgagcgg aaatggctta cgaacggggc 780
ggagatttcc tggaagatgc caggaagata cttaacaggg aagtgagagg gccgcggcaa 840
agccgttttt ccataggctc cgcccccctg acaagcatca cgaaatctga cgctcaaatc 900
agtggtggcg aaacccgaca ggactataaa gataccaggc gtttccccct ggcggctccc 960
tcgtgcgctc tcctgttcct gcctttcggt ttaccggtgt cattccgctg ttatggccgc 1020
gtttgtctca ttccacgcct gacactcagt tccgggtagg cagttcgctc caagctggac 1080
tgtatgcacg aaccccccgt tcagtccgac cgctgcgcct tatccggtaa ctatcgtctt 1140
gagtccaacc cggaaagaca tgcaaaagca ccactggcag cagccactgg taattgattt 1200
agaggagtta gtcttgaagt catgcgccgg ttaaggctaa actgaaagga caagttttgg 1260
tgactgcgct cctccaagcc agttacctcg gttcaaagag ttggtagctc agagaacctt 1320
cgaaaaaccg ccctgcaagg cggttttttc gttttcagag caagagatta cgcgcagacc 1380
aaaacgatct caagaagatc atcttattaa tcagataaaa tatttctaga tttcagtgca 1440
atttatctct tcaaatgtag cacctgaagt cagccccata cgatataagt tgtaattctc 1500
atgtttgaca gcttatcatc gataagcttg catgcatctt ctattttcat cttaatttta 1560
tatattaaag gctaattttt caatatttta tcgcaaacta ttgtgttaat tacatcaaat 1620
atataaactt ttctatttaa agctaatgaa aaagtgaata tatacacata gagtaatgtt 1680
atgatgtata tatcaaaaaa ataggagtga ttcctatgac cgagtacaag cccacggtgc 1740
gcctcgccac ccgcgacgac gtcccccggg ccgtacgcac cctcgccgcc gcgttcgccg 1800
actaccccgc cacgcgccac accgtcgacc cggaccgcca catcgagcgg gtcaccgagc 1860

tgcaagaact cttcctcacg cgcgtcgggc tcgacatcgg caaggtgtgg gtcgcggacg 1920

acggcgccgc ggtggcggtc tggaccacgc cggagagcgt cgaagcgggg gcggtgttcg 1980

ccgagatcgg cccgcgcatg gccgagttga gcggttcccg gctggccgcg cagcaacaga 2040

tggaaggcct cctggcgccg caccggccca aggagcccgc gtggttcctg gccaccgtcg 2100

gcgtctcgcc cgaccaccag ggcaagggtc tgggcagcgc cgtcgtgctc cccggagtgg 2160

aggcggccga gcgcgccggg gtgcccgcct tcctggagac ctccgcgccc cgcaacctcc 2220

ccttctacga gcggctcggc ttcaccgtca ccgccgacgt cgaggtgccc gaaggaccgc 2280

gcacctggtg catgacccgc aagcccggtg cctgagttaa ttactaattt attattaatt 2340

tattattaga ttgggcaaaa tagtaaaaga aaactaaagg aaacctaata tggtttcctt 2400

tttttatata tttttaaaaa ttgattatat tatatgtaaa caattataat taatgttaat 2460

cgaattcaat ttttatttaa taaacggcgc gccacgcgtc cttcaattgc cgcgatcgtt 2520

aattaaatga tttttaaaaa aatacatgaa ttcatctagc ggagaacaca aaagatttaa 2580

gtaccttcta aacgaatgag atttcattgg gaaagtggac acttaagaac gacgcggtac 2640

ttgatttatt gagtgcaaaa gcactcgatt aggtgaccag tcccaaagtg attttaataa 2700

attaaggagg aaattaaaat gactgccgac aacaatagta tgccccatgg tgcagtatct 2760

agttacgcca aattagtgca aaaccaaaca cctgaagaca ttttggaaga gtttcctgaa 2820

attattccat tacaacaaag acctaatacc cgatctagtg agacgtcaaa tgacgaaagc 2880

ggagaaacat gtttttctgg tcatgatgag gagcaaatta agttaatgaa tgaaaattgt 2940

attgttttgg attgggacga taatgctatt ggtgccggta ccaagaaagt ttgtcattta 3000

atggaaaata ttgaaaaggg tttactacat cgtgcattct ccgtctttat tttcaatgaa 3060

caaggtgaat tacttttaca acaaagagcc actgaaaaaa taactttccc tgatctttgg 3120

actaacacat gctgctctca tccactatgt attgatgacg aattaggttt gaagggtaag 3180

ctagacgata agattaaggg cgctattact gcggcggtga gaaaactaga tcatgaatta 3240

ggtattccag aagatgaaac taagacaagg ggtaagtttc actttttaaa cagaatccat 3300

tacatggcac caagcaatga accatggggt gaacatgaaa ttgattacat cctattttat 3360

aagatcaacg ctaaagaaaa cttgactgtc aacccaaacg tcaatgaagt tagagacttc 3420

aaatgggttt caccaaatga tttgaaaact atgtttgctg acccaagtta caagtttacg 3480

ccttggttta agattatttg cgagaattac ttattcaact ggtgggagca attagatgac 3540

ctttctgaag tggaaaatga caggcaaatt catagaatgc tataaacaaa ttagttaaaa 3600

ggtctaaaga tggaagccag acggtctgcc aattatgaac caaatagctg ggattatgat 3660

tttttgctgt cttcagacac tgacgaatcg attgaagtat acaaagacaa ggccaaaaag 3720

ctggaggctg aggtgagaag agagattaac aatgaaaagg cagagttttt gactctgctt 3780

gaactgatag ataatgtcca aaggttagga ttgggttacc ggttcgagag tgacataagg 3840

agagccctcg acagatttgt ttcttcagga ggatttgatg gtgttacaaa aactagcctt 3900

catgctactg ctcttagctt caggcttctc agacagcatg gctttgaggt ctctcaagaa 3960

gcgttcagtg gattcaagga tcaaaatggc aatttcttgg aaaaccttaa ggaggacacc 4020

aaggcaatac taagcctata tgaagcttca tttcttgcat tagaaggaga aaatatcttg 4080

gatgaggcca gggtgtttgc aatatcacat ctaaaagagc tcagcgaaga aaagattgga 4140

aaagagctgg ccgaacaggt gaatcatgca ttggagcttc cattgcatcg caggacgcaa 4200

agactagaag ctgtttggag tattgaagca taccgtaaaa aggaagatgc aaatcaagta 4260

ctgctagaac ttgctatatt ggactacaac atgattcaat cagtatacca aagagatctt 4320

cgcgagacat caaggtggtg gaggcgagtg ggtcttgcaa caaagttgca ttttgctaaa 4380

gacaggttaa ttgaaagctt ttactgggca gttggagttg cgttcgaacc tcaatacagt 4440

gattgccgta attcagtagc aaaaatgttt tcatttgtaa caatcattga tgatatctat 4500

gatgtttatg gtactctgga tgagctggag ctatttacag atgctgttga gagatgggat 4560

gttaacgcca tcaatgatct tccggattat atgaagctct gcttcctagc tctctacaac 4620

actatcaatg agatagctta tgacaatctg aaggacaagg gggaaaacat tcttccatac 4680

ctaacaaaag cgtgggcaga tttatgcaat gcattcctac aagaagcaaa atggctgtac 4740

aataagtcca caccaacatt tgatgactat ttcggaaatg catggaaatc atcctcaggg 4800

cctcttcaac taatttttgc ctactttgcc gtggttcaaa acatcaagaa agaggaaatt 4860

gaaaacttac aaaagtatca tgatatcatc agtaggcctt cccacatctt tcgtctttgc 4920

aacgacctgg cttcagcatc ggctgagata gcgagaggtg aaactgcgaa ttccgtatcc 4980

tgctacatgc gtacaaaagg catttctgag gaacttgcta ctgaatccgt aatgaatttg 5040

atcgacgaaa cctgtaaaaa gatgaacaaa gaaaagcttg gtggctcttt gtttgcaaaa 5100

ccttttgtcg aaacagctat taaccttgca cggcaatccc attgcactta tcataacgga 5160

gatgcgcata cttcaccaga cgagctaact aggaaacgtg tcctgtcagt aatcacagag 5220

cctattctac cctttgagag ataaactcaa taaattaaac aacttaaaac agagcaggcc 5280

ttcgggcctg ctttttcttt ctttcccgag tcccgcctcg ggagggcggt gtcctttccg 5340

ccccgaattg cgcatcggga gcacgcggtc cttttcgctc gctttgctct ctcaagagga 5400

ctaatttaca aaattacata ttgagagcgc ggccgcacta gtaagttata gaaatgtcat 5460

ccgataccga aaaagaaaac ggaataaatc agtaacaaat caaaaaaaca aaactataac 5520

atttaatctt cgtaaaatca atatcaatcg accatacaca aaacatgttc aaataaaata 5580

catgggtgta catatgtcaa ccgacattga aaaagaaaac ggaataaatc agtaacaaat 5640

caaaaaaaca aaaccataac atttctataa cattccatag agtaaaaatc aacatcgaat 5700

aaccttacac aaaccataca taaaccatac ataaaaatac acatatgtca accgttaccg 5760

taaaaagatt acttaaataa ttttatactc acctcttatt ttttgtgttt tttattatcc 5820

agggcaacct ttcccaggga gaaattcttc cttgcagttg caggcaagaa ccaggacttt 5880

cttccagggc atactttccc agggagaaat tcttccttgc agctgcaggc aagaaccagg 5940

actttccccc aggaccagga cctcaaggtc tgcatccaga ggaagacctt cccagggcgc 6000

caagatcccg cagattatgg aacattattt cccgggaaat aattcagggc ttaatgccca 6060

actgaggaat acattttaaa taagcttctc ttagttcatc cttttctatg tggtcgtaca 6120

cgtctattgc ttcgcctctc gaatctcccc ttaattcctg aatgaatgtc ctttccatac 6180

ctgaacgtcg aagccatgta gtgaaccaat gccggcagca atgcggagta aaatgttctt 6240

caagtctccc ctcggggtta tggagtccga tagaggaagc ggcagatgtg acaatatcgt 6300

acactaaatt tctttgtaca cggtgaccgt tttgtctgcc tatgaataat gctttttctt 6360

tatcatgcct cgtgttaagg tacgcttcca ggaaggcctt agtctcaagg tcaaagaata 6420

cgatacgatt tgacctttta gctgtcttct tgagtctcac cgtacacttt tccagatcta 6480

tgtcagctcg atcaagagta attaattcat ttctcctgat gccagtttta gctaaaaaga 6540

ttatcaatgc ttgatacatg gggtaacgtg gtactttgac aagctttgcc atgtcttcaa 6600

tggtaatcaa ttgccttgtt tccggagtat ggtatttttt gtagcttctt agatagcgtt 6660

ttctgaacgt gggaatgttg tttctctcga ttatatgctt gaattcaagg aagtcaaaaa 6720

aggtactcag tgaagcaaag taattgttac aggttgcgag tgtgtactgc ctttcatctc 6780

ttatgtgaac aaggaaccct tcaagctccg gaaaccctgc attcactcca cacatttgaa 6840

ggaaaaactt agcatgactt acgtatcctt ctattgttct tttagagtaa ccccttccga 6900

tacaatcaga agtgaataaa cttataagtc tttcattatc aagcggttca tagataccag 6960

ttagctgaat agctgccatt tcacccataa atcaataaaa gaagtatgta aataaatact 7020

ttgtgcatag atgtcattta aacacaaagt ataagtacat ctaccttctt ttagtatatg 7080

ctggtaacag caaaaaagcg ccaaacaacg catctttacc caatagaatg acgtcgatta 7140

aaagagtaag ttggtggtga gccttaaaaa aggtaaaaga gagcttactc ttaagttaaa 7200

aacggaattg gcaaacagta aggattgttc cagcagtcct tactaaaaac attactttat 7260

taattctata tagtcacaac attcacaaaa ataggctttt ttctcgattt ttccttttga 7320

atgtttataa agaaaaatca tatagcattt attacactgt ggacatattt tcttttcaat 7380

tttgcccctt ccaatttgtg gaagcctttt ttcagtaaca cgcttaggca taatgaaaga 7440

tcggtgtatt aagtattaac attttctaac ttgtgcaata ataattattc ggggttgtcg 7500

aagaacttcc aaacagtaga ccgatccatt ttccacatta ttctaatgaa gccaagactc 7560

aaggatgggg ggaaatatcc ccccacaccc cccaatgaga ctacccaaga cagcccgtat 7620

gccgacagcg acacagttaa atatcaatgt gtattaatac atatcaacaa gtatcaacac 7680

acacaattaa acaataatac acatcaatgg agaacaatac acatgccaac acaagaatac 7740

acacggacaa acattacatt accagaacat gttagagcaa tgattaaaga gtacaataaa 7800

agaagtacat ggggagacct caacgtatca gcagtatgct ctaatgcaat agcagacaga 7860

attagagcta cattcccaaa attagaaaaa gaactatttc cagaacaatt tccagaactt 7920

aacccaaaaa tgacaaaaac cagctcacca ggacaaccag aaataacaca aaaaggaaaa 7980

tccaaacaag gagactctaa agatagaata tgcgcttatt gtggccaacc ttttgaaccg 8040

aaatcgcata atcaaaaatt ctgtaaagat gcatgtaagt cagcaaatta tagaaaaaac 8100

aaaaaaacac agagttaaac ccattaacca cactcttttt ttgaatttat ctgcatttca 8160

cggtactttt tatccaagaa agcccacaaa tcacgctctt taaacggttt catggaaaaa 8220

ttttcccgga atgcattaat taacatcttt tcaattttag ggttgaaata agccccataa 8280

cggtaatctg gcgaccaatc tgaagcttta acctcttcat aagtccgata cggcacatta 8340

cccggaagaa gcccaggaac cacaagcaga taatagatat agcaccttat acacatatgt 8400

gattttttag tagtctttaa ccattcaata tatgaaagat ccgcacctgt tcgataatcc 8460

tcaaaaagaa ttaagttcct ttgcctccga gatccacaca cacagcaata cttcaattcc 8520

ataatacata acaccccgaa ataataagac atctgaaaaa ccgtatcact ttaggcttta 8580

agcatcttat ccggaccttc gagcccaaaa gccttgttga acgtggcatc tcaatcaccc 8640

ccaaacatca tggagcgtga ttttaagctt gtaaatagct cttcataatt aatttcggaa 8700

gccttttgca agctgttaga aggcatttca ggcgattctt caattataca gccatcatgc 8760

tcccattgat gagagtcaac caccaggaag ccggcagggg gatcaggaag cccttctaag 8820

aaccataccc gaagaagact cccggcaggt tcaagcttca aaaactccgg taattcatcc 8880

actttgaagg gagaagccca taatttacgc cattttttag gaactttgtt tttgccatct 8940

cgccctttta ggttatgata aaaattattt cttgcaatta attgttgcaa gtaatcggca 9000

tattcaggtt taaccgcttt agcttccttc aaataagcat gtagagcctg tcttccaccc 9060

gaaaaatgat cccgaagctt tgttaacggt atccatccag tatcatcgtt ttcctgtcct 9120

tcccacttca actctccatt ataaacgagt tttacttctc gaccttcatt gattaaatta 9180

aggacttcgg ttctggtatc tatcaattgc tcaaatgtca gctttccgga ggaacttaca 9240

actttatcag gatgccaccg gaacaaagca ccaaagcgag attctatatg gatattaccg 9300

tatctgttat ccgtgagctg cccaacgtga gaataaagat aagtcacctg cttgtaaata 9360

tccataaggc tccgcaattc ccattgcctt ttaccattga ctttatgaga aagcctctga 9420

ataaatatat catattcatc actcttgcat ctctttttac tcttcggatt ctttccagtt 9480

atacgaatgt ttccagggaa aatgaaaaaa tgaaaatgag gagcaaggat aaggcagtca 9540

taaatagagt ttatttcgat atttagcaag tccgcgatct tgtttaaatt gccttcgttg 9600

tggtcgtctc ttatgtaatt ccaaaaagct tcggagtcgt cattaccttt taaacctgtt 9660

aacttacgga tagcccgagt tatttccggc ttaatcctgt aagggtgaaa gattgtagta 9720

ccagcagtta cgccccagcg ttccagcctg tcctttaaat tcctccggaa aagtcggatt 9780

gcttccacag atactccccg gctaaagtcc atcgaagcaa acccccatga aggaaactgc 9840

ccagaaaaca gggcataagt ccacaaaatt acaactaatg caaatctatg ttgcaattcc 9900

gataggatgc cgcaaccagg acaagaaagt gtattgcagt gagaattaag atcctttcgt 9960

gcattataat tttcagatac agcataagtg aaaggacgcc cacagtaagc gggtcttttt 10020

ccctttgctg gaagaacaaa gtcgccaact tttagaggac gatacttaga gcatttagaa 10080

aaatcgaagt caggacaaga gtagtattta cagatagaaa ccccgttctt tttgttgcga 10140

accgggcaca aaacaccatc ggtacatata cacgtcgcaa aatctctaca ctctggagca 10200

taggaaacgg cgccaggtct agaactaaaa tcagaactca gagaacattt cctgtcgaaa 10260

atttaaaccg taaaccaaag ataacaattt tcataatatt acagaaccta tattttttgt 10320

aactcgacat tacaaaaatg taccacaaaa cataaaaaca ttttcataga agaaatattc 10380

acaaacaata gaaaaaaatt ataagaataa gattaacgcc taccttatta aaagaaggtg 10440

tcgagtcttc aggttccatc aaggcaggcg ttgatattct atatatgtta cacataacat 10500

atataacata cgtacctcag aacattcaga cctgaatgct ctaaaatgaa ataagcttta 10560

aactttataa attcatctgc tataaatagt tttagagata tagaaagccc aaggaagaaa 10620

aagaatagca aaaataaaaa agaaaaacgg cccgcgacgg caaacccgga gacttataat 10680

caaagtaaga cgaaaaggaa gccgacaacg atttgataat aacaaatcat cttttatttt 10740

ggcaatattc atatgatata tgaagaaatc aacatcaagc ggattagtat ttttgctatt 10800

tgtaaatcaa tatcgggtga gacagtatat caaaaacaaa cttcattttt ttcgaacaaa 10860

aaaatcacta tcaaatgaca ttgtagtaag aaactacatc aaaactagtt gatcatatcg 10920

tcaattatta cctccacggg gagagcctga gcaaactggc ctcaggcatt tgagaagcac 10980

acggtcacac tgcttccggt agtcaataaa ccggtaaacc agcaatagac ataagcggct 11040

atttaacgac cctgccctga accgacgacc gggtcgaatt tgctttcgaa tttctgccat 11100

tcatccgctt attatcactt attcaggcgt agcaccaggc gtttaagggc accaataact 11160

gccttaaaaa aattacgccc cgccctgcca ctcatcgcag tactgttgta attcattaag 11220

cattctgccg acatggaagc catcacagac ggcatgatga acctgaatcg ccagcggcat 11280

cagcaccttg tcgccttgcg tataatattt gcccatggtg aaaacggggg cgaagaagtt 11340

gtccatattg gccacgttta aatcaaaact ggtgaaactc acccagggat tggctgagac 11400

gaaaaacata ttctcaataa accctttagg gaaataggcc aggttttcac cgtaacacgc 11460

cacatcttgc gaatatatgt gtagaaactg ccggaaatcg tcgtggtatt cactccagag 11520

cgatgaaaac gtttcagttt gctcatggaa aacggtgtaa caagggtgaa cactatccca 11580

tatcaccagc tcaccgtctt tcattgccat acg 11613

<210> 10
<211> 8728
<212> DNA
<213> Artificial

<220>
<223> Vector pAC:Pur

<400> 10
gaattccgga tgagcattca tcaggcgggc aagaatgtga ataaaggccg gataaaactt 60

gtgcttattt ttctttacgg tctttaaaaa ggccgtaata tccagctgaa cggtctggtt 120

ataggtacat tgagcaactg actgaaatgc ctcaaaatgt tctttacgat gccattggga 180

tatatcaacg gtggtatatc cagtgatttt tttctccatt ttagcttcct tagctcctga 240

aaatctcgat aactcaaaaa atacgcccgg tagtgatctt atttcattat ggtgaaagtt 300

ggaacctctt acgtgccgat caacgtctca ttttcgccaa aagttggccc agggcttccc 360

ggtatcaaca gggacaccag gatttattta ttctgcgaag tgatcttccg tcacaggtat 420

ttattcggcg caaagtgcgt cgggtgatgc tgccaactta ctgatttagt gtatgatggt 480

gtttttgagg tgctccagtg gcttctgttt ctatcagctg tccctcctgt tcagctactg 540

acggggtggt gcgtaacggc aaaagcaccg ccggacatca gcgctagcgg agtgtatact 600

ggcttactat gttggcactg atgagggtgt cagtgaagtg cttcatgtgg caggagaaaa 660

aaggctgcac cggtgcgtca gcagaatatg tgatacagga tatattccgc ttcctcgctc 720

actgactcgc tacgctcggt cgttcgactg cggcgagcgg aaatggctta cgaacggggc 780

ggagatttcc tggaagatgc caggaagata cttaacaggg aagtgagagg gccgcggcaa 840

agccgttttt ccataggctc cgcccccctg acaagcatca cgaaatctga cgctcaaatc 900

agtggtggcg aaacccgaca ggactataaa gataccaggc gtttccccct ggcggctccc 960

tcgtgcgctc tcctgttcct gcctttcggt ttaccggtgt cattccgctg ttatggccgc 1020

gtttgtctca ttccacgcct gacactcagt tccgggtagg cagttcgctc caagctggac 1080

tgtatgcacg aaccccccgt tcagtccgac cgctgcgcct tatccggtaa ctatcgtctt 1140

gagtccaacc cggaaagaca tgcaaaagca ccactggcag cagccactgg taattgattt 1200

agaggagtta gtcttgaagt catgcgccgg ttaaggctaa actgaaagga caagttttgg 1260

tgactgcgct cctccaagcc agttacctcg gttcaaagag ttggtagctc agagaacctt 1320

cgaaaaaccg ccctgcaagg cggttttttc gttttcagag caagagatta cgcgcagacc 1380

aaaacgatct caagaagatc atcttattaa tcagataaaa tatttctaga tttcagtgca 1440

atttatctct tcaaatgtag cacctgaagt cagccccata cgatataagt tgtaattctc 1500

atgtttgaca gcttatcatc gataagcttg catgcatctt ctattttcat cttaatttta 1560

tatattaaag gctaattttt caatatttta tcgcaaacta ttgtgttaat tacatcaaat 1620

atataaactt ttctatttaa agctaatgaa aaagtgaata tatacacata gagtaatgtt 1680

atgatgtata tatcaaaaaa ataggagtga ttcctatgac cgagtacaag cccacggtgc 1740

gcctcgccac ccgcgacgac gtcccccggg ccgtacgcac cctcgccgcc gcgttcgccg 1800

actaccccgc cacgcgccac accgtcgacc cggaccgcca catcgagcgg gtcaccgagc 1860

tgcaagaact cttcctcacg cgcgtcgggc tcgacatcgg caaggtgtgg gtcgcggacg 1920

acggcgccgc ggtggcggtc tggaccacgc cggagagcgt cgaagcgggg gcggtgttcg 1980

ccgagatcgg cccgcgcatg gccgagttga gcggttcccg gctggccgcg cagcaacaga 2040

tggaaggcct cctggcgccg caccggccca aggagcccgc gtggttcctg gccaccgtcg 2100

gcgtctcgcc cgaccaccag ggcaagggtc tgggcagcgc cgtcgtgctc cccggagtgg 2160

aggcggccga gcgcgccggg gtgcccgcct tcctggagac ctccgcgccc cgcaacctcc 2220

ccttctacga gcggctcggc ttcaccgtca ccgccgacgt cgaggtgccc gaaggaccgc 2280

gcacctggtg catgacccgc aagcccggtg cctgagttaa ttactaattt attattaatt 2340

tattattaga ttgggcaaaa tagtaaaaga aaactaaagg aaacctaata tggtttcctt 2400

tttttatata tttttaaaaa ttgattatat tatatgtaaa caattataat taatgttaat 2460

cgaattcaat ttttatttaa taaacggcgc gccacgcgtc cttcaattgc cgcgatcgtt 2520

aattaaccgc cacctaggcc tctgcggccg cactagtaag ttatagaaat gtcatccgat 2580

accgaaaaag aaaacggaat aaatcagtaa caaatcaaaa aaacaaaact ataacattta 2640

atcttcgtaa aatcaatatc aatcgaccat acacaaaaca tgttcaaata aaatacatgg 2700

gtgtacatat gtcaaccgac attgaaaaag aaaacggaat aaatcagtaa caaatcaaaa 2760

aaacaaaacc ataacatttc tataacattc catagagtaa aaatcaacat cgaataacct 2820

tacacaaacc atacataaac catacataaa aatacacata tgtcaaccgt taccgtaaaa 2880

agattactta aataatttta tactcacctc ttattttttg tgttttttat tatccagggc 2940

aacctttccc agggagaaat tcttccttgc agttgcaggc aagaaccagg actttcttcc 3000

agggcatact ttcccaggga gaaattcttc cttgcagctg caggcaagaa ccaggacttt 3060

cccccaggac caggacctca aggtctgcat ccagaggaag accttcccag ggcgccaaga 3120

tcccgcagat tatggaacat tatttcccgg gaaataattc agggcttaat gcccaactga 3180

ggaatacatt ttaaataagc ttctcttagt tcatcctttt ctatgtggtc gtacacgtct 3240

attgcttcgc ctctcgaatc tccccttaat tcctgaatga atgtcctttc catacctgaa 3300

cgtcgaagcc atgtagtgaa ccaatgccgg cagcaatgcg gagtaaaatg ttcttcaagt 3360

ctcccctcgg ggttatggag tccgatagag gaagcggcag atgtgacaat atcgtacact 3420

aaatttcttt gtacacggtg accgttttgt ctgcctatga ataatgcttt ttctttatca 3480

tgcctcgtgt taaggtacgc ttccaggaag gccttagtct caaggtcaaa gaatacgata 3540

cgatttgacc ttttagctgt cttcttgagt ctcaccgtac acttttccag atctatgtca 3600

gctcgatcaa gagtaattaa ttcatttctc ctgatgccag ttttagctaa aaagattatc 3660

aatgcttgat acatggggta acgtggtact ttgacaagct ttgccatgtc ttcaatggta 3720

atcaattgcc ttgtttccgg agtatggtat tttttgtagc ttcttagata gcgttttctg 3780

aacgtgggaa tgttgtttct ctcgattata tgcttgaatt caaggaagtc aaaaaaggta 3840

ctcagtgaag caaagtaatt gttacaggtt gcgagtgtgt actgcctttc atctcttatg 3900

tgaacaagga acccttcaag ctccggaaac cctgcattca ctccacacat ttgaaggaaa 3960

aacttagcat gacttacgta tccttctatt gttcttttag agtaacccct tccgatacaa 4020

tcagaagtga ataaacttat aagtctttca ttatcaagcg gttcatagat accagttagc 4080

tgaatagctg ccatttcacc cataaatcaa taaaagaagt atgtaaataa atactttgtg 4140

catagatgtc atttaaacac aaagtataag tacatctacc ttcttttagt atatgctggt 4200

aacagcaaaa aagcgccaaa caacgcatct ttacccaata gaatgacgtc gattaaaaga 4260

gtaagttggt ggtgagcctt aaaaaaggta aaagagagct tactcttaag ttaaaaacgg 4320

aattggcaaa cagtaaggat tgttccagca gtccttacta aaaacattac tttattaatt 4380

ctatatagtc acaacattca caaaaatagg cttttttctc gatttttcct tttgaatgtt 4440

tataaagaaa aatcatatag catttattac actgtggaca tattttcttt tcaattttgc 4500

cccttccaat ttgtggaagc cttttttcag taacacgctt aggcataatg aaagatcggt 4560

gtattaagta ttaacatttt ctaacttgtg caataataat tattcggggt tgtcgaagaa 4620

cttccaaaca gtagaccgat ccattttcca cattattcta atgaagccaa gactcaagga 4680

tggggggaaa tatcccccca caccccccaa tgagactacc caagacagcc cgtatgccga 4740

cagcgacaca gttaaatatc aatgtgtatt aatacatatc aacaagtatc aacacacaca 4800

attaaacaat aatacacatc aatggagaac aatacacatg ccaacacaag aatacacacg 4860

gacaaacatt acattaccag aacatgttag agcaatgatt aaagagtaca ataaaagaag 4920

tacatgggga gacctcaacg tatcagcagt atgctctaat gcaatagcag acagaattag 4980

agctacattc ccaaaattag aaaaagaact atttccagaa caatttccag aacttaaccc 5040

aaaaatgaca aaaaccagct caccaggaca accagaaata acacaaaaag gaaaatccaa 5100

acaaggagac tctaaagata gaatatgcgc ttattgtggc caaccttttg aaccgaaatc 5160

gcataatcaa aaattctgta aagatgcatg taagtcagca aattatagaa aaaacaaaaa 5220

aacacagagt taaacccatt aaccacactc tttttttgaa tttatctgca tttcacggta 5280

ctttttatcc aagaaagccc acaaatcacg ctctttaaac ggtttcatgg aaaaattttc 5340

ccggaatgca ttaattaaca tcttttcaat tttagggttg aaataagccc cataacggta 5400

atctggcgac caatctgaag ctttaacctc ttcataagtc cgatacggca cattacccgg 5460

aagaagccca ggaaccacaa gcagataata gatatagcac cttatacaca tatgtgattt 5520

tttagtagtc tttaaccatt caatatatga aagatccgca cctgttcgat aatcctcaaa 5580

aagaattaag ttcctttgcc tccgagatcc acacacacag caatacttca attccataat 5640

acataacacc ccgaaataat aagacatctg aaaaaccgta tcactttagg ctttaagcat 5700

cttatccgga ccttcgagcc caaaagcctt gttgaacgtg gcatctcaat cacccccaaa 5760

catcatggag cgtgatttta agcttgtaaa tagctcttca taattaattt cggaagcctt 5820

ttgcaagctg ttagaaggca tttcaggcga ttcttcaatt atacagccat catgctccca 5880

ttgatgagag tcaaccacca ggaagccggc agggggatca ggaagccctt ctaagaacca 5940

tacccgaaga agactcccgg caggttcaag cttcaaaaac tccggtaatt catccacttt 6000

gaagggagaa gcccataatt tacgccattt tttaggaact ttgtttttgc catctcgccc 6060

ttttaggtta tgataaaaat tatttcttgc aattaattgt tgcaagtaat cggcatattc 6120

aggtttaacc gctttagctt ccttcaaata agcatgtaga gcctgtcttc cacccgaaaa 6180

atgatcccga agctttgtta acggtatcca tccagtatca tcgttttcct gtccttccca 6240

cttcaactct ccattataaa cgagttttac ttctcgacct tcattgatta aattaaggac 6300

ttcggttctg gtatctatca attgctcaaa tgtcagcttt ccggaggaac ttacaacttt 6360

atcaggatgc caccggaaca aagcaccaaa gcgagattct atatggatat taccgtatct 6420

gttatccgtg agctgcccaa cgtgagaata aagataagtc acctgcttgt aaatatccat 6480

aaggctccgc aattcccatt gccttttacc attgacttta tgagaaagcc tctgaataaa 6540

tatatcatat tcatcactct tgcatctctt tttactcttc ggattctttc cagttatacg 6600

aatgtttcca gggaaaatga aaaaatgaaa atgaggagca aggataaggc agtcataaat 6660

agagtttatt tcgatattta gcaagtccgc gatcttgttt aaattgcctt cgttgtggtc 6720

gtctcttatg taattccaaa aagcttcgga gtcgtcatta ccttttaaac ctgttaactt 6780

acggatagcc cgagttattt ccggcttaat cctgtaaggg tgaaagattg tagtaccagc 6840

agttacgccc cagcgttcca gcctgtcctt taaattcctc cggaaaagtc ggattgcttc 6900

cacagatact ccccggctaa agtccatcga agcaaacccc catgaaggaa actgcccaga 6960

aaacagggca taagtccaca aaattacaac taatgcaaat ctatgttgca attccgatag 7020

gatgccgcaa ccaggacaag aaagtgtatt gcagtgagaa ttaagatcct ttcgtgcatt 7080

ataattttca gatacagcat aagtgaaagg acgcccacag taagcgggtc tttttccctt 7140

tgctggaaga acaaagtcgc caacttttag aggacgatac ttagagcatt tagaaaaatc 7200

gaagtcagga caagagtagt atttacagat agaaaccccg ttctttttgt tgcgaaccgg 7260

gcacaaaaca ccatcggtac atatacacgt cgcaaaatct ctacactctg gagcatagga 7320

aacggcgcca ggtctagaac taaaatcaga actcagagaa catttcctgt cgaaaattta 7380

aaccgtaaac caaagataac aattttcata atattacaga acctatattt tttgtaactc 7440

gacattacaa aaatgtacca caaaacataa aaacattttc atagaagaaa tattcacaaa 7500

caatagaaaa aaattataag aataagatta acgcctacct tattaaaaga aggtgtcgag 7560

tcttcaggtt ccatcaaggc aggcgttgat attctatata tgttacacat aacatatata 7620

acatacgtac ctcagaacat tcagacctga atgctctaaa atgaaataag ctttaaactt 7680

tataaattca tctgctataa atagttttag agatatagaa agcccaagga agaaaaagaa 7740

tagcaaaaat aaaaaagaaa aacggcccgc gacggcaaac ccggagactt ataatcaaag 7800

taagacgaaa aggaagccga caacgatttg ataataacaa atcatctttt attttggcaa 7860

tattcatatg atatatgaag aaatcaacat caagcggatt agtatttttg ctatttgtaa 7920

atcaatatcg ggtgagacag tatatcaaaa acaaacttca tttttttcga acaaaaaaat 7980

cactatcaaa tgacattgta gtaagaaact acatcaaaac tagttgatca tatcgtcaat 8040

tattacctcc acggggagag cctgagcaaa ctggcctcag gcatttgaga agcacacggt 8100

cacactgctt ccggtagtca ataaaccggt aaaccagcaa tagacataag cggctattta 8160

acgaccctgc cctgaaccga cgaccgggtc gaatttgctt tcgaatttct gccattcatc 8220

cgcttattat cacttattca ggcgtagcac caggcgttta agggcaccaa taactgcctt 8280

aaaaaaatta cgccccgccc tgccactcat cgcagtactg ttgtaattca ttaagcattc 8340

tgccgacatg gaagccatca cagacggcat gatgaacctg aatcgccagc ggcatcagca 8400

ccttgtcgcc ttgcgtataa tatttgccca tggtgaaaac gggggcgaag aagttgtcca 8460

tattggccac gtttaaatca aaactggtga aactcaccca gggattggct gagacgaaaa 8520

acatattctc aataaaccct ttagggaaat aggccaggtt ttcaccgtaa cacgccacat 8580

cttgcgaata tatgtgtaga aactgccgga aatcgtcgtg gtattcactc cagagcgatg 8640

aaaacgtttc agtttgctca tggaaaacgg tgtaacaagg gtgaacacta tcccatatca 8700

ccagctcacc gtctttcatt gccatacg 8728

<210>  11
<211>  3008
<212>  DNA
<213>  Artificial

<220>
<223>  DNA fragment encoding IDI-FnS

<400>  11
ttaattaaat gatttttaaa aaaatacatg aattcatcta gcggagaaca caaaagattt      60

aagtaccttc taaacgaatg agatttcatt gggaaagtgg acacttaaga acgacgcggt     120

acttgattta ttgagtgcaa aagcactcga ttaggtgacc agtcccaaag tgattttaat     180

aaattaagga ggaaattaaa atgactgccg acaacaatag tatgccccat ggtgcagtat     240

ctagttacgc caaattagtg caaaaccaaa cacctgaaga cattttggaa gagtttcctg     300

aaattattcc attacaacaa agacctaata cccgatctag tgagacgtca aatgacgaaa     360

gcggagaaac atgttttctt ggtcatgatg aggagcaaat taagttaatg aatgaaaatt     420

gtattgtttt ggattgggac gataatgcta ttggtgccgg taccaagaaa gtttgtcatt     480

taatggaaaa tattgaaaag ggtttactac atcgtgcatt ctccgtcttt attttcaatg     540

aacaaggtga attactttta caacaaagag ccactgaaaa aataactttc cctgatcttt     600

ggactaacac atgctgctct catccactat gtattgatga cgaattaggt ttgaagggta     660

agctagacga taagattaag ggcgctatta ctgcggcggt gagaaaacta gatcatgaat     720

taggtattcc agaagatgaa actaagacaa ggggtaagtt tcacttttta aacagaatcc     780

attacatggc accaagcaat gaaccatggg gtgaacatga aattgattac atcctatttt     840

ataagatcaa cgctaaagaa aacttgactg tcaacccaaa cgtcaatgaa gttagagact     900

tcaaatgggt ttcaccaaat gatttgaaaa ctatgtttgc tgacccaagt tacaagttta     960

cgccttggtt taagattatt tgcgagaatt acttattcaa ctggtgggag caattagatg    1020

acctttctga agtggaaaat gacaggcaaa ttcatagaat gctataaaca aattagttaa    1080

aaggactaaa gatgtcgact cttcctattt ctagtgtttc attctcttca tctacgtcac    1140

ctttggttgt tgacgataaa gttagcacga aaccagatgt tatccgccat accatgaatt    1200

tcaatgctag catatgggga gatcaatttc ttacatatga tgagccagaa gatttggtta    1260

tgaagaaaca acttgttgaa gagctcaaag aggaagtcaa gaaagagcta ataacgatca    1320

aaggttcaaa tgaaccgatg cagcatgtca agttgataga acttattgat gcagtccaac    1380

ggcttggcat agcctatcat tttgaagagg agattgaaga agccttgcaa catatccatg    1440

ttacatatgg cgagcagtgg gtcgataaag aaaacctaca aagcatttct ctttggttcc    1500

gactcctacg acaacaaggc ttcaacgtct catcaggagt attcaaggac tttatggacg    1560

agaagggaaa atttaaggaa tccttatgta atgatgctca gggaattctt gctctgtatg    1620

aagcggcatt tatgagggtg gaagatgaaa caatactaga taatgcgctc gagttcacta 1680

aagttcacct tgacatcata gccaaggatc cttcttgtga ctcttcccta agaacccaaa 1740

tacaccaagc gttaaagcag ccacttagga gaaggctggc aaggattgag gcgttgcatt 1800

acatgcctat ctaccaacaa gaaacatccc acgatgaggt cttactgaag cttgcaaaat 1860

tagattttag cgtgcttcag tcgatgcaca aaaaggaact tagccacatt tgcaaatggt 1920

ggaaggattt ggacctccaa aataagcttc cttatgttcg agacagggtg gtggaaggct 1980

atttttggat actatccatc tattacgagc ctcaacatgc tcgaacaaga atgttcctaa 2040

tgaaaacatg catgtggtta gtcgttttag atgatacatt tgataattat ggtacttatg 2100

aagaactcga aatctttaca caagctgttg aaagatggtc aataagctgc ctggatatgc 2160

ttccagaata catgaaacta atatatcaag agcttgtgaa tcttcacgtg gaaatggagg 2220

aatcacttga aaaggaggga aaaacatatc aaattcacta tgtcaaggag atggcaaaag 2280

agttggttcg caattacttg gtagaagcca gatggctaaa agaggggtac atgccaactc 2340

ttgaggagta catgtctgtg tcaatggtga ctggtaccta tggcttgatg atagcgagat 2400

cttatgtcgg caggggtgat atcgtcaccg aggatacctt taaatgggtg tcctcgtatc 2460

ctcctattat aaaagcttca tgtgtgattg taagacttat ggatgatatt gtcagccaca 2520

aggaggaaca agagagaggc catgttgctt caagcatcga atgctattct aaggaaagtg 2580

gtgcatcaga ggaggaagcg tgtgaatata tctcaagaaa agttgaagat gcatggaaag 2640

ttataaaccg agagtcgctc aggcctacag ctgtcccatt tcctctactt atgcctgcaa 2700

tcaaccttgc acgtatgtgt gaagtcctat atagcgtcaa cgatggcttc actcatgctg 2760

agggagacat gaaaagttac atgaaatcgt tcttcgttca ccctatggtt gtctaaactc 2820

aataaattaa acaacttaaa acagagcagg ccttcgggcc tgctttttct ttctttcccg 2880

agtcccgcct cgggagggcg gtgtcctttc cgccccgaat tgcgcatcgg gagcacgcgg 2940

tccttttcgc tcgctttgct ctctcaagag gactaattta caaaattaca tattgagagc 3000

gcggccgc 3008

<210> 12
<211> 4083
<212> DNA
<213> Artificial

<220>
<223> DNA fragment encoding IDI-FnS-FPS

<400> 12
ttaattaaat gatttttaaa aaaatacatg aattcatcta gcggagaaca caaaagattt 60

aagtaccttc taaacgaatg agatttcatt gggaaagtgg acacttaaga acgacgcggt 120

acttgattta ttgagtgcaa aagcactcga ttaggtgacc agtcccaaag tgattttaat 180

aaattaagga ggaaattaaa atgactgccg acaacaatag tatgccccat ggtgcagtat 240

ctagttacgc caaattagtg caaaaccaaa cacctgaaga cattttggaa gagtttcctg 300

aaattattcc attacaacaa agacctaata cccgatctag tgagacgtca aatgacgaaa 360

gcggagaaac atgtttttct ggtcatgatg aggagcaaat taagttaatg aatgaaaatt 420

gtattgtttt ggattgggac gataatgcta ttggtgccgg taccaagaaa gtttgtcatt 480

taatggaaaa tattgaaaag ggtttactac atcgtgcatt ctccgtcttt attttcaatg 540

aacaaggtga attactttta caacaaagag ccactgaaaa aataactttc cctgatcttt 600

ggactaacac atgctgctct catccactat gtattgatga cgaattaggt ttgaagggta 660

agctagacga taagattaag ggcgctatta ctgcggcggt gagaaaacta gatcatgaat 720

taggtattcc agaagatgaa actaagacaa ggggtaagtt tcacttttta aacagaatcc 780

attacatggc accaagcaat gaaccatggg gtgaacatga aattgattac atcctatttt 840

ataagatcaa cgctaaagaa aacttgactg tcaacccaaa cgtcaatgaa gttagagact 900

tcaaatgggt ttcaccaaat gatttgaaaa ctatgtttgc tgacccaagt tacaagttta 960

cgccttggtt taagattatt tgcgagaatt acttattcaa ctggtgggag caattagatg 1020

acctttctga agtggaaaat gacaggcaaa ttcatagaat gctataaaca aattagttaa 1080

aaggactaaa gatgtcgact cttcctattt ctagtgtttc attctcttca tctacgtcac 1140

ctttggttgt tgacgataaa gttagcacga aaccagatgt tatccgccat accatgaatt 1200

tcaatgctag catatgggga gatcaatttc ttacatatga tgagccagaa gatttggtta 1260

tgaagaaaca acttgttgaa gagctcaaag aggaagtcaa gaaagagcta ataacgatca 1320

aaggttcaaa tgaaccgatg cagcatgtca agttgataga acttattgat gcagtccaac 1380

ggcttggcat agcctatcat tttgaagagg agattgaaga agccttgcaa catatccatg 1440

ttacatatgg cgagcagtgg gtcgataaag aaaacctaca aagcatttct ctttggttcc 1500

gactcctacg acaacaaggc ttcaacgtct catcaggagt attcaaggac tttatggacg 1560

agaaggaaaa atttaaggaa tccttatgta atgatgctca gggaattctt gctctgtatg 1620

aagcggcatt tatgagggtg gaagatgaaa caatactaga taatgcgctc gagttcacta 1680

aagttcacct tgacatcata gccaaggatc cttcttgtga ctcttcccta agaacccaaa 1740

tacaccaagc gttaaagcag ccacttagga gaaggctggc aaggattgag gcgttgcatt 1800

acatgcctat ctaccaacaa gaaacatccc acgatgaggt cttactgaag cttgcaaaat 1860

tagattttag cgtgcttcag tcgatgcaca aaaaggaact tagccacatt tgcaaatggt 1920

ggaaggattt ggacctccaa aataagcttc cttatgttcg agacagggtg gtggaaggct 1980

atttttggat actatccatc tattacgagc ctcaacatgc tcgaacaaga atgttcctaa 2040

tgaaaacatg catgtggtta gtcgttttag atgatacatt tgataattat ggtacttatg 2100

aagaactcga aatctttaca caagctgttg aaagatggtc aataagctgc ctggatatgc 2160

ttccagaata catgaaacta atatatcaag agcttgtgaa tcttcacgtg gaaatggagg 2220

aatcacttga aaaggaggga aaaacatatc aaattcacta tgtcaaggag atggcaaaag 2280

agttggttcg caattacttg gtagaagcca gatggctaaa agaggggtac atgccaactc 2340

ttgaggagta catgtctgtg tcaatggtga ctggtaccta tggcttgatg atagcgagat 2400

cttatgtcgg caggggtgat atcgtcaccg aggatacctt taaatgggtg tcctcgtatc 2460

ctcctattat aaaagcttca tgtgtgattg taagacttat ggatgatatt gtcagccaca 2520

aggaggaaca agagagaggc catgttgctt caagcatcga atgctattct aaggaaagtg 2580

gtgcatcaga ggaggaagcg tgtgaatata tctcaagaaa agttgaagat gcatggaaag 2640

ttataaaccg agagtcgctc aggcctacag ctgtcccatt tcctctactt atgcctgcaa 2700

tcaaccttgc acgtatgtgt gaagtcctat atagcgtcaa cgatggcttc actcatgctg 2760

agggagacat gaaaagttac atgaaatcgt tcttcgttca ccctatggtt gtctaaaaag 2820

gaggagatca taatggcttc agaaaaagaa attaggagag agagattctt gaacgttttc 2880

cctaaattag tagaggaatt gaacgcatcg cttttggctt acggtatgcc taaggaagca 2940

tgtgactggt atgcccactc attgaactac aacactccag gcggtaagct aaatagaggt 3000

ttgtccgttg tggacacgta tgctattctc tccaacaaga ccgttgaaca attggggcaa 3060

gaagaatacg aaaaggttgc cattctaggt tggtgcattg agttgttgca ggcttacttc 3120

ttggtcgccg atgatatgat ggacaagtcc attaccagaa gaggccaacc atgttggtac 3180

aaggttcctg aagttgggga aattgccatc aatgacgcat tcatgttaga ggctgctatc 3240

tacaagcttt tgaaatctca cttcagaaac gaaaaatact acatagatat caccgaattg 3300

ttccatgagg tcaccttcca aaccgaattg ggccaattga tggacttaat cactgcacct 3360

gaagacaaag tcgacttgag taagttctcc ctaaagaagc actccttcat agttactttc 3420

aagactgctt actattcttt ctacttgcct gtcgcattgg ccatgtacgt tgccggtatc 3480

acggatgaaa aggatttgaa acaagccaga gatgtcttga ttccattggg tgaatacttc 3540

caaattcaag atgactactt agactgcttc ggtaccccag aacagatcgg taagatcggt 3600

acagatatcc aagataacaa atgttcttgg gtaatcaaca aggcattgga acttgcttcc 3660

gcagaacaaa gaaagacttt agacgaaaat tacggtaaga aggactcagt cgcagaagcc 3720

aaatgcaaaa agattttcaa tgacttgaaa attgaacagc tataccacga atatgaagag 3780

tctattgcca aggatttgaa ggccaaaatt tctcaggtcg atgagtctcg tggcttcaaa 3840

gctgatgtct taactgcgtt cttgaacaaa gtttacaaga gaagcaaata gactcaataa 3900

attaaacaac ttaaaacaga gcaggccttc gggcctgctt tttctttctt tcccgagtcc 3960

cgcctcggga gggcggtgtc ctttccgccc cgaattgcgc atcgggagca cgcggtcctt 4020

ttcgctcgct ttgctctctc aagaggacta atttacaaaa ttacatattg agagcgcggc 4080

cgc 4083

<210> 13
<211> 11703
<212> DNA
<213> Artificial

<220>
<223> Vector pAC:Pmcr-IDI-FnS

<400> 13
gaattccgga tgagcattca tcaggcgggc aagaatgtga ataaaggccg gataaaactt 60

gtgcttattt ttctttacgg tctttaaaaa ggccgtaata tccagctgaa cggtctggtt 120

ataggtacat tgagcaactg actgaaatgc ctcaaaatgt tctttacgat gccattggga 180

tatatcaacg gtggtatatc cagtgatttt tttctccatt ttagcttcct tagctcctga 240

aaatctcgat aactcaaaaa atacgcccgg tagtgatctt atttcattat ggtgaaagtt 300

ggaacctctt acgtgccgat caacgtctca ttttcgccaa aagttggccc agggcttccc 360

ggtatcaaca gggacaccag gatttattta ttctgcgaag tgatcttccg tcacaggtat 420

ttattcggcg caaagtgcgt cgggtgatgc tgccaactta ctgatttagt gtatgatggt 480

gtttttgagg tgctccagtg gcttctgttt ctatcagctg tccctcctgt tcagctactg 540

acggggtggt gcgtaacggc aaaagcaccg ccggacatca gcgctagcgg agtgtatact 600

ggcttactat gttggcactg atgagggtgt cagtgaagtg cttcatgtgg caggagaaaa 660

aaggctgcac cggtgcgtca gcagaatatg tgatacagga tatattccgc ttcctcgctc 720

actgactcgc tacgctcggt cgttcgactg cggcgagcgg aaatggctta cgaacggggc 780

ggagatttcc tggaagatgc caggaagata cttaacaggg aagtgagagg gccgcggcaa 840

agccgttttt ccataggctc cgcccccctg acaagcatca cgaaatctga cgctcaaatc 900

agtggtggcg aaacccgaca ggactataaa gataccaggc gtttccccct ggcggctccc 960

tcgtgcgctc tcctgttcct gcctttcggt ttaccggtgt cattccgctg ttatggccgc 1020

gtttgtctca ttccacgcct gacactcagt tccgggtagg cagttcgctc caagctggac 1080

tgtatgcacg aaccccccgt tcagtccgac cgctgcgcct tatccggtaa ctatcgtctt 1140

gagtccaacc cggaaagaca tgcaaaagca ccactggcag cagccactgg taattgattt 1200

agaggagtta gtcttgaagt catgcgccgg ttaaggctaa actgaaagga caagttttgg 1260

tgactgcgct cctccaagcc agttacctcg gttcaaagag ttggtagctc agagaacctt 1320

cgaaaaaccg ccctgcaagg cggttttttc gttttcagag caagagatta cgcgcagacc 1380

aaaacgatct caagaagatc atcttattaa tcagataaaa tatttctaga tttcagtgca 1440

atttatctct tcaaatgtag cacctgaagt cagccccata cgatataagt tgtaattctc 1500

atgtttgaca gcttatcatc gataagcttg catgcatctt ctattttcat cttaatttta 1560

tatattaaag gctaattttt caatatttta tcgcaaacta ttgtgttaat tacatcaaat 1620

atataaactt ttctatttaa agctaatgaa aaagtgaata tatacacata gagtaatgtt 1680

atgatgtata tatcaaaaaa ataggagtga ttcctatgac cgagtacaag cccacggtgc 1740

gcctcgccac ccgcgacgac gtcccccggg ccgtacgcac cctcgccgcc gcgttcgccg 1800

actaccccgc cacgcgccac accgtcgacc cggaccgcca catcgagcgg gtcaccgagc 1860

tgcaagaact cttcctcacg cgcgtcgggc tcgacatcgg caaggtgtgg gtcgcggacg 1920

acggcgccgc ggtggcggtc tggaccacgc cggagagcgt cgaagcgggg gcggtgttcg 1980

ccgagatcgg cccgcgcatg gccgagttga gcggttcccg gctggccgcg cagcaacaga 2040

tggaaggcct cctggcgccg caccggccca aggagcccgc gtggttcctg gccaccgtcg 2100

gcgtctcgcc cgaccaccag ggcaagggtc tgggcagcgc cgtcgtgctc cccggagtgg 2160

aggcggccga gcgcgccggg gtgcccgcct tcctggagac ctccgcgccc cgcaacctcc 2220

ccttctacga gcggctcggc ttcaccgtca ccgccgacgt cgaggtgccc gaaggaccgc 2280

gcacctggtg catgacccgc aagcccggtg cctgagttaa ttactaattt attattaatt 2340

tattattaga ttgggcaaaa tagtaaaaga aaactaaagg aaacctaata tggtttcctt 2400

tttttatata tttttaaaaa ttgattatat tatatgtaaa caattataat taatgttaat 2460

cgaattcaat ttttatttaa taaacggcgc gccacgcgtc cttcaattgc cgcgatcgtt 2520

aattaaatga tttttaaaaa aatacatgaa ttcatctagc ggagaacaca aaagatttaa 2580

gtaccttcta aacgaatgag atttcattgg gaaagtggac acttaagaac gacgcggtac 2640

ttgatttatt gagtgcaaaa gcactcgatt aggtgaccag tcccaaagtg attttaataa 2700

attaaggagg aaattaaaat gactgccgac aacaatagta tgccccatgg tgcagtatct 2760

agttacgcca aattagtgca aaaccaaaca cctgaagaca ttttggaaga gtttcctgaa 2820

attattccat tacaacaaag acctaatacc cgatctagtg agacgtcaaa tgacgaaagc 2880

ggagaaacat gtttttctgg tcatgatgag gagcaaatta agttaatgaa tgaaaattgt 2940

attgttttgg attgggacga taatgctatt ggtgccggta ccaagaaagt ttgtcattta 3000

atggaaaata ttgaaaaggg tttactacat cgtgcattct ccgtctttat tttcaatgaa 3060

caaggtgaat tacttttaca acaaagagcc actgaaaaaa taactttccc tgatctttgg 3120

actaacacat gctgctctca tccactatgt attgatgacg aattaggttt gaagggtaag 3180

ctagacgata agattaaggg cgctattact gcggcggtga gaaaactaga tcatgaatta 3240

ggtattccag aagatgaaac taagacaagg ggtaagtttc actttttaaa cagaatccat 3300

tacatggcac caagcaatga accatggggt gaacatgaaa ttgattacat cctattttat 3360

aagatcaacg ctaaagaaaa cttgactgtc aacccaaacg tcaatgaagt tagagacttc 3420

aaatgggttt caccaaatga tttgaaaact atgtttgctg acccaagtta caagtttacg 3480

ccttggttta agattatttg cgagaattac ttattcaact ggtgggagca attagatgac 3540

ctttctgaag tggaaaatga caggcaaatt catagaatgc tataaacaaa ttagttaaaa 3600

ggactaaaga tgtcgactct tcctatttct agtgtttcat tctcttcatc tacgtcacct 3660

ttggttgttg acgataaagt tagcacgaaa ccagatgtta tccgccatac catgaatttc 3720

aatgctagca tatggggaga tcaatttctt acatatgatg agccagaaga tttggttatg 3780

aagaaacaac ttgttgaaga gctcaaagag gaagtcaaga aagagctaat aacgatcaaa 3840

ggttcaaatg aaccgatgca gcatgtcaag ttgatagaac ttattgatgc agtccaacgg 3900

cttggcatag cctatcattt tgaagaggag attgaagaag ccttgcaaca tatccatgtt 3960

acatatggcg agcagtgggt cgataaagaa aacctacaaa gcatttctct ttggttccga 4020

ctcctacgac aacaaggctt caacgtctca tcaggagtat tcaaggactt tatggacgag 4080

aagggaaaat ttaaggaatc cttatgtaat gatgctcagg gaattcttgc tctgtatgaa 4140

gcggcattta tgagggtgga agatgaaaca atactagata atgcgctcga gttcactaaa 4200

gttcaccttg acatcatagc caaggatcct tcttgtgact cttccctaag aacccaaata 4260

caccaagcgt taaagcagcc acttaggaga aggctggcaa ggattgaggc gttgcattac 4320

atgcctatct accaacaaga aacatcccac gatgaggtct tactgaagct tgcaaaatta 4380

gattttagcg tgcttcagtc gatgcacaaa aaggaactta gccacatttg caaatggtgg 4440

aaggatttgg acctccaaaa taagcttcct tatgttcgag acagggtggt ggaaggctat 4500

ttttggatac tatccatcta ttacgagcct caacatgctc gaacaagaat gttcctaatg 4560

aaaacatgca tgtggttagt cgttttagat gatacatttg ataattatgg tacttatgaa 4620

gaactcgaaa tctttacaca agctgttgaa agatggtcaa taagctgcct ggatatgctt 4680

ccagaataca tgaaactaat atatcaagag cttgtgaatc ttcacgtgga aatggaggaa 4740

tcacttgaaa aggagggaaa aacatatcaa attcactatg tcaaggagat ggcaaaagag 4800

ttggttcgca attacttggt agaagccaga tggctaaaag aggggtacat gccaactctt 4860

gaggagtaca tgtctgtgtc aatggtgact ggtacctatg gcttgatgat agcgagatct 4920

tatgtcggca ggggtgatat cgtcaccgag gataccttta aatgggtgtc ctcgtatcct 4980

cctattataa aagcttcatg tgtgattgta agacttatgg atgatattgt cagccacaag 5040

gaggaacaag agagaggcca tgttgcttca agcatcgaat gctattctaa ggaaagtggt 5100

gcatcagagg aggaagcgtg tgaatatatc tcaagaaaag ttgaagatgc atggaaagtt 5160

ataaaccgag agtcgctcag gcctacagct gtcccatttc ctctacttat gcctgcaatc 5220

aaccttgcac gtatgtgtga agtcctatat agcgtcaacg atggcttcac tcatgctgag 5280

ggagacatga aaagttacat gaaatcgttc ttcgttcacc ctatggttgt ctaaactcaa 5340

taaattaaac aacttaaaac agagcaggcc ttcgggcctg cttttctttt ctttcccgag 5400

tcccgcctcg ggagggcggt gtcctttccg ccccgaattg cgcatcggga gcacgcggtc 5460

cttttcgctc gctttgctct ctcaagagga ctaatttaca aaattacata ttgagagcgc 5520

ggccgcacta gtaagttata gaaatgtcat ccgataccga aaaagaaaac ggaataaatc 5580

agtaacaaat caaaaaaaca aaactataac atttaatctt cgtaaaatca atatcaatcg 5640

accatacaca aaacatgttc aaataaaata catgggtgta catatgtcaa ccgacattga 5700

aaaagaaaac ggaataaatc agtaacaaat caaaaaaaca aaaccataac atttctataa 5760

cattccatag agtaaaaatc aacatcgaat aaccttacac aaaccataca taaaccatac 5820

ataaaaatac acatatgtca accgttaccg taaaaagatt acttaaataa ttttatactc 5880

acctcttatt ttttgtgttt tttattatcc agggcaacct ttcccaggga gaaattcttc 5940

cttgcagttg caggcaagaa ccaggacttt cttccagggc atactttccc agggagaaat 6000

tcttccttgc agctgcaggc aagaaccagg actttccccc aggaccagga cctcaaggtc 6060

tgcatccaga ggaagacctt cccagggcgc caagatcccg cagattatgg aacattattt 6120

cccgggaaat aattcagggc ttaatgccca actgaggaat acattttaaa taagcttctc 6180

ttagttcatc cttttctatg tggtcgtaca cgtctattgc ttcgcctctc gaatctcccc 6240

ttaattcctg aatgaatgtc ctttccatac ctgaacgtcg aagccatgta gtgaaccaat 6300

gccggcagca atgcggagta aaatgttctt caagtctccc ctcggggtta tggagtccga 6360

tagaggaagc ggcagatgtg acaatatcgt acactaaatt tctttgtaca cggtgaccgt 6420

tttgtctgcc tatgaataat gctttttctt tatcatgcct cgtgttaagg tacgcttcca 6480

ggaaggcctt agtctcaagg tcaaagaata cgatacgatt tgacctttta gctgtcttct 6540

tgagtctcac cgtacacttt tccagatcta tgtcagctcg atcaagagta attaattcat 6600

ttctcctgat gccagtttta gctaaaaaga ttatcaatgc ttgatacatg gggtaacgtg 6660

gtactttgac aagctttgcc atgtcttcaa tggtaatcaa ttgccttgtt tccggagtat 6720

ggtatttttt gtagcttctt agatagcgtt ttctgaacgt gggaatgttg tttctctcga 6780

ttatatgctt gaattcaagg aagtcaaaaa aggtactcag tgaagcaaag taattgttac 6840

aggttgcgag tgtgtactgc ctttcatctc ttatgtgaac aaggaaccct tcaagctccg 6900

gaaaccctgc attcactcca cacatttgaa ggaaaaactt agcatgactt acgtatcctt 6960

ctattgttct tttagagtaa ccccttccga tacaatcaga agtgaataaa cttataagtc 7020

tttcattatc aagcggttca tagataccag ttagctgaat agctgccatt tcacccataa 7080

atcaataaaa gaagtatgta aataaatact ttgtgcatag atgtcattta aacacaaagt 7140

ataagtacat ctaccttctt ttagtatatg ctggtaacag caaaaaagcg ccaaacaacg 7200

catctttacc caatagaatg acgtcgatta aaagagtaag ttggtggtga gccttaaaaa 7260

aggtaaaaga gagcttactc ttaagttaaa aacggaattg gcaaacagta aggattgttc 7320

cagcagtcct tactaaaaac attactttat taattctata tagtcacaac attcacaaaa 7380

ataggctttt ttctcgattt ttccttttga atgtttataa agaaaaatca tatagcattt 7440

attacactgt ggacatattt tcttttcaat tttgcccctt ccaatttgtg gaagcctttt 7500

ttcagtaaca cgcttaggca taatgaaaga tcggtgtatt aagtattaac attttctaac 7560

ttgtgcaata ataattattc ggggttgtcg aagaacttcc aaacagtaga ccgatccatt 7620

ttccacatta ttctaatgaa gccaagactc aaggatgggg ggaaatatcc ccccacaccc 7680

cccaatgaga ctacccaaga cagcccgtat gccgacagcg acacagttaa atatcaatgt 7740

gtattaatac atatcaacaa gtatcaacac acacaattaa acaataatac acatcaatgg 7800

agaacaatac acatgccaac acaagaatac acacggacaa acattacatt accagaacat 7860

gttagagcaa tgattaaaga gtacaataaa agaagtacat ggggagacct caacgtatca 7920

gcagtatgct ctaatgcaat agcagacaga attagagcta cattcccaaa attagaaaaa 7980

gaactatttc cagaacaatt tccagaactt aacccaaaaa tgacaaaaac cagctcacca 8040

ggacaaccag aaataacaca aaaaggaaaa tccaaacaag gagactctaa agatagaata 8100

tgcgcttatt gtggccaacc ttttgaaccg aaatcgcata atcaaaaatt ctgtaaagat 8160

gcatgtaagt cagcaaatta tagaaaaaac aaaaaaacac agagttaaac ccattaacca 8220

cactcttttt ttgaatttat ctgcatttca cggtactttt tatccaagaa agcccacaaa 8280

tcacgctctt taaacggttt catggaaaaa ttttcccgga atgcattaat taacatcttt 8340

tcaattttag ggttgaaata agccccataa cggtaatctg gcgaccaatc tgaagcttta 8400

acctcttcat aagtccgata cggcacatta cccggaagaa gcccaggaac cacaagcaga 8460

taatagatat agcaccttat acacatatgt gattttttag tagtctttaa ccattcaata 8520

tatgaaagat ccgcacctgt tcgataatcc tcaaaaagaa ttaagttcct ttgcctccga 8580

gatccacaca cacagcaata cttcaattcc ataatacata acaccccgaa ataataagac 8640

atctgaaaaa ccgtatcact ttaggcttta agcatcttat ccggaccttc gagcccaaaa 8700

gccttgttga acgtggcatc tcaatcaccc ccaaacatca tggagcgtga ttttaagctt 8760

gtaaatagct cttcataatt aatttcggaa gccttttgca agctgttaga aggcatttca 8820

ggcgattctt caattataca gccatcatgc tcccattgat gagagtcaac caccaggaag 8880

ccggcagggg gatcaggaag cccttctaag aaccataccc gaagaagact cccggcaggt 8940

tcaagcttca aaaactccgg taattcatcc actttgaagg gagaagccca taatttacgc 9000

cattttttag gaactttgtt tttgccatct cgccctttta ggttatgata aaaattattt 9060

cttgcaatta attgttgcaa gtaatcggca tattcaggtt taaccgcttt agcttccttc 9120

aaataagcat gtagagcctg tcttccaccc gaaaaatgat cccgaagctt tgttaacggt 9180

atccatccag tatcatcgtt ttcctgtcct tcccacttca actctccatt ataaacgagt 9240

tttacttctc gaccttcatt gattaaatta aggacttcgg ttctggtatc tatcaattgc 9300

tcaaatgtca gctttccgga ggaacttaca actttatcag gatgccaccg gaacaaagca 9360

ccaaagcgag attctatatg gatattaccg tatctgttat ccgtgagctg cccaacgtga 9420

gaataaagat aagtcacctg cttgtaaata tccataaggc tccgcaattc ccattgcctt 9480

ttaccattga ctttatgaga aagcctctga ataaatatat catattcatc actcttgcat 9540

ctctttttac tcttcggatt ctttccagtt atacgaatgt ttccagggaa aatgaaaaaa 9600

tgaaaatgag gagcaaggat aaggcagtca taaatagagt ttatttcgat atttagcaag 9660

tccgcgatct tgtttaaatt gccttcgttg tggtcgtctc ttatgtaatt ccaaaaagct 9720

tcggagtcgt cattaccttt taaacctgtt aacttacgga tagcccgagt tatttccggc 9780

ttaatcctgt aagggtgaaa gattgtagta ccagcagtta cgccccagcg ttccagcctg 9840

tcctttaaat tcctccggaa aagtcggatt gcttccacag atactccccg gctaaagtcc 9900

atcgaagcaa acccccatga aggaaactgc ccagaaaaca gggcataagt ccacaaaatt 9960

acaactaatg caaatctatg ttgcaattcc gataggatgc cgcaaccagg acaagaaagt 10020

gtattgcagt gagaattaag atcctttcgt gcattataat tttcagatac agcataagtg 10080

aaaggacgcc cacagtaagc gggtcttttt ccctttgctg gaagaacaaa gtcgccaact 10140

tttagaggac gatacttaga gcatttagaa aaatcgaagt caggacaaga gtagtattta 10200

cagatagaaa ccccgttctt tttgttgcga accgggcaca aaacaccatc ggtacatata 10260

cacgtcgcaa aatctctaca ctctggagca taggaaacgg cgccaggtct agaactaaaa 10320

tcagaactca gagaacattt cctgtcgaaa atttaaaccg taaaccaaag ataacaattt 10380

tcataatatt acagaaccta tattttttgt aactcgacat tacaaaaatg taccacaaaa 10440

cataaaaaca ttttcataga agaaatattc acaaacaata gaaaaaaatt ataagaataa 10500

gattaacgcc taccttatta aaagaaggtg tcgagtcttc aggttccatc aaggcaggcg 10560

ttgatattct atatatgtta cacataacat atataacata cgtacctcag aacattcaga 10620

cctgaatgct ctaaaatgaa ataagcttta aactttataa attcatctgc tataaatagt 10680

tttagagata tagaaagccc aaggaagaaa aagaatagca aaaataaaaa agaaaaacgg 10740

cccgcgacgg caaacccgga gacttataat caaagtaaga cgaaaaggaa gccgacaacg 10800

atttgataat aacaaatcat cttttatttt ggcaatattc atatgatata tgaagaaatc 10860

aacatcaagc ggattagtat ttttgctatt tgtaaatcaa tatcgggtga gacagtatat 10920

caaaaacaaa cttcattttt ttcgaacaaa aaaatcacta tcaaatgaca ttgtagtaag 10980

aaactacatc aaaactagtt gatcatatcg tcaattatta cctccacggg gagagcctga 11040

gcaaactggc ctcaggcatt tgagaagcac acggtcacac tgcttccggt agtcaataaa 11100

ccggtaaacc agcaatagac ataagcggct atttaacgac cctgccctga accgacgacc 11160

gggtcgaatt tgctttcgaa tttctgccat tcatccgctt attatcactt attcaggcgt 11220

agcaccaggc gtttaagggc accaataact gccttaaaaa aattacgccc cgccctgcca 11280

ctcatcgcag tactgttgta attcattaag cattctgccg acatggaagc catcacagac 11340

ggcatgatga acctgaatcg ccagcggcat cagcaccttg tcgccttgcg tataatattt 11400

gcccatggtg aaaacggggg cgaagaagtt gtccatattg gccacgttta aatcaaaact 11460

ggtgaaactc acccagggat tggctgagac gaaaaacata ttctcaataa accctttagg 11520

gaaataggcc aggttttcac cgtaacacgc cacatcttgc gaatatatgt gtagaaactg 11580

ccggaaatcg tcgtggtatt cactccagag cgatgaaaac gtttcagttt gctcatggaa 11640

aacggtgtaa caagggtgaa cactatccca tatcaccagc tcaccgtctt tcattgccat 11700

acg 11703

<210> 14
<211> 12778
<212> DNA
<213> Artificial

<220>
<223> pAC:Pmcr-IDI-FnS-FPS

<400> 14
gaattccgga tgagcattca tcaggcgggc aagaatgtga ataaaggccg gataaaactt 60

gtgcttattt ttctttacgg tctttaaaaa ggccgtaata tccagctgaa cggtctggtt 120

ataggtacat tgagcaactg actgaaatgc ctcaaaatgt tctttacgat gccattggga 180

tatatcaacg gtggtatatc cagtgatttt tttctccatt ttagcttcct tagctcctga 240

aaatctcgat aactcaaaaa atacgcccgg tagtgatctt atttcattat ggtgaaagtt 300

ggaacctctt acgtgccgat caacgtctca ttttcgccaa aagttggccc agggcttccc 360

ggtatcaaca gggacaccag gatttattta ttctgcgaag tgatcttccg tcacaggtat 420

ttattcggcg caaagtgcgt cgggtgatgc tgccaactta ctgatttagt gtatgatggt 480

gtttttgagg tgctccagtg gcttctgttt ctatcagctg tccctcctgt tcagctactg 540

acggggtggt gcgtaacggc aaaagcaccg ccggacatca gcgctagcgg agtgtatact 600

ggcttactat gttggcactg atgagggtgt cagtgaagtg cttcatgtgg caggagaaaa 660

aaggctgcac cggtgcgtca gcagaatatg tgatacagga tatattccgc ttcctcgctc 720

actgactcgc tacgctcggt cgttcgactg cggcgagcgg aaatggctta cgaacggggc 780

ggagatttcc tggaagatgc caggaagata cttaacaggg aagtgagagg gccgcggcaa 840

agccgttttt ccataggctc cgcccccctg acaagcatca cgaaatctga cgctcaaatc 900

agtggtggcg aaacccgaca ggactataaa gataccaggc gtttccccct ggcggctccc 960

tcgtgcgctc tcctgttcct gcctttcggt ttaccggtgt cattccgctg ttatggccgc 1020

gtttgtctca ttccacgcct gacactcagt tccgggtagg cagttcgctc caagctggac 1080

tgtatgcacg aaccccccgt tcagtccgac cgctgcgcct tatccggtaa ctatcgtctt 1140

gagtccaacc cggaaagaca tgcaaaagca ccactggcag cagccactgg taattgattt 1200

agaggagtta gtcttgaagt catgcgccgg ttaaggctaa actgaaagga caagttttgg 1260

tgactgcgct cctccaagcc agttacctcg gttcaaagag ttggtagctc agagaacctt 1320

cgaaaaaccg ccctgcaagg cggttttttc gttttcagag caagagatta cgcgcagacc 1380

aaaacgatct caagaagatc atcttattaa tcagataaaa tatttctaga tttcagtgca 1440

atttatctct tcaaatgtag cacctgaagt cagccccata cgatataagt tgtaattctc 1500

atgtttgaca gcttatcatc gataagcttg catgcatctt ctattttcat cttaatttta 1560

tatattaaag gctaattttt caatatttta tcgcaaacta ttgtgttaat tacatcaaat 1620

atataaactt ttctatttaa agctaatgaa aaagtgaata tatacacata gagtaatgtt 1680

atgatgtata tatcaaaaaa ataggagtga ttcctatgac cgagtacaag cccacggtgc 1740

gcctcgccac ccgcgacgac gtcccccggg ccgtacgcac cctcgccgcc gcgttcgccg 1800

actaccccgc cacgcgccac accgtcgacc cggaccgcca catcgagcgg gtcaccgagc 1860

tgcaagaact cttcctcacg cgcgtcgggc tcgacatcgg caaggtgtgg gtcgcggacg 1920

acggcgccgc ggtggcggtc tggaccacgc cggagagcgt cgaagcgggg gcggtgttcg 1980

ccgagatcgg cccgcgcatg gccgagttga gcggttcccg gctggccgcg cagcaacaga 2040

tggaaggcct cctggcgccg caccggccca aggagcccgc gtggttcctg gccaccgtcg 2100

gcgtctcgcc cgaccaccag ggcaagggtc tgggcagcgc cgtcgtgctc cccggagtgg 2160

aggcggccga gcgcgccggg gtgcccgcct tcctggagac ctccgcgccc cgcaacctcc 2220

ccttctacga gcggctcggc ttcaccgtca ccgccgacgt cgaggtgccc gaaggaccgc 2280

gcacctggtg catgacccgc aagcccggtg cctgagttaa ttactaattt attattaatt 2340

tattattaga ttgggcaaaa tagtaaaaga aaactaaagg aaacctaata tggtttcctt 2400

tttttatata tttttaaaaa ttgattatat tatatgtaaa caattataat taatgttaat 2460

cgaattcaat ttttatttaa taaacggcgc gccacgcgtc cttcaattgc cgcgatcgtt 2520

aattaaatga tttttaaaaa aatacatgaa ttcatctagc ggagaacaca aaagatttaa 2580

gtaccttcta aacgaatgag atttcattgg gaaagtggac acttaagaac gacgcggtac 2640

ttgatttatt gagtgcaaaa gcactcgatt aggtgaccag tcccaaagtg attttaataa 2700

attaaggagg aaattaaaat gactgccgac aacaatagta tgccccatgg tgcagtatct 2760

agttacgcca aattagtgca aaaccaaaca cctgaagaca ttttggaaga gtttcctgaa 2820

attattccat tacaacaaag acctaatacc cgatctagtg agacgtcaaa tgacgaaagc 2880

ggagaaacat gtttttctgg tcatgatgag gagcaaatta agttaatgaa tgaaaattgt 2940

attgttttgg attgggacga taatgctatt ggtgccggta ccaagaaagt ttgtcattta 3000

atggaaaata ttgaaaaggg tttactacat cgtgcattct ccgtctttat tttcaatgaa 3060

caaggtgaat tacttttaca acaaagagcc actgaaaaaa taactttccc tgatctttgg 3120

actaacacat gctgctctca tccactatgt attgatgacg aattaggttt gaagggtaag 3180

ctagacgata agattaaggg cgctattact gcggcggtga gaaaactaga tcatgaatta 3240

ggtattccag aagatgaaac taagacaagg ggtaagtttc actttttaaa cagaatccat 3300

tacatggcac caagcaatga accatggggt gaacatgaaa ttgattacat cctattttat 3360

aagatcaacg ctaaagaaaa cttgactgtc aacccaaacg tcaatgaagt tagagacttc 3420

aaatgggttt caccaaatga tttgaaaact atgtttgctg acccaagtta caagtttacg 3480

ccttggttta agattatttg cgagaattac ttattcaact ggtgggagca attagatgac 3540

ctttctgaag tggaaaatga caggcaaatt catagaatgc tataaacaaa ttagttaaaa 3600

ggactaaaga tgtcgactct tcctatttct agtgtttcat tctcttcatc tacgtcacct 3660

ttggttgttg acgataaagt tagcacgaaa ccagatgtta tccgccatac catgaatttc 3720

aatgctagca tatggggaga tcaatttctt acatatgatg agccagaaga tttggttatg 3780

aagaaacaac ttgttgaaga gctcaaagag gaagtcaaga aagagctaat aacgatcaaa 3840

ggttcaaatg aaccgatgca gcatgtcaag ttgatagaac ttattgatgc agtccaacgg 3900

cttggcatag cctatcattt tgaagaggag attgaagaag ccttgcaaca tatccatgtt 3960

acatatggcg agcagtgggt cgataaagaa aacctacaaa gcatttctct ttggttccga 4020

ctcctacgac aacaaggctt caacgtctca tcaggagtat tcaaggactt tatggacgag 4080

aagggaaaat ttaaggaatc cttatgtaat gatgctcagg gaattcttgc tctgtatgaa 4140

gcggcattta tgagggtgga agatgaaaca atactagata atgcgctcga gttcactaaa 4200

gttcaccttg acatcatagc caaggatcct tcttgtgact cttccctaag aacccaaata 4260

caccaagcgt taaagcagcc acttaggaga aggctggcaa ggattgaggc gttgcattac 4320

atgcctatct accaacaaga aacatcccac gatgaggtct tactgaagct tgcaaaatta 4380

gattttagcg tgcttcagtc gatgcacaaa aaggaactta gccacatttg caaatggtgg 4440

aaggatttgg acctccaaaa taagcttcct tatgttcgag acagggtggt ggaaggctat 4500

ttttggatac tatccatcta ttacgagcct caacatgctc gaacaagaat gttcctaatg 4560

aaaacatgca tgtggttagt cgttttagat gatacatttg ataattatgg tacttatgaa 4620

gaactcgaaa tctttacaca agctgttgaa agatggtcaa taagctgcct ggatatgctt 4680

ccagaataca tgaaactaat atatcaagag cttgtgaatc ttcacgtgga aatggaggaa 4740

tcacttgaaa aggagggaaa aacatatcaa attcactatg tcaaggagat ggcaaaagag 4800

ttggttcgca attacttggt agaagccaga tggctaaaag aggggtacat gccaactctt 4860

gaggagtaca tgtctgtgtc aatggtgact ggtacctatg gcttgatgat agcgagatct 4920

tatgtcggca ggggtgatat cgtcaccgag gataccttta aatgggtgtc ctcgtatcct 4980

cctattataa aagcttcatg tgtgattgta agacttatgg atgatattgt cagccacaag 5040

gaggaacaag agagaggcca tgttgcttca agcatcgaat gctattctaa ggaaagtggt 5100

gcatcagagg aggaagcgtg tgaatatatc tcaagaaaag ttgaagatgc atggaaagtt 5160

ataaaccgag agtcgctcag gcctacagct gtcccatttc ctctacttat gcctgcaatc 5220

aaccttgcac gtatgtgtga agtcctatat agcgtcaacg atggcttcac tcatgctgag 5280

ggagacatga aaagttacat gaaatcgttc ttcgttcacc ctatggttgt ctaaaaagga 5340

ggagatcata atggcttcag aaaaagaaat taggagagag agattcttga acgttttccc 5400

taaattagta gaggaattga acgcatcgct tttggcttac ggtatgccta aggaagcatg 5460

tgactggtat gcccactcat tgaactacaa cactccaggc ggtaagctaa atagaggttt 5520

gtccgttgtg gacacgtatg ctattctctc caacaagacc gttgaacaat tggggcaaga 5580

agaatacgaa aaggttgcca ttctaggttg gtgcattgag ttgttgcagg cttacttctt 5640

ggtcgccgat gatatgatgg acaagtccat taccagaaga ggccaaccat gttggtacaa 5700

ggttcctgaa gttggggaaa ttgccatcaa tgacgcattc atgttagagg ctgctatcta 5760

caagcttttg aaatctcact tcagaaacga aaaatactac atagatatca ccgaattgtt 5820

ccatgaggtc accttccaaa ccgaattggg ccaattgatg gacttaatca ctgcacctga 5880

agacaaagtc gacttgagta agttctccct aaagaagcac tccttcatag ttactttcaa 5940

gactgcttac tattctttct acttgcctgt cgcattggcc atgtacgttg ccggtatcac 6000

ggatgaaaag gatttgaaac aagccagaga tgtcttgatt ccattgggtg aatacttcca 6060

aattcaagat gactacttag actgcttcgg taccccagaa cagatcggta agatcggtac 6120

agatatccaa gataacaaat gttcttgggt aatcaacaag gcattggaac ttgcttccgc 6180

agaacaaaga aagactttag acgaaaatta cggtaagaag gactcagtcg cagaagccaa 6240

atgcaaaaag attttcaatg acttgaaaat tgaacagcta taccacgaat atgaagagtc 6300

tattgccaag gatttgaagg ccaaaatttc tcaggtcgat gagtctcgtg gcttcaaagc 6360

tgatgtctta actgcgttct tgaacaaagt ttacaagaga agcaaataga ctcaataaat 6420

taaacaactt aaaacagagc aggccttcgg gcctgctttt tctttctttc ccgagtcccg 6480

cctcgggagg gcggtgtcct ttccgccccg aattgcgcat cgggagcacg cggtcctttt 6540

cgctcgcttt gctctctcaa gaggactaat ttacaaaatt acatattgag agcgcggccg 6600

cactagtaag ttatagaaat gtcatccgat accgaaaaag aaaacggaat aaatcagtaa 6660

caaatcaaaa aaacaaaact ataacattta atcttcgtaa aatcaatatc aatcgaccat 6720

acacaaaaca tgttcaaata aaatacatgg gtgtacatat gtcaaccgac attgaaaaag 6780

aaaacggaat aaatcagtaa caaatcaaaa aaacaaaacc ataacatttc tataacattc 6840

catagagtaa aaatcaacat cgaataacct tacacaaacc atacataaac catacataaa 6900

aatacacata tgtcaaccgt taccgtaaaa agattactta aataatttta tactcacctc 6960

ttattttttg tgttttttat tatccagggc aacctttccc agggagaaat tcttccttgc 7020

agttgcaggc aagaaccagg actttcttcc agggcatact ttcccaggga gaaattcttc 7080

cttgcagctg caggcaagaa ccaggacttt cccccaggac caggacctca aggtctgcat 7140

ccagaggaag accttcccag ggcgccaaga tcccgcagat tatggaacat tatttcccgg 7200

gaaataattc agggcttaat gcccaactga ggaatacatt ttaaataagc ttctcttagt 7260

tcatcctttt ctatgtggtc gtacacgtct attgcttcgc ctctcgaatc tccccttaat 7320

tcctgaatga atgtcctttc catacctgaa cgtcgaagcc atgtagtgaa ccaatgccgg 7380

cagcaatgcg gagtaaaatg ttcttcaagt ctcccctcgg ggttatggag tccgatagag 7440

gaagcggcag atgtgacaat atcgtacact aaatttcttt gtacacggtg accgttttgt 7500

ctgcctatga ataatgcttt ttctttatca tgcctcgtgt taaggtacgc ttccaggaag 7560

gccttagtct caaggtcaaa gaatacgata cgatttgacc ttttagctgt cttcttgagt 7620

ctcaccgtac acttttccag atctatgtca gctcgatcaa gagtaattaa ttcatttctc 7680

ctgatgccag ttttagctaa aaagattatc aatgcttgat acatggggta acgtggtact 7740

ttgacaagct ttgccatgtc ttcaatggta atcaattgcc ttgtttccgg agtatggtat 7800

tttttgtagc ttcttagata gcgttttctg aacgtgggaa tgttgtttct ctcgattata 7860

tgcttgaatt caaggaagtc aaaaaaggta ctcagtgaag caaagtaatt gttacaggtt 7920

gcgagtgtgt actgcctttc atctcttatg tgaacaagga acccttcaag ctccggaaac 7980

cctgcattca ctccacacat ttgaaggaaa aacttagcat gacttacgta tccttctatt 8040

gttcttttag agtaacccct tccgatacaa tcagaagtga ataaacttat aagtctttca 8100

ttatcaagcg gttcatagat accagttagc tgaatagctg ccatttcacc cataaatcaa 8160

taaaagaagt atgtaaataa atactttgtg catagatgtc atttaaacac aaagtataag 8220

tacatctacc ttcttttagt atatgctggt aacagcaaaa aagcgccaaa caacgcatct 8280

ttacccaata gaatgacgtc gattaaaaga gtaagttggt ggtgagcctt aaaaaaggta 8340

aaagagagct tactcttaag ttaaaaacgg aattggcaaa cagtaaggat tgttccagca 8400

gtccttacta aaaacattac tttattaatt ctatatagtc acaacattca caaaaatagg 8460

cttttttctc gatttttcct tttgaatgtt tataaagaaa aatcatatag catttattac 8520

actgtggaca tattttcttt tcaattttgc cccttccaat ttgtggaagc cttttttcag 8580

taacacgctt aggcataatg aaagatcggt gtattaagta ttaacatttt ctaacttgtg 8640

caataataat tattcggggt tgtcgaagaa cttccaaaca gtagaccgat ccattttcca 8700

cattattcta atgaagccaa gactcaagga tggggggaaa tatcccccca caccccccaa 8760

tgagactacc caagacagcc cgtatgccga cagcgacaca gttaaatatc aatgtgtatt 8820

aatacatatc aacaagtatc aacacacaca attaaacaat aatacacatc aatggagaac 8880

aatacacatg ccaacacaag aatacacacg gacaaacatt acattaccag aacatgttag 8940

agcaatgatt aaagagtaca ataaaagaag tacatgggga gacctcaacg tatcagcagt 9000

atgctctaat gcaatagcag acagaattag agctacattc ccaaaattag aaaaagaact 9060

atttccagaa caatttccag aacttaaccc aaaaatgaca aaaaccagct caccaggaca 9120

accagaaata acacaaaaag gaaaatccaa acaaggagac tctaaagata gaatatgcgc 9180

ttattgtggc caaccttttg aaccgaaatc gcataatcaa aaattctgta aagatgcatg 9240

taagtcagca aattatagaa aaaacaaaaa aacacagagt taaacccatt aaccacactc 9300

tttttttgaa tttatctgca tttcacggta ctttttatcc aagaaagccc acaaatcacg 9360

ctctttaaac ggtttcatgg aaaaattttc ccggaatgca ttaattaaca tcttttcaat 9420

tttagggttg aaataagccc cataacggta atctggcgac caatctgaag ctttaacctc 9480

ttcataagtc cgatacggca cattacccgg aagaagccca ggaaccacaa gcagataata 9540

gatatagcac cttatacaca tatgtgattt tttagtagtc tttaaccatt caatatatga 9600

aagatccgca cctgttcgat aatcctcaaa aagaattaag ttcctttgcc tccgagatcc 9660

acacacacag caatacttca attccataat acataacacc ccgaaataat aagacatctg 9720

aaaaaccgta tcactttagg ctttaagcat cttatccgga ccttcgagcc caaaagcctt 9780

gttgaacgtg gcatctcaat cacccccaaa catcatggag cgtgatttta agcttgtaaa 9840

tagctcttca taattaattt cggaagcctt ttgcaagctg ttagaaggca tttcaggcga 9900

ttcttcaatt atacagccat catgctccca ttgatgagag tcaaccacca ggaagccggc 9960

agggggatca ggaagccctt ctaagaacca tacccgaaga agactcccgg caggttcaag 10020

cttcaaaaac tccggtaatt catccacttt gaagggagaa gcccataatt tacgccattt 10080

tttaggaact ttgtttttgc catctcgccc ttttaggtta tgataaaaat tatttcttgc 10140

aattaattgt tgcaagtaat cggcatattc aggtttaacc gctttagctt ccttcaaata 10200

agcatgtaga gcctgtcttc cacccgaaaa atgatcccga agctttgtta acggtatcca 10260

tccagtatca tcgttttcct gtccttccca cttcaactct ccattataaa cgagttttac 10320

ttctcgacct tcattgatta aattaaggac ttcggttctg gtatctatca attgctcaaa 10380

tgtcagcttt ccggaggaac ttacaacttt atcaggatgc caccggaaca aagcaccaaa 10440

gcgagattct atatggatat taccgtatct gttatccgtg agctgcccaa cgtgagaata 10500

aagataagtc acctgcttgt aaatatccat aaggctccgc aattcccatt gccttttacc 10560

attgacttta tgagaaagcc tctgaataaa tatatcatat tcatcactct tgcatctctt 10620

tttactcttc ggattctttc cagttatacg aatgtttcca gggaaaatga aaaaatgaaa 10680

atgaggagca aggataaggc agtcataaat agagtttatt tcgatattta gcaagtccgc 10740

gatcttgttt aaattgcctt cgttgtggtc gtctcttatg taattccaaa aagcttcgga 10800

gtcgtcatta ccttttaaac ctgttaactt acggatagcc cgagttattt ccggcttaat 10860

cctgtaaggg tgaaagattg tagtaccagc agttacgccc cagcgttcca gcctgtcctt 10920

taaattcctc cggaaaagtc ggattgcttc cacagatact ccccggctaa agtccatcga 10980

agcaaacccc catgaaggaa actgcccaga aaacagggca taagtccaca aaattacaac 11040

taatgcaaat ctatgttgca attccgatag gatgccgcaa ccaggacaag aaagtgtatt 11100

gcagtgagaa ttaagatcct ttcgtgcatt ataattttca gatacagcat aagtgaaagg 11160

acgcccacag taagcgggtc tttttccctt tgctggaaga acaaagtcgc caacttttag 11220

aggacgatac ttagagcatt tagaaaaatc gaagtcagga caagagtagt atttacagat 11280

agaaaccccg ttctttttgt tgcgaaccgg gcacaaaaca ccatcggtac atatacacgt 11340

cgcaaaatct ctacactctg gagcatagga aacggcgcca ggtctagaac taaaatcaga 11400

actcagagaa catttcctgt cgaaaattta aaccgtaaac caaagataac aattttcata 11460

atattacaga acctatattt tttgtaactc gacattacaa aaatgtacca caaaacataa 11520

aaacattttc atagaagaaa tattcacaaa caatagaaaa aaattataag aataagatta 11580

acgcctacct tattaaaaga aggtgtcgag tcttcaggtt ccatcaaggc aggcgttgat 11640

attctatata tgttacacat aacatatata acatacgtac ctcagaacat tcagacctga 11700

atgctctaaa atgaaataag ctttaaactt tataaattca tctgctataa atagttttag 11760

agatatagaa agcccaagga agaaaaagaa tagcaaaaat aaaaaagaaa aacggcccgc 11820

gacggcaaac ccggagactt ataatcaaag taagacgaaa aggaagccga caacgatttg 11880

ataataacaa atcatctttt attttggcaa tattcatatg atatatgaag aaatcaacat 11940

caagcggatt agtatttttg ctatttgtaa atcaatatcg ggtgagacag tatatcaaaa 12000

acaaacttca tttttttcga acaaaaaaat cactatcaaa tgacattgta gtaagaaact 12060

acatcaaaac tagttgatca tatcgtcaat tattacctcc acggggagag cctgagcaaa 12120

ctggcctcag gcatttgaga agcacacggt cacactgctt ccggtagtca ataaaccggt 12180

aaaccagcaa tagacataag cggctattta acgaccctgc cctgaaccga cgaccgggtc 12240

gaatttgctt tcgaatttct gccattcatc cgcttattat cacttattca ggcgtagcac 12300

caggcgttta agggcaccaa taactgcctt aaaaaaatta cgccccgccc tgccactcat 12360

cgcagtactg ttgtaattca ttaagcattc tgccgacatg gaagccatca cagacggcat 12420

gatgaacctg aatcgccagc ggcatcagca ccttgtcgcc ttgcgtataa tatttgccca 12480

tggtgaaaac gggggcgaag aagttgtcca tattggccac gtttaaatca aaactggtga 12540

aactcaccca gggattggct gagacgaaaa acatattctc aataaaccct ttagggaaat 12600

aggccaggtt ttcaccgtaa cacgccacat cttgcgaata tatgtgtaga aactgccgga 12660

aatcgtcgtg gtattcactc cagagcgatg aaaacgtttc agtttgctca tggaaaacgg 12720

tgtaacaagg gtgaacacta tcccatatca ccagctcacc gtctttcatt gccatacg 12778

<210> 15
<211> 10722
<212> DNA
<213> Artificial

<220>
<223> pAC:Pmcr-IspS

<400> 15
gaattccgga tgagcattca tcaggcgggc aagaatgtga ataaaggccg gataaaactt 60

gtgcttattt ttctttacgg tctttaaaaa ggccgtaata tccagctgaa cggtctggtt 120

ataggtacat tgagcaactg actgaaatgc ctcaaaatgt tctttacgat gccattggga 180

tatatcaacg gtggtatatc cagtgatttt tttctccatt ttagcttcct tagctcctga 240

aaatctcgat aactcaaaaa atacgcccgg tagtgatctt atttcattat ggtgaaagtt 300

ggaacctctt acgtgccgat caacgtctca ttttcgccaa aagttggccc agggcttccc 360

ggtatcaaca gggacaccag gatttattta ttctgcgaag tgatcttccg tcacaggtat 420

ttattcggcg caaagtgcgt cgggtgatgc tgccaactta ctgatttagt gtatgatggt 480

gtttttgagg tgctccagtg gcttctgttt ctatcagctg tccctcctgt tcagctactg 540

acggggtggt gcgtaacggc aaaagcaccg ccggacatca gcgctagcgg agtgtatact 600

ggcttactat gttggcactg atgagggtgt cagtgaagtg cttcatgtgg caggagaaaa 660

aaggctgcac cggtgcgtca gcagaatatg tgatacagga tatattccgc ttcctcgctc 720

actgactcgc tacgctcggt cgttcgactg cggcgagcgg aaatggctta cgaacggggc 780

ggagatttcc tggaagatgc caggaagata cttaacaggg aagtgagagg gccgcggcaa 840

agccgttttt ccataggctc cgcccccctg acaagcatca cgaaatctga cgctcaaatc 900

agtggtggcg aaacccgaca ggactataaa gataccaggc gtttccccct ggcggctccc 960

tcgtgcgctc tcctgttcct gcctttcggt ttaccggtgt cattccgctg ttatggccgc 1020

gtttgtctca ttccacgcct gacactcagt tccgggtagg cagttcgctc caagctggac 1080

tgtatgcacg aaccccccgt tcagtccgac cgctgcgcct tatccggtaa ctatcgtctt 1140

gagtccaacc cggaaagaca tgcaaaagca ccactggcag cagccactgg taattgattt 1200

agaggagtta gtcttgaagt catgcgccgg ttaaggctaa actgaaagga caagttttgg 1260

tgactgcgct cctccaagcc agttacctcg gttcaaagag ttggtagctc agagaacctt 1320

cgaaaaaccg ccctgcaagg cggttttttc gttttcagag caagagatta cgcgcagacc 1380

aaaacgatct caagaagatc atcttattaa tcagataaaa tatttctaga tttcagtgca 1440

atttatctct tcaaatgtag cacctgaagt cagccccata cgatataagt tgtaattctc 1500

atgtttgaca gcttatcatc gataagcttg catgcatctt ctattttcat cttaatttta 1560

tatattaaag gctaattttt caatatttta tcgcaaacta ttgtgttaat tacatcaaat 1620

atataaactt ttctatttaa agctaatgaa aaagtgaata tatacacata gagtaatgtt 1680

atgatgtata tatcaaaaaa ataggagtga ttcctatgac cgagtacaag cccacggtgc 1740

gcctcgccac ccgcgacgac gtcccccggg ccgtacgcac cctcgccgcc gcgttcgccg 1800

actaccccgc cacgcgccac accgtcgacc cggaccgcca catcgagcgg gtcaccgagc 1860

tgcaagaact cttcctcacg cgcgtcgggc tcgacatcgg caaggtgtgg gtcgcggacg 1920

acggcgccgc ggtggcggtc tggaccacgc cggagagcgt cgaagcgggg gcggtgttcg 1980

ccgagatcgg cccgcgcatg gccgagttga gcggttcccg gctggccgcg cagcaacaga 2040

tggaaggcct cctggcgccg caccggccca aggagcccgc gtggttcctg gccaccgtcg 2100

gcgtctcgcc cgaccaccag ggcaagggtc tgggcagcgc cgtcgtgctc cccggagtgg 2160

aggcggccga gcgcgccggg gtgcccgcct tcctggagac ctccgcgccc cgcaacctcc 2220

ccttctacga gcggctcggc ttcaccgtca ccgccgacgt cgaggtgccc gaaggaccgc 2280

gcacctggtg catgacccgc aagcccggtg cctgagttaa ttactaattt attattaatt 2340

tattattaga ttgggcaaaa tagtaaaaga aaactaaagg aaacctaata tggtttcctt 2400

tttttatata tttttaaaaa ttgattatat tatatgtaaa caattataat taatgttaat 2460

cgaattcaat tttttattaa taaacggcgc gccacgcgtc cttcaattgc cgcgatcgtt 2520

aattaaatga tttttaaaaa aatacatgaa ttcatctagc ggagaacaca aaagatttaa 2580

gtaccttcta aacgaatgag atttcattgg gaaagtggac acttaagaac gacgcggtac 2640

ttgatttatt gagtgcaaaa gcactcgatt aggtgaccag tcccaaagtg attttaataa 2700

attaaggagg aaattaaaat ggaagccaga cggtctgcca attatgaacc aaatagctgg 2760

gattatgatt ttttgctgtc ttcagacact gacgaatcga ttgaagtata caaagacaag 2820

gccaaaaagc tggaggctga ggtgagaaga gagattaaca atgaaaaggc agagtttttg 2880

actctgcttg aactgataga taatgtccaa aggttaggat tgggttaccg gttcgagagt 2940

gacataagga gagccctcga cagatttgtt tcttcaggag gatttgatgg tgttacaaaa 3000

actagccttc atgctactgc tcttagcttc aggcttctca gacagcatgg ctttgaggtc 3060

tctcaagaag cgttcagtgg attcaaggat caaaatggca atttcttgga aaaccttaag 3120

gaggacacca aggcaatact aagcctatat gaagcttcat ttcttgcatt agaaggagaa 3180

aatatcttgg atgaggccag ggtgtttgca atatcacatc taaaagagct cagcgaagaa 3240

aagattggaa aagagctggc cgaacaggtg aatcatgcat tggagcttcc attgcatcgc 3300

aggacgcaaa gactagaagc tgtttggagt attgaagcat accgtaaaaa ggaagatgca 3360

aatcaagtac tgctagaact tgctatattg gactacaaca tgattcaatc agtataccaa 3420

agagatcttc gcgagacatc aaggtggtgg aggcgagtgg gtcttgcaac aaagttgcat 3480

tttgctaaag acaggttaat tgaaagcttt tactgggcag ttggagttgc gttcgaacct 3540

caatacagtg attgccgtaa ttcagtagca aaaatgtttt catttgtaac aatcattgat 3600

gatatctatg atgtttatgg tactctggat gagctggagc tatttacaga tgctgttgag 3660

agatgggatg ttaacgccat caatgatctt ccggattata tgaagctctg cttcctagct 3720

ctctacaaca ctatcaatga gatagcttat gacaatctga aggacaaggg ggaaaacatt 3780

cttccatacc taacaaaagc gtgggcagat ttatgcaatg cattcctaca agaagcaaaa 3840

tggctgtaca ataagtccac accaacattt gatgactatt tcggaaatgc atggaaatca 3900

tcctcagggc ctcttcaact aatttttgcc tactttgccg tggttcaaaa catcaagaaa 3960

gaggaaattg aaaacttaca aaagtatcat gatatcatca gtaggccttc ccacatcttt 4020

cgtctttgca acgacctggc ttcagcatcg gctgagatag cgagaggtga aactgcgaat 4080

tccgtatcct gctacatgcg tacaaaaggc atttctgagg aacttgctac tgaatccgta 4140

atgaatttga tcgacgaaac ctgtaaaaag atgaacaaag aaaagcttgg tggctctttg 4200

tttgcaaaac cttttgtcga aacagctatt aaccttgcac ggcaatccca ttgcacttat 4260

cataacggag atgcgcatac ttcaccagac gagctaacta ggaaacgtgt cctgtcagta 4320

atcacagagc ctattctacc ctttgagaga taaactcaat aaattaaaca acttaaaaca 4380

gagcaggcct tcgggcctgc tttttctttc tttcccgagt cccgcctcgg gagggcggtg 4440

tcctttccgc cccgaattgc gcatcgggag cacgcggtcc ttttcgctcg ctttgctctc 4500

tcaagaggac taatttacaa aattacatat tgagagcgcg gccgcactag taagttatag 4560

aaatgtcatc cgataccgaa aaagaaaacg gaataaatca gtaacaaatc aaaaaaacaa 4620

aactataaca tttaatcttc gtaaaatcaa tatcaatcga ccatacacaa aacatgttca 4680

aataaaatac atgggtgtac atatgtcaac cgacattgaa aaagaaaacg gaataaatca 4740

gtaacaaatc aaaaaaacaa aaccataaca tttctataac attccataga gtaaaaatca 4800

acatcgaata accttacaca aaccatacat aaaccataca taaaaataca catatgtcaa 4860

ccgttaccgt aaaaagatta cttaaataat tttatactca cctcttattt tttgtgtttt 4920

ttattatcca gggcaacctt tcccagggag aaattcttcc ttgcagttgc aggcaagaac 4980

caggactttc ttccagggca tactttccca gggagaaatt cttccttgca gctgcaggca 5040

agaaccagga ctttccccca ggaccaggac ctcaaggtct gcatccagag gaagaccttc 5100

ccagggcgcc aagatcccgc agattatgga acattatttc ccgggaaata attcagggct 5160

taatgcccaa ctgaggaata cattttaaat aagcttctct tagttcatcc ttttctatgt 5220

ggtcgtacac gtctattgct tcgcctctcg aatctcccct taattcctga atgaatgtcc 5280

tttccatacc tgaacgtcga agccatgtag tgaaccaatg ccggcagcaa tgcggagtaa 5340

aatgttcttc aagtctcccc tcggggttat ggagtccgat agaggaagcg gcagatgtga 5400

caatatcgta cactaaattt ctttgtacac ggtgaccgtt ttgtctgcct atgaataatg 5460

ctttttcttt atcatgcctc gtgttaaggt acgcttccag gaaggcctta gtctcaaggt 5520

caaagaatac gatacgattt gaccttttag ctgtcttctt gagtctcacc gtacactttt 5580

ccagatctat gtcagctcga tcaagagtaa ttaattcatt tctcctgatg ccagttttag 5640

ctaaaaagat tatcaatgct tgatacatgg ggtaacgtgg tactttgaca agctttgcca 5700

tgtcttcaat ggtaatcaat tgccttgttt ccggagtatg gtattttttg tagcttctta 5760

gatagcgttt tctgaacgtg ggaatgttgt ttctctcgat tatatgcttg aattcaagga 5820

agtcaaaaaa ggtactcagt gaagcaaagt aattgttaca ggttgcgagt gtgtactgcc 5880

tttcatctct tatgtgaaca aggaaccctt caagctccgg aaaccctgca ttcactccac 5940

acatttgaag gaaaaactta gcatgactta cgtatccttc tattgttctt ttagagtaac 6000

cccttccgat acaatcagaa gtgaataaac ttataagtct ttcattatca agcggttcat 6060

agataccagt tagctgaata gctgccattt cacccataaa tcaataaaag aagtatgtaa 6120

ataaatactt tgtgcataga tgtcatttaa acacaaagta taagtacatc taccttcttt 6180

tagtatatgc tggtaacagc aaaaaagcgc caaacaacgc atctttaccc aatagaatga 6240

cgtcgattaa aagagtaagt tggtggtgag ccttaaaaaa ggtaaaagag agcttactct 6300

taagttaaaa acggaattgg caaacagtaa ggattgttcc agcagtcctt actaaaaaca 6360

ttactttatt aattctatat agtcacaaca ttcacaaaaa taggcttttt tctcgatttt 6420

tccttttgaa tgtttataaa gaaaaatcat atagcattta ttacactgtg gacatatttt 6480

cttttcaatt ttgccccttc caatttgtgg aagccttttt tcagtaacac gcttaggcat 6540
aatgaaagat cggtgtatta agtattaaca ttttctaact tgtgcaataa taattattcg 6600
gggttgtcga agaacttcca aacagtagac cgatccattt tccacattat tctaatgaag 6660
ccaagactca aggatggggg gaaatatccc cccacacccc ccaatgagac tacccaagac 6720
agcccgtatg ccgacagcga cacagttaaa tatcaatgtg tattaataca tatcaacaag 6780
tatcaacaca cacaattaaa caataataca catcaatgga gaacaataca catgccaaca 6840
caagaataca cacggacaaa cattacatta ccagaacatg ttagagcaat gattaaagag 6900
tacaataaaa gaagtacatg gggagacctc aacgtatcag cagtatgctc taatgcaata 6960
gcagacagaa ttagagctac attcccaaaa ttagaaaaag aactatttcc agaacaattt 7020
ccagaactta acccaaaaat gacaaaaacc agctcaccag gacaaccaga aataacacaa 7080
aaaggaaaat ccaaacaagg agactctaaa gatagaatat gcgcttattg tggccaacct 7140
tttgaaccga aatcgcataa tcaaaaattc tgtaaagatg catgtaagtc agcaaattat 7200
agaaaaaaca aaaaaacaca gagttaaacc cattaaccac actctttttt tgaatttatc 7260
tgcatttcac ggtacttttt atccaagaaa gcccacaaat cacgctcttt aaacggtttc 7320
atggaaaaat tttcccggaa tgcattaatt aacatctttt caattttagg gttgaaataa 7380
gccccataac ggtaatctgg cgaccaatct gaagctttaa cctcttcata agtccgatac 7440
ggcacattac ccggaagaag cccaggaacc acaagcagat aatagatata gcaccttata 7500
cacatatgtg attttttagt agtctttaac cattcaatat atgaaagatc cgcacctgtt 7560
cgataatcct caaaaagaat taagttcctt tgcctccgag atccacacac acagcaatac 7620
ttcaattcca taatacataa caccccgaaa taataagaca tctgaaaaac cgtatcactt 7680
taggctttaa gcatcttatc cggaccttcg agcccaaaag ccttgttgaa cgtggcatct 7740
caatcacccc caaacatcat ggagcgtgat tttaagcttg taaatagctc ttcataatta 7800
atttcggaag ccttttgcaa gctgttagaa ggcatttcag gcgattcttc aattatacag 7860
ccatcatgct cccattgatg agagtcaacc accaggaagc cggcaggggg atcaggaagc 7920
ccttctaaga accatacccg aagaagactc ccggcaggtt caagcttcaa aaactccggt 7980
aattcatcca ctttgaaggg agaagcccat aatttacgcc attttttagg aactttgttt 8040
ttgccatctc gcccttttag gttatgataa aaattatttc ttgcaattaa ttgttgcaag 8100
taatcggcat attcaggttt aaccgcttta gcttccttca aataagcatg tagagcctgt 8160
cttccacccg aaaaatgatc ccgaagcttt gttaacggta tccatccagt atcatcgttt 8220
tcctgtcctt cccacttcaa ctctccatta taaacgagtt ttacttctcg accttcattg 8280
attaaattaa ggacttcggt tctggtatct atcaattgct caaatgtcag ctttccggag 8340
gaacttacaa ctttatcagg atgccaccgg aacaaagcac caaagcgaga ttctatatgg 8400

atattaccgt atctgttatc cgtgagctgc ccaacgtgag aataaagata agtcacctgc 8460

ttgtaaatat ccataaggct ccgcaattcc cattgccttt taccattgac tttatgagaa 8520

agcctctgaa taaatatatc atattcatca ctcttgcatc tctttttact cttcggattc 8580

tttccagtta tacgaatgtt tccagggaaa atgaaaaaat gaaaatgagg agcaaggata 8640

aggcagtcat aaatagagtt tatttcgata tttagcaagt ccgcgatctt gtttaaattg 8700

ccttcgttgt ggtcgtctct tatgtaattc caaaaagctt cggagtcgtc attacctttt 8760

aaacctgtta acttacggat agcccgagtt atttccggct taatcctgta agggtgaaag 8820

attgtagtac cagcagttac gccccagcgt tccagcctgt cctttaaatt cctccggaaa 8880

agtcggattg cttccacaga tactccccgg ctaaagtcca tcgaagcaaa cccccatgaa 8940

ggaaactgcc cagaaaacag ggcataagtc cacaaaatta caactaatgc aaatctatgt 9000

tgcaattccg ataggatgcc gcaaccagga caagaaagtg tattgcagtg agaattaaga 9060

tcctttcgtg cattataatt ttcagataca gcataagtga aaggacgccc acagtaagcg 9120

ggtctttttc cctttgctgg aagaacaaag tcgccaactt ttagaggacg atacttagag 9180

catttagaaa aatcgaagtc aggacaagag tagtatttac agatagaaac cccgttcttt 9240

ttgttgcgaa ccgggcacaa aacaccatcg gtacatatac acgtcgcaaa atctctacac 9300

tctggagcat aggaaacggc gccaggtcta gaactaaaat cagaactcag agaacatttc 9360

ctgtcgaaaa tttaaaccgt aaaccaaaga taacaatttt cataatatta cagaacctat 9420

attttttgta actcgacatt acaaaaatgt accacaaaac ataaaaacat tttcatagaa 9480

gaaatattca caaacaatag aaaaaaatta taagaataag attaacgcct accttattaa 9540

aagaaggtgt cgagtcttca ggttccatca aggcaggcgt tgatattcta tatatgttac 9600

acataacata tataacatac gtacctcaga acattcagac ctgaatgctc taaaatgaaa 9660

taagctttaa actttataaa ttcatctgct ataaatagtt ttagagatat agaaagccca 9720

aggaagaaaa agaatagcaa aaataaaaaa gaaaaacggc ccgcgacggc aaacccggag 9780

acttataatc aaagtaagac gaaaaggaag ccgacaacga tttgataata acaaatcatc 9840

ttttattttg gcaatattca tatgatatat gaagaaatca acatcaagcg gattagtatt 9900

tttgctattt gtaaatcaat atcgggtgag acagtatatc aaaaacaaac ttcatttttt 9960

tcgaacaaaa aaatcactat caaatgacat tgtagtaaga aactacatca aaactagttg 10020

atcatatcgt caattattac ctccacgggg agagcctgag caaactggcc tcaggcattt 10080

gagaagcaca cggtcacact gcttccggta gtcaataaac cggtaaacca gcaatagaca 10140

taagcggcta tttaacgacc ctgccctgaa ccgacgaccg ggtcgaattt gctttcgaat 10200

ttctgccatt catccgctta ttatcactta ttcaggcgta gcaccaggcg tttaagggca 10260

ccaataactg ccttaaaaaa attacgcccc gccctgccac tcatcgcagt actgttgtaa 10320

ttcattaagc attctgccga catggaagcc atcacagacg gcatgatgaa cctgaatcgc 10380

cagcggcatc agcaccttgt cgccttgcgt ataatatttg cccatggtga aaacgggggc 10440

gaagaagttg tccatattgg ccacgtttaa atcaaaactg gtgaaactca cccagggatt 10500

ggctgagacg aaaaacatat tctcaataaa ccctttaggg aaataggcca ggttttcacc 10560

gtaacacgcc acatcttgcg aatatatgtg tagaaactgc cggaaatcgt cgtggtattc 10620

actccagagc gatgaaaacg tttcagtttg ctcatggaaa acggtgtaac aagggtgaac 10680

actatcccat atcaccagct caccgtcttt cattgccata cg 10722

<210> 16
<211> 14004
<212> DNA
<213> Artificial

<220>
<223> pAC:Pmcr-MVA1-IDI-IspS

<400> 16
   tttagcttcc ttagctcctg aaaatctcga taactcaaaa aatacgcccg gtagtgatct 60
   tatttcatta tggtgaaagt tggaacctct tacgtgccga tcaacgtctc attttcgcca 120
   aaagttggcc cagggcttcc cggtatcaac agggacacca ggatttattt attctgcgaa 180
   gtgatcttcc gtcacaggta tttattcggc gcaaagtgcg tcgggtgatg ctgccaactt 240
   actgatttag tgtatgatgg tgtttttgag gtgctccagt ggcttctgtt tctatcagct 300
   gtccctcctg ttcagctact gacggggtgg tgcgtaacgg caaaagcacc gccggacatc 360
   agcgctagcg gagtgtatac tggcttacta tgttggcact gatgagggtg tcagtgaagt 420
   gcttcatgtg gcaggagaaa aaaggctgca ccggtgcgtc agcagaatat gtgatacagg 480
   atatattccg cttcctcgct cactgactcg ctacgctcgg tcgttcgact gcggcgagcg 540
   gaaatggctt acgaacgggg cggagatttc ctggaagatg ccaggaagat acttaacagg 600
   gaagtgagag ggccgcggca aagccgtttt tccataggct ccgcccccct gacaagcatc 660
   acgaaatctg acgctcaaat cagtggtggc gaaacccgac aggactataa agataccagg 720
   cgtttccccc tggcggctcc ctcgtgcgct ctcctgttcc tgcctttcgg tttaccggtg 780
   tcattccgct gttatggccg cgtttgtctc attccacgcc tgacactcag ttccgggtag 840
   gcagttcgct ccaagctgga ctgtatgcac gaaccccccg ttcagtccga ccgctgcgcc 900
   ttatccggta actatcgtct tgagtccaac ccggaaagac atgcaaaagc accactggca 960
   gcagccactg gtaattgatt tagaggagtt agtcttgaag tcatgcgccg gttaaggcta 1020
   aactgaaagg acaagttttg gtgactgcgc tcctccaagc cagttacctc ggttcaaaga 1080
   gttggtagct cagagaacct tcgaaaaacc gccctgcaag gcggtttttt cgttttcaga 1140
   gcaagagatt acgcgcagac caaaacgatc tcaagaagat catcttatta atcagataaa 1200
   atatttctag atttcagtgc aatttatctc ttcaaatgta gcacctgaag tcagccccat 1260
   acgatataag ttgtaattct catgtttgac agcttatcat cgataagctt gcatgcatct 1320
   tctattttca tcttaatttt atatattaaa ggctaatttt tcaatatttt atcgcaaact 1380
   attgtgttaa ttacatcaaa tatataaact tttctattta aagctaatga aaaagtgaat 1440
   atatacacat agagtaatgt tatgatgtat atatcaaaaa aataggagtg attcctatga 1500
   ccgagtacaa gcccacggtg cgcctcgcca cccgcgacga cgtcccccgg gccgtacgca 1560
   ccctcgccgc cgcgttcgcc gactaccccg ccacgcgcca caccgtcgac ccggaccgcc 1620
   acatcgagcg ggtcaccgag ctgcaagaac tcttcctcac gcgcgtcggg ctcgacatcg 1680
   gcaaggtgtg ggtcgcggac gacggcgccg cggtggcggt ctggaccacg ccggagagcg 1740
   tcgaagcggg ggcggtgttc gccgagatcg gcccgcgcat ggccgagttg agcggttccc 1800
   ggctggccgc gcagcaacag atggaaggcc tcctggcgcc gcaccggccc aaggagcccg 1860
   cgtggttcct ggccaccgtc ggcgtctcgc ccgaccacca gggcaagggt ctgggcagcg 1920
   ccgtcgtgct ccccggagtg gaggcggccg agcgcgccgg ggtgcccgcc ttcctggaga 1980
   cctccgcgcc ccgcaacctc cccttctacg agcggctcgg cttcaccgtc accgccgacg 2040
   tcgaggtgcc cgaaggaccg cgcacctggt gcatgacccg caagcccggt gcctgagtta 2100
   attactaatt tattattaat ttattattag attgggcaaa atagtaaaag aaaactaaag 2160
   gaaacctaat atggtttcct ttttttatat atttttaaaa attgattata ttatatgtaa 2220
   acaattataa ttaatgttaa tcgaattcaa tttttattta ataaacggcg cgccacgcgt 2280
   ccttcaattg ccgcgatcgt taattaaatg atttttaaaa aaatacatga attcatctag 2340
   cggagaacac aaaagattta agtaccttct aaacgaatga gatttcattg ggaaagtgga 2400
   cacttaagaa cgacgcggta cttgatttat tgagtgcaaa agcactcgat taggtgacca 2460
   gtcccaaagt gattttaata aattaaggag gaaattaaaa tgaggaagag aataaaaaga 2520
   tcaaccggtg atttcatgtt tttaaatgat tatgaacttg gtgaggaaga aaagcttctt 2580
   ttacagaagg ttcttgatgg agatatagcc tttcgtaaga tcgaagagtt tgcagagccg 2640
   ctaacagccg taaaaattcg gagacttgct attcaggagt acgcaaaact cgaatttgaa 2700
   cacatccaga acttctcttt agatgtagaa attgtgacaa agagaaacat tgaaaacatg 2760
   ataggagcag ttcagatccc tctggggact gccggccttc tgaaagtaaa cggggaatat 2820
   gcagacgccg aatactatat ccctctggct acaacggaag gagcccttgt tgccagtgta 2880
   aaccgcggat gttcggtcat cacaaagtca ggcggggcaa atgtcagagt gtttgaagat 2940
   gaaatgacca gagctccggt cttcaagctt gaaagccttg acagagccaa gaaattttat 3000
   gaatgggtaa agcgtcccga gattttcgag cagatgaaag aggttgctga aaagacgacc 3060
   aggtttggca agctggtttc ggtaaagcct ttcgttaccg ggacgtatgt ctatctccgg 3120
   ttttcctatg acacaaaaga tgcgatgggc atgaatatgg tgaccatagc cactgatgcg 3180
   gtgatgcacc tcatagaaga cgaatttggc gcgcatccca ttaccctttc gagcaatatg 3240
   tgcacagaca aaaaacctgc gtccataagt accattctcg ggagaggaaa gaccgtggtt 3300
   gccgaagtaa ctatccctga agagatagtc aaggagactc tcaagtgtac ccctgagtca 3360
   atgtttgaag tgaattacag taaaaacctg cttggttcgg caagagccgg agccatggga 3420
   tttaatgccc atgctgcaaa cgtcatcgct gctctttacc ttgcctgcgg gcaggacgct 3480
   gcccacgtcg ttgaaggcag caccgcaatt acgagtatgg aacttacaaa atacggggag 3540
   atacactgct cggtcaccct gccagctctt cctgtcggca cggtgggcgg aggcaccggc 3600
   cttggaaccc agagggattg cctcaatatc cttggggtgg caggagcagg agacgagccc 3660
   ggcataaact cgctaaagtt tgcagaaatc gtggcctctg cagtacttgc aggggagatt 3720
   tcccttatag gggcacaggc agccggacac ctggcacgcg cacatgcaca actcggacgc 3780
   ggaaagttct gaacaaatta gttaaaaggt ctaaagatga ctatcggaat tgtatcttac 3840
   ggtgcttatg tcccgagata ccggataaaa attgaagaaa tcgcccggct ctggggtgat 3900
   gacgccgagg ctctcaaaaa tggcctcatg gtgtacgaaa agtccgtgcc tgatgtcgac 3960
   gaagacgcag caacgattgc agtggaagcg gcaagatatg caatggcaag aagcggcgta 4020
   gatccctcca gaatcggggc ggtgtacacg ggttcagaaa gtcatcccta cgctgtaaaa 4080
   cctacaagca ccatcgtcgc ccaggctatc ggcgcaaccc ccgaaatgac tgcagcagac 4140
   tttgagtttg cctgtaaagc aggaacagcc gcagttcagg cctgtatggg cctcgtgggc 4200
   tcgggaatga tcgaccttgg cctggccatc ggagcagacg tttcccaggg agccccaagc 4260
   gatgcccttg aatacactgc agctgcaggc ggagttgcct gcctgatcgg aagaaaagaa 4320
   tccgaacttg ccgcaatcat tgaagataca tactctttta caacggatac ccctgacttc 4380
   tggagaaggg aaggaatgcc ctatcccgaa catggaggca ggtttacggg agagcccgga 4440
   tacttcaagc acgtgacaaa cggcgcaaaa gggctgcttg aaaaactcgg gacaaaacct 4500
   gaagattatg attatgcagt cttccaccag ccaaacggga aattccctag caaagctgca 4560
   aagatactcg ggtttacaaa agcccagatt actcccggac ttgttgtccc gaaaataggg 4620
   aacacctatt ctggttcctg cctgatggga attgctgcaa cccttgacca ggcaaagccc 4680
   ggagaccgta tttttgcaac cgccttcggg tcaggagcag ggtccgacgc gttcagcata 4740
   acagttaccg acaggattga ggaaatccga aatagggctc cgaaggtttc cgaacttatc 4800
   aaggatcccg tatacatcga ctacgcaagg tatgccaggc ataaaggcaa gatccgtctg 4860
   gcctgaacaa attagttaaa aggtctaaag atgactgccg acaacaatag tatgccccat 4920
   ggtgcagtat ctagttacgc caaattagtg caaaaccaaa cacctgaaga cattttggaa 4980
   gagtttcctg aaattattcc attacaacaa agacctaata cccgatctag tgagacgtca 5040
   aatgacgaaa gcggagaaac atgtttttct ggtcatgatg aggagcaaat taagttaatg 5100
   aatgaaaatt gtattgtttt ggattgggac gataatgcta ttggtgccgg taccaagaaa 5160
   gtttgtcatt taatggaaaa tattgaaaag ggtttactac atcgtgcatt ctccgtcttt 5220
   attttcaatg aacaaggtga attactttta caacaaagag ccactgaaaa aataactttc 5280
   cctgatcttt ggactaacac atgctgctct catccactat gtattgatga cgaattaggt 5340
   ttgaagggta agctagacga taagattaag ggcgctatta ctgcggcggt gagaaaacta 5400
   gatcatgaat taggtattcc agaagatgaa actaagacaa ggggtaagtt tcacttttta 5460
   aacagaatcc attacatggc accaagcaat gaaccatggg gtgaacatga aattgattac 5520
   atcctatttt ataagatcaa cgctaaagaa aacttgactg tcaacccaaa cgtcaatgaa 5580
   gttagagact tcaaatgggt ttcaccaaat gatttgaaaa ctatgtttgc tgacccaagt 5640
   tacaagttta cgccttggtt taagattatt tgcgagaatt acttattcaa ctggtgggag 5700
   caattagatg acctttctga agtggaaaat gacaggcaaa ttcatagaat gctataaaca 5760
   aattagttaa aaggtctaaa gatggaagcc agacggtctg ccaattatga accaaatagc 5820
   tgggattatg attttttgct gtcttcagac actgacgaat cgattgaagt atacaaagac 5880
   aaggccaaaa agctggaggc tgaggtgaga agagagatta acaatgaaaa ggcagagttt 5940
   ttgactctgc ttgaactgat agataatgtc caaaggttag gattgggtta ccggttcgag 6000
   agtgacataa ggagagccct cgacagattt gtttcttcag gaggatttga tggtgttaca 6060
   aaaactagcc ttcatgctac tgctcttagc ttcaggcttc tcagacagca tggctttgag 6120
   gtctctcaag aagcgttcag tggattcaag gatcaaaatg gcaatttctt ggaaaacctt 6180
   aaggaggaca ccaaggcaat actaagccta tatgaagctt catttcttgc attagaagga 6240
   gaaaatatct tggatgaggc cagggtgttt gcaatatcac atctaaaaga gctcagcgaa 6300
   gaaaagattg gaaaagagct ggccgaacag gtgaatcatg cattggagct tccattgcat 6360
   cgcaggacgc aaagactaga agctgtttgg agtattgaag cataccgtaa aaaggaagat 6420
   gcaaatcaag tactgctaga acttgctata ttggactaca acatgattca atcagtatac 6480
   caaagagatc ttcgcgagac atcaaggtgg tggaggcgag tgggtcttgc aacaaagttg 6540
   cattttgcta aagacaggtt aattgaaagc ttttactggg cagttggagt tgcgttcgaa 6600
   cctcaataca gtgattgccg taattcagta gcaaaaatgt tttcatttgt aacaatcatt 6660
   gatgatatct atgatgttta tggtactctg gatgagctgg agctatttac agatgctgtt 6720
   gagagatggg atgttaacgc catcaatgat cttccggatt atatgaagct ctgcttccta 6780
   gctctctaca acactatcaa tgagatagct tatgacaatc tgaaggacaa gggggaaaac 6840
   attcttccat acctaacaaa agcgtgggca gatttatgca atgcattcct acaagaagca 6900
   aaatggctgt acaataagtc cacaccaaca tttgatgact atttcggaaa tgcatggaaa 6960
   tcatcctcag ggcctcttca actaattttt gcctactttg ccgtggttca aaacatcaag 7020
   aaagaggaaa ttgaaaactt acaaaagtat catgatatca tcagtaggcc ttcccacatc 7080
   tttcgtcttt gcaacgacct ggcttcagca tcggctgaga tagcgagagg tgaaactgcg 7140
   aattccgtat cctgctacat gcgtacaaaa ggcatttctg aggaacttgc tactgaatcc 7200
   gtaatgaatt tgatcgacga aacctgtaaa aagatgaaca aagaaaagct tggtggctct 7260
   ttgtttgcaa aaccttttgt cgaaacagct attaaccttg cacggcaatc ccattgcact 7320
   tatcataacg gagatgcgca tacttcacca gacgagctaa ctaggaaacg tgtcctgtca 7380
   gtaatcacag agcctattct accctttgag agataaactc aataaattaa acaacttaaa 7440
   acagagcagg ccttcgggcc tgctttttct ttctttcccg agtcccgcct cgggagggcg 7500
   gtgtcctttc cgccccgaat tgcgcatcgg gagcacgcgg tccttttcgc tcgctttgct 7560
   ctctcaagag gactaattta caaaattaca tattgagagc gcggccgcac tagtaagtta 7620
   tagaaatgtc atccgatacc gaaaaagaaa acggaataaa tcagtaacaa atcaaaaaaa 7680
   caaaactata acatttaatc ttcgtaaaat caatatcaat cgaccataca caaaacatgt 7740
   tcaaataaaa tacatgggtg tacatatgtc aaccgacatt gaaaaagaaa acggaataaa 7800
   tcagtaacaa atcaaaaaaa caaaaccata acatttctat aacattccat agagtaaaaa 7860
   tcaacatcga ataaccttac acaaaccata cataaaccat acataaaaat acacatatgt 7920
   caaccgttac cgtaaaaaga ttacttaaat aattttatac tcacctctta ttttttgtgt 7980
   tttttattat ccagggcaac ctttcccagg gagaaattct tccttgcagt tgcaggcaag 8040
   aaccaggact ttcttccagg gcatactttc ccagggagaa attcttcctt gcagctgcag 8100
   gcaagaacca ggactttccc ccaggaccag gacctcaagg tctgcatcca gaggaagacc 8160
   ttcccagggc gccaagatcc cgcagattat ggaacattat ttcccgggaa ataattcagg 8220
   gcttaatgcc caactgagga atacatttta aataagcttc tcttagttca tccttttcta 8280
   tgtggtcgta cacgtctatt gcttcgcctc tcgaatctcc ccttaattcc tgaatgaatg 8340
   tcctttccat acctgaacgt cgaagccatg tagtgaacca atgccggcag caatgcggag 8400
   taaaatgttc ttcaagtctc ccctcggggt tatggagtcc gatagaggaa gcggcagatg 8460
   tgacaatatc gtacactaaa tttctttgta cacggtgacc gttttgtctg cctatgaata 8520
   atgctttttc tttatcatgc ctcgtgttaa ggtacgcttc caggaaggcc ttagtctcaa 8580
   ggtcaaagaa tacgatacga tttgaccttt tagctgtctt cttgagtctc accgtacact 8640
   tttccagatc tatgtcagct cgatcaagag taattaattc atttctcctg atgccagttt 8700
   tagctaaaaa gattatcaat gcttgataca tggggtaacg tggtactttg acaagctttg 8760
   ccatgtcttc aatggtaatc aattgccttg tttccggagt atggtatttt ttgtagcttc 8820
   ttagatagcg ttttctgaac gtgggaatgt tgtttctctc gattatatgc ttgaattcaa 8880
   ggaagtcaaa aaaggtactc agtgaagcaa agtaattgtt acaggttgcg agtgtgtact 8940
   gcctttcatc tcttatgtga acaaggaacc cttcaagctc cggaaaccct gcattcactc 9000
   cacacatttg aaggaaaaac ttagcatgac ttacgtatcc ttctattgtt cttttagagt 9060
   aaccccttcc gatacaatca gaagtgaata aacttataag tctttcatta tcaagcggtt 9120
   catagatacc agttagctga atagctgcca tttcacccat aaatcaataa aagaagtatg 9180
   taaataaata ctttgtgcat agatgtcatt taaacacaaa gtataagtac atctaccttc 9240
   ttttagtata tgctggtaac agcaaaaaag cgccaaacaa cgcatcttta cccaatagaa 9300
   tgacgtcgat taaaagagta agttggtggt gagccttaaa aaaggtaaaa gagagcttac 9360
   tcttaagtta aaaacggaat tggcaaacag taaggattgt tccagcagtc cttactaaaa 9420
   acattacttt attaattcta tatagtcaca acattcacaa aaataggctt ttttctcgat 9480
   ttttcctttt gaatgtttat aaagaaaaat catatagcat ttattacact gtggacatat 9540
   tttcttttca attttgcccc ttccaatttg tggaagcctt ttttcagtaa cacgcttagg 9600
   cataatgaaa gatcggtgta ttaagtatta acattttcta acttgtgcaa taataattat 9660
   tcggggttgt cgaagaactt ccaaacagta gaccgatcca ttttccacat tattctaatg 9720
   aagccaagac tcaaggatgg ggggaaatat ccccccacac cccccaatga gactacccaa 9780
   gacagcccgt atgccgacag cgacacagtt aaatatcaat gtgtattaat acatatcaac 9840
   aagtatcaac acacacaatt aaacaataat acacatcaat ggagaacaat acacatgcca 9900
   acacaagaat acacacggac aaacattaca ttaccagaac atgttagagc aatgattaaa 9960
   gagtacaata aaagaagtac atggggagac ctcaacgtat cagcagtatg ctctaatgca 10020
   atagcagaca gaattagagc tacattccca aaattagaaa aagaactatt tccagaacaa 10080
   tttccagaac ttaacccaaa aatgacaaaa accagctcac caggacaacc agaaataaca 10140
   caaaaaggaa aatccaaaca aggagactct aaagatagaa tatgcgctta ttgtggccaa 10200
   ccttttgaac cgaaatcgca taatcaaaaa ttctgtaaag atgcatgtaa gtcagcaaat 10260
   tatagaaaaa acaaaaaaac acagagttaa acccattaac cacactcttt ttttgaattt 10320
   atctgcattt cacggtactt tttatccaag aaagcccaca aatcacgctc tttaaacggt 10380
   ttcatggaaa aattttcccg gaatgcatta attaacatct tttcaatttt agggttgaaa 10440
   taagccccat aacggtaatc tggcgaccaa tctgaagctt taacctcttc ataagtccga 10500
   tacggcacat tacccggaag aagcccagga accacaagca gataatagat atagcacctt 10560
   atacacatat gtgatttttt agtagtcttt aaccattcaa tatatgaaag atccgcacct 10620
   gttcgataat cctcaaaaag aattaagttc ctttgcctcc gagatccaca cacacagcaa 10680
   tacttcaatt ccataataca taacaccccg aaataataag acatctgaaa aaccgtatca 10740
   ctttaggctt taagcatctt atccggacct tcgagcccaa aagccttgtt gaacgtggca 10800
   tctcaatcac ccccaaacat catggagcgt gattttaagc ttgtaaatag ctcttcataa 10860
   ttaatttcgg aagccttttg caagctgtta gaaggcattt caggcgattc ttcaattata 10920
   cagccatcat gctcccattg atgagagtca accaccagga agccggcagg gggatcagga 10980
   agcccttcta agaaccatac ccgaagaaga ctcccggcag gttcaagctt caaaaactcc 11040
   ggtaattcat ccactttgaa gggagaagcc cataatttac gccatttttt aggaactttg 11100
   tttttgccat ctcgcccttt taggttatga taaaaattat ttcttgcaat taattgttgc 11160
   aagtaatcgg catattcagg tttaaccgct ttagcttcct tcaaataagc atgtagagcc 11220
   tgtcttccac ccgaaaaatg atcccgaagc tttgttaacg gtatccatcc agtatcatcg 11280
   ttttcctgtc cttcccactt caactctcca ttataaacga gttttacttc tcgaccttca 11340
   ttgattaaat taaggacttc ggttctggta tctatcaatt gctcaaatgt cagctttccg 11400
   gaggaactta caactttatc aggatgccac cggaacaaag caccaaagcg agattctata 11460
   tggatattac cgtatctgtt atccgtgagc tgcccaacgt gagaataaag ataagtcacc 11520
   tgcttgtaaa tatccataag gctccgcaat tcccattgcc ttttaccatt gactttatga 11580
   gaaagcctct gaataaatat atcatattca tcactcttgc atctcttttt actcttcgga 11640
   ttctttccag ttatacgaat gtttccaggg aaaatgaaaa aatgaaaatg aggagcaagg 11700
   ataaggcagt cataaataga gtttatttcg atatttagca agtccgcgat cttgtttaaa 11760
   ttgccttcgt tgtggtcgtc tcttatgtaa ttccaaaaag cttcggagtc gtcattacct 11820
   tttaaacctg ttaacttacg gatagcccga gttatttccg gcttaatcct gtaagggtga 11880
   aagattgtag taccagcagt tacgccccag cgttccagcc tgtcctttaa attcctccgg 11940
   aaaagtcgga ttgcttccac agatactccc cggctaaagt ccatcgaagc aaacccccat 12000
   gaaggaaact gcccagaaaa cagggcataa gtccacaaaa ttacaactaa tgcaaatcta 12060
   tgttgcaatt ccgataggat gccgcaacca ggacaagaaa gtgtattgca gtgagaatta 12120
   agatcctttc gtgcattata attttcagat acagcataag tgaaaggacg cccacagtaa 12180
   gcgggtcttt ttccctttgc tggaagaaca aagtcgccaa cttttagagg acgatactta 12240
   gagcatttag aaaaatcgaa gtcaggacaa gagtagtatt tacagataga aaccccgttc 12300
   tttttgttgc gaaccgggca caaaacacca tcggtacata tacacgtcgc aaaatctcta 12360
   cactctggag cataggaaac ggcgccaggt ctagaactaa aatcagaact cagagaacat 12420
   ttcctgtcga aaatttaaac cgtaaaccaa agataacaat tttcataata ttacagaacc 12480
   tatatttttt gtaactcgac attacaaaaa tgtaccacaa aacataaaaa cattttcata 12540
   gaagaaatat tcacaaacaa tagaaaaaaa ttataagaat aagattaacg cctaccttat 12600
   taaaagaagg tgtcgagtct tcaggttcca tcaaggcagg cgttgatatt ctatatatgt 12660
   tacacataac atatataaca tacgtacctc agaacattca gacctgaatg ctctaaaatg 12720
   aaataagctt taaactttat aaattcatct gctataaata gttttagaga tatagaaagc 12780
   ccaaggaaga aaaagaatag caaaaataaa aaagaaaaac ggcccgcgac ggcaaacccg 12840
   gagacttata atcaaagtaa gacgaaaagg aagccgacaa cgatttgata ataacaaatc 12900
   atcttttatt ttggcaatat tcatatgata tatgaagaaa tcaacatcaa gcggattagt 12960
   atttttgcta tttgtaaatc aatatcgggt gagacagtat atcaaaaaca aacttcattt 13020
   ttttcgaaca aaaaaatcac tatcaaatga cattgtagta agaaactaca tcaaaactag 13080
   ttgatcatat cgtcaattat tacctccacg gggagagcct gagcaaactg gcctcaggca 13140
   tttgagaagc acacggtcac actgcttccg gtagtcaata aaccggtaaa ccagcaatag 13200
   acataagcgg ctatttaacg accctgccct gaaccgacga ccgggtcgaa tttgctttcg 13260
   aatttctgcc attcatccgc ttattatcac ttattcaggc gtagcaccag gcgtttaagg 13320
   gcaccaataa ctgccttaaa aaaattacgc cccgccctgc cactcatcgc agtactgttg 13380
   taattcatta agcattctgc cgacatggaa gccatcacag acggcatgat gaacctgaat 13440
   cgccagcggc atcagcacct tgtcgccttg cgtataatat ttgcccatgg tgaaaacggg 13500
   ggcgaagaag ttgtccatat tggccacgtt taaatcaaaa ctggtgaaac tcacccaggg 13560
   attggctgag acgaaaaaca tattctcaat aaacccttta gggaaatagg ccaggttttc 13620
   accgtaacac gccacatctt gcgaatatat gtgtagaaac tgccggaaat cgtcgtggta 13680
   ttcactccag agcgatgaaa acgtttcagt ttgctcatgg aaaacggtgt aacaagggtg 13740
   aacactatcc catatcacca gctcaccgtc tttcattgcc atacggaatt ccggatgagc 13800
   attcatcagg cgggcaagaa tgtgaataaa ggccggataa aacttgtgct tatttttctt 13860
   tacggtcttt aaaaaggccg taatatccag ctgaacggtc tggttatagg tacattgagc 13920
   aactgactga aatgcctcaa aatgttcttt acgatgccat tgggatatat caacggtggt 13980
   atatccagtg atttttttct ccat 14004

## Description

### TECHNICAL FIELD

The present invention relates to a recombinant cell, and a method for producing isoprene or terpene. In particular, the present invention relates to an archael recombinant cell that produces isoprene or terpene, and a method for producing isoprene or terpene using the recombinant cell.

### BACKGROUND ART

In recent years, development of technology for manufacturing petrification alternative chemicals from syngas (mixed gas of CO, CO₂, and H₂), which is combustion gas of waste and the like using a microorganism, has been advanced. Among them, it is important to construct a petrification alternative process of isoprene as raw material of tire, and therefore, techniques for producing isoprene and terpene using a recombinant syngas assimilating bacterium belonging to the genus *Clostridium* have been considered (see, for example, Patent documents 1 to 3). In the conventional techniques, however, although a foreign gene for synthesizing an objective compound is expressed under a strong constitutive expression promoter, it is negatively controlled by a host cell, and therefore, only low productivity can be achieved.

Meanwhile, production of isoprene or terpene by, for example, wild-type *Bacillus subtilis*, recombinant *E. coli*, recombinant yeast, has been conventionally attempted. However, at present, the cost is still high, and practical application is difficult. Furthermore, since synthesis and metabolism of isoprene and terpene are strictly controlled in almost all organisms, in the production of isoprene and terpene using recombinant microorganisms, no examples have demonstrated production that satisfies a commercialization level. In Patent Documents 1 and 3, production of isoprene using a constitutive expression system is verified, but its productivity is very low such as about several times as that of the wild-type.

Mass-production (yield: 60 g/L) of isoprene from glucose by recombinant *E. coli,* into which a heterologous mevalonate pathway gene and an isoprene synthase gene have been introduced, has been demonstrated (Non-Patent Document 1). In this technique, production of isoprene from glucose is promoted by induction of IPTG. However, the process using glucose as a raw material has a problem that raw material supply is restrictive. Furthermore, since production of a substance using an inducible expression system has a low carbon converting rate (15% or less) and is carried out by not continuous expression process but batch expression process, it is not suitable for producing isoprene for commercialization.

Furthermore, it is reported that when an operon gene including a heterologous mevalonate pathway gene and an α-humulene (C15 cyclic terpene) synthase gene is constitutively expressed in *Methanobacterium extroquence* that is a methylotroph, a transformant is not produced due to toxicity caused by the pathway gene expression, and α-humulene is efficiently produced only by an induction expression method having strictly controlled activity (Non-Patent Document 2).

As mentioned above, production of isoprene and terpene by microorganisms has been much considered. However, there are still very few examples of productivity at practical application level. Establishment of a more efficient microorganism process is desired.

### PRIOR ART DOCUMENTS

### Patent Documents

Patent Document 1: WO2013/180584
Patent Document 2: WO2013/181647
Patent Document 3: WO2014/065271

### Non-Patent Documents

Non-Patent Document 1: Whited GM et al., Industrial biotechnology 2010, 6 (3), 152-163
Non-Patent Document 2: Sonntag F. et al., Metabolic Engineering 2015, 32, 82-94

### DISCLOSURE OF INVENTION

### TECHNICAL PROBLEM

On the other hand, there is no specific report about techniques for producing isoprene or terpene using archaea such as natural archaea, mutant archaea, and recombinant archaea. An object of the present invention is to provide an archael recombinant cell that can efficiently produce isoprene or terpene from abundant and low-cost raw materials such as syngas, methanol, and methane, and a method for producing isoprene or terpene using the recombinant cell.

### SOLUTION TO PROBLEM

One aspect of the present invention is a recombinant cell being an anaerobic archaeon, including a gene encoding isoprene synthase, a gene encoding monoterpene synthase, a gene encoding sesquiterpene synthase, a gene encoding diterpene synthase, a gene encoding squalene synthase, or a gene encoding phytoene synthase as a first foreign gene, wherein the first foreign gene is expressed, and the recombinant cell produces isoprene or terpene having 10, 15, 20, 30, or 40 carbon atoms.

Preferably, the recombinant cell grows using at least one selected from the group consisting of carbon monoxide, carbon dioxide, methane, methanol, and acetic acid as a sole carbon source.

Preferably, the recombinant cell includes carbon monoxide dehydrogenase.

Preferably, the recombinant cell has a function of synthesizing acetyl-CoA from methyltetrahydropterin, carbon monoxide, and CoA.

Preferably, the recombinant cell has a methane formation potential.

Preferably, the recombinant cell grows using carbon dioxide as a sole carbon source and hydrogen as an energy source.

Preferably, the recombinant cell grows using methanol as a sole carbon source.

Preferably, the recombinant cell grows using methane as a sole carbon source.

Preferably, the recombinant cell belongs to the genus *Methanosarcina,* the genus *Methanococcus,* the genus *Methanothermococcus,* the genus *Methanothermobacter,* the genus *Methanothrix,* the genus *Thermococcus,* the genus *Thermofilum,* or the genus *Archaeoglobus.*

Preferably, the recombinant cell belongs to the genus *Methanosarcina,* the genus *Methanococcus,* or the genus *Methanothermococcus.*

Preferably, the recombinant cell is *Methanosarcina barkeri, Methanosarcina mazei, Methanosarcina acetivorans, Methanococcus voltae, Methanococcus vannielii, Methanococcus maripaludis,* or *Methanothermococcus (Methanococcus) thermolithotrophicus.*

Preferably, the recombinant cell further includes a gene encoding carbon monoxide dehydrogenase as a second foreign gene.

Preferably, in the recombinant cell, the first foreign gene is a gene encoding isoprene synthase, the recombinant cell produces isoprene, and the isoprene synthase is derived from a plant.

Preferably, in the recombinant cell, the isoprene synthase is a protein of the following (a-1), (a-2), or (a-3):
(a-1) a protein consisting of an amino acid sequence of SEQ ID NO: 1,
(a-2) a protein consisting of an amino acid sequence in which 1 to 20 amino acids are deleted, substituted, or added in the amino acid sequence of SEQ ID NO: 1, and having isoprene synthase activity, and
(a-3) a protein consisting of an amino acid sequence having identity of 90% or more with the amino acid sequence of SEQ ID NO: 1, and having isoprene synthase activity.

Preferably, the recombinant cell further includes a gene encoding isopentenyl diphosphate isomerase as a third foreign gene.

Preferably, in the recombinant cell, the first foreign gene is a gene encoding sesquiterpene synthase, the recombinant cell produces terpene having 15 carbon atoms, and the sesquiterpene synthase is farnesene synthase.

Preferably, in the recombinant cell, the first foreign gene is a gene encoding sesquiterpene synthase, the recombinant cell produces terpene having 15 carbon atoms, and the sesquiterpene synthase is cyclic sesquiterpene synthase.

Preferably, in the recombinant cell, the farnesene synthase is a protein of the following (d-1), (d-2), or (d-3):
(d-1) a protein consisting of an amino acid sequence of SEQ ID NO: 4,
(d-2) a protein consisting of an amino acid sequence in which 1 to 20 amino acids are deleted, substituted, or added in the amino acid sequence of SEQ ID NO: 4, and having farnesene synthase activity, and
(d-3) a protein consisting of an amino acid sequence having identity of 90% or more with the amino acid sequence of SEQ ID NO: 4, and having farnesene synthase activity.

Preferably, the recombinant cell further includes a gene encoding isopentenyl diphosphate isomerase as a fourth foreign gene.

Preferably, the recombinant cell further includes at least one gene selected from the group consisting of a gene encoding geranyl diphosphate synthase and a gene encoding farnesyl diphosphate synthase, as a fifth foreign gene.

Preferably, in the recombinant cell, the first foreign gene is a gene encoding monoterpene synthase, the recombinant cell produces terpene having 10 carbon atoms, and the monoterpene synthase is cyclic monoterpene synthase.

Preferably, the recombinant cell further includes a gene encoding isopentenyl diphosphate isomerase as a sixth foreign gene.

Preferably, the recombinant cell further includes a gene encoding geranyl diphosphate synthase as a seventh foreign gene.

Preferably, the recombinant cell further includes a gene encoding neryl diphosphate synthase as an eighth foreign gene.

Preferably, in the recombinant cell, the cyclic monoterpene synthase is phellandrene synthase.

Preferably, in the recombinant cell, the phellandrene synthase is a protein of the following (b-1), (b-2), or (b-3):
(b-1) a protein consisting of an amino acid sequence of SEQ ID NO: 2;
(b-2) a protein consisting of an amino acid sequence in which 1 to 20 amino acids are deleted, substituted, or added in the amino acid sequence of SEQ ID NO: 2, and having α-phellandrene synthase; and
(b-3) a protein consisting of an amino acid sequence having identity of 90% or more with the amino acid sequence of SEQ ID NO: 2, and having α-phellandrene synthase activity.

Preferably, in the recombinant cell, the phellandrene synthase is a protein of the following (c-1), (c-2), or (c-3):
(c-1) a protein consisting of an amino acid sequence of SEQ ID NO: 3;
(c-2) a protein consisting of an amino acid sequence in which 1 to 20 amino acids are deleted, substituted, or added in the amino acid sequence of SEQ ID NO: 3, and having β-phellandrene synthase; and
(c-3) a protein consisting of an amino acid sequence having identity of 90% or more with the amino acid sequence of SEQ ID NO: 3, and having β-phellandrene synthase activity.

Preferably, in the recombinant cell, the first foreign gene is a gene encoding diterpene synthase, the recombinant cell produces terpene having 20 carbon atoms, and the diterpene synthase is cyclic diterpene synthase.

Preferably, the recombinant cell further includes a gene encoding copalyl diphosphate synthase as a ninth foreign gene.

Preferably, the recombinant cell further includes a gene encoding isopentenyl diphosphate isomerase as a tenth foreign gene.

Preferably, the recombinant cell further includes at least one gene selected from the group consisting of a gene encoding geranyl diphosphate synthase, a gene encoding farnesyl diphosphate synthase, and a gene encoding geranylgeranyl diphosphate synthase as an eleventh foreign gene.

Preferably, in the recombinant cell, the first foreign gene is a gene encoding squalene synthase, and the recombinant cell produces terpene having 30 carbon atoms. The recombinant cell further includes a gene encoding isopentenyl diphosphate isomerase as a twelfth foreign gene.

Preferably, the recombinant cell further includes at least one gene selected from the group consisting of a gene encoding geranyl diphosphate synthase and a gene encoding farnesyl diphosphate synthase as a thirteenth foreign gene.

Preferably, in the recombinant cell, the first foreign gene is a gene encoding phytoene synthase, and the recombinant cell produces terpene having 40 carbon atoms, and the recombinant cell further includes a gene encoding isopentenyl diphosphate isomerase as a fourteenth foreign gene.

Preferably, the recombinant cell further includes at least one gene selected from the group consisting of a gene encoding geranyl diphosphate synthase, a gene encoding farnesyl diphosphate synthase, and geranylgeranyl diphosphate synthase as a fifteenth foreign gene.

Preferably, the recombinant cell further includes at least one gene selected from the group consisting of acetoacetyl-CoA thiolase, HMG-CoA synthase, HMG-CoA reductase, mevalonate-5-kinase, mevalonate-5-phosphate decarboxylase, isopentenyl phosphate kinase, and isopentenyl diphosphate isomerase as a sixteenth foreign gene.

Preferably, in the recombinant cell, the first foreign gene is expressed under a constitutive expression promoter.

Another aspect of the present invention is a method for producing isoprene or terpene, the method including bringing at least one carbon source selected from the group consisting of carbon monoxide, carbon dioxide, methane, methanol, and acetic acid into contact with the above-described recombinant cell, thereby allowing the recombinant cell to produce isoprene or terpene having 10, 15, 20, 30, or 40 carbon atoms from the carbon source.

Preferably, the method includes culturing the recombinant cell using at least one carbon source selected from the group consisting of carbon monoxide, carbon dioxide, methane, methanol, and acetic acid, and obtaining isoprene or terpene having 10, 15, 20, 30, or 40 carbon atoms from the cultured product.

Preferably, in the method, a cell concentration of a culture solution of the recombinant cell in a proliferation stationary phase is 1 g or more of dry cells per liter, and the method includes continuously supplying a culture medium and discharging a culture solution.

Preferably, the recombinant cell is cultured in a culture tank, and a mixed gas consisting of carbon monoxide, carbon dioxide, and hydrogen is supplied to the culture tank.

Preferably, in the method, a carbon monoxide concentration in the mixed gas is 20% or more.

Preferably, in the method, a carbon dioxide concentration in the mixed gas is 5% or more.

Preferably, in the method, a hydrogen sulfide concentration in the mixed gas is 10 ppm or less.

Preferably, the method further include adding methanol and/or acetic acid in a culture solution.

### EFFECT OF INVENTION

The present invention permits production of isoprene or terpene with high efficiency using a recombinant cell.

### BRIEF DESCRIPTION OF DRAWINGS

Fig. 1 is an explanatory diagram showing a mevalonate pathway and a membrane lipid synthesis pathway in archaea.
Fig. 2 is an explanatory diagram showing diversity of pathways in which isopentenyl diphosphate is synthesized from mevalonate.
Fig. 3 is an explanatory diagram showing a pathway of assimilation and metabolism of carbon monoxide and acetic acid by methane-generating archaea *Methanosarcina acetivorans.*
Fig. 4 is an explanatory diagram showing a pathway of assimilation of methanol and methane in archaea.
Fig. 5 is an explanatory diagram showing a configuration of a shuttle vector pAC:Pmcr-IDI-IspS for production of isoprene.
Fig. 6 is an explanatory diagram showing a configuration of a shuttle vector pAC:Pmcr-IDI-FnS for production of farnesene.
Fig. 7 is an explanatory diagram showing a configuration of a shuttle vector pAC:Pmcr-IDI-FnS-FPS for production of farnesene.
Fig. 8 is an explanatory diagram showing a configuration of a shuttle vector pAC:Pmcr-MVA1-IDI-IspS for production of isoprene.

### BEST MODE FOR CARRYING OUT THE INVENTION

Hereinafter, the exemplary embodiment of the present invention will be described. Note here that in the present invention, all the terms "gene" can be replaced with terms "nucleic acid" or "DNA".

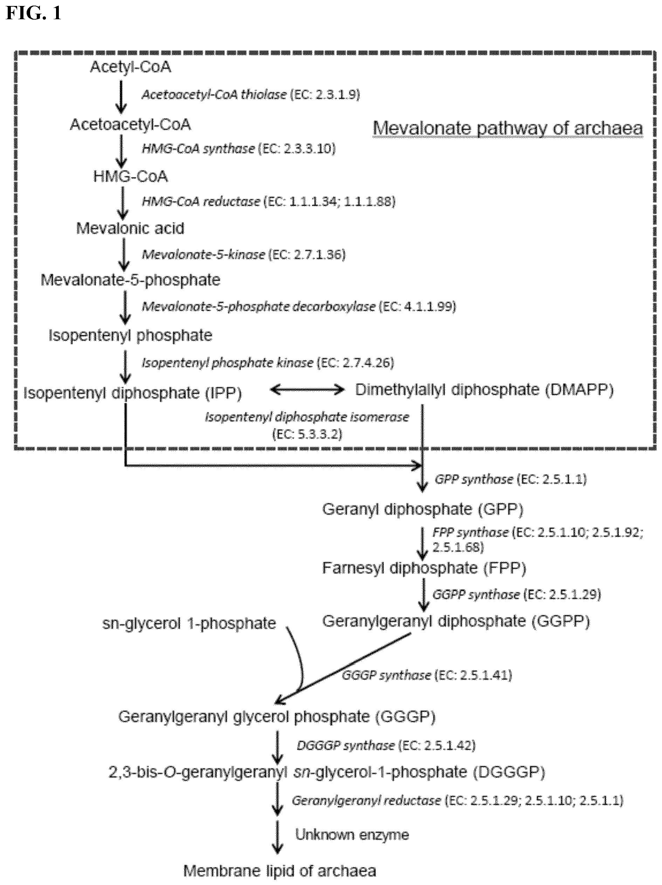

A recombinant cell of the present invention belongs to anaerobic archaea. In general, membrane lipid of archaea is ether polar lipid in which hydrocarbon (the number of carbon atoms: 20 to 40) including an isoprene skeleton is ether-linked to a glycerol skeleton. The membrane lipid peculiar to archaea is synthesized as shown in Fig. 1, in which geranylgeranyl glycerol phosphate (3-O-geranylgeranyl sn-glycerol-1-phosphate (GGGP)) is generated from geranylgeranyl diphosphate (GGPP) as a C20 compound and glycerol-1-phosphate (sn-glycerol 1-phosphate), and then two stages of reactions are carried out. The geranylgeranyl diphosphate (the number of carbon atoms: 20) is synthesized, as shown in Fig. 1, isopentenyl diphosphate (IPP) having 5 carbon atoms, geranyl diphosphate (GPP) having 10 carbon atoms, and farnesyl diphosphate (FPP) having 15 carbon atoms, which are supplied by endogenous mevalonate pathway of archaea. In this way, in archaea, unlike the other organisms, a large amount of geranylgeranyl diphosphate is always supplied through the mevalonate pathway for synthesis of membrane lipid accompanying the proliferation. The present invention uses main metabolism peculiar to archaea as described above to efficiently produce isoprene or terpene having 10 to 40 carbon atoms by a recombinant cell.

The anaerobic archaeon is referred to as an archaeon that can proliferate under the condition in which molecular oxygen is hardly present (100 ppm or less). The anaerobic archaeon in the present invention includes both strictly anaerobic one and facultative anaerobic one.

In the present invention, the recombinant cell having an isoprene synthase gene as the first foreign gene can produce isoprene. Furthermore, the recombinant cell having a monoterpene synthase gene as the first foreign gene can produce monoterpene (terpene having 10 carbon atoms). Furthermore, the recombinant cell having a sesquiterpene synthase gene as the first foreign gene can produce sesquiterpene (terpene having 15 carbon atoms). Furthermore, the recombinant cell having diterpene synthase gene as the first foreign gene can produce diterpene (terpene having 20 carbon atoms). Furthermore, the recombinant cell having a squalene synthase gene as the first foreign gene can produce triterpene (terpene having 30 carbon atoms). Furthermore, the recombinant cell having a phytoene synthase gene as the first foreign gene can produce tetraterpene (terpene having 40 carbon atoms). Hereinafter, each enzyme and each gene are described sequentially.

### <Isoprene synthase>

Isoprene synthase (IspS) has action of converting dimethylallyl diphosphate (DMAPP) as an isomer of isopentenyl diphosphate (IPP) into isoprene. Note here that the structural conversion between the isopentenyl diphosphate and dimethylallyl diphosphate is catalyzed by isopentenyl diphosphate isomerase (IDI). The isopentenyl diphosphate isomerase is present in all organisms.

The isoprene synthase (IspS) used in the present invention is not particularly limited. For example, isoprene synthase derived from eukaryote such as plant can be used. General examples of the isoprene synthase derived from plants include, but not particularly limited to, isoprene synthase derived from *Populus nigra, Stizolobium deeringianum*, and *Pueraria lobata Ohwi.* Specific examples of the isoprene synthase include Q50L36, Q6EJ97, Q9AR86, Q7XAS7, A0PFK2, A0A0M4UQH9, A0A0M5MSL0 (all of the above is UniProtKB entry).

SEQ ID NO: 1 shows a nucleotide sequence of a nucleic acid (DNA) encoding the isoprene synthase derived from *Populus nigra* (GenBank Accession No.: AM410988.1) and a corresponding amino acid sequence. SEQ ID NO: 2 shows only the amino acid sequence.

The isoprene synthase used in the present invention may be not only a naturally occurring and isolated isoprene synthase but also a modified product thereof. For example, it may be proteins that are partial fragments of the existing isoprene synthase or may be amino acid substitution variants and have activity as isoprene synthase.

For example, the isoprene synthase used in the present invention includes at least the following protein (a-1) to (a-3):
(a-1) a protein consisting of an amino acid sequence of SEQ ID NO: 1,
(a-2) a protein consisting of an amino acid sequence in which 1 to 20 amino acids are deleted, substituted, or added in the amino acid sequence of SEQ ID NO: 1, and having isoprene synthase activity, and
(a-3) a protein consisting of an amino acid sequence having identity of 90% or more with the amino acid sequence of SEQ ID NO: 1, and having isoprene synthase activity.

Note here that the identity of an amino acid sequence in (a-3) is more preferably 92% or more, further more preferably 95% or more, and particularly preferably 98% or more.

The preferable embodiment further includes a gene encoding isopentenyl diphosphate isomerase (IDI), in addition to the isoprene synthase gene, as a foreign gene. Introduction of the IDI gene enhances the conversion from IPP to DMAPP, and can enhance the isoprene synthesis ability. The IDI used in the present invention is not particularly limited, and examples thereof include P61615, Q13907, Q46822, P50740, Q8TT35, P15496, Q10132, and Q9KWG2 (UniProtKB entry), and the like. Furthermore, in the present invention, IDI derived from archaea may be used, and, for example, it is preferable to use Q8TT35, Q8PW37, Q46CL4, A0A0E3LM81, and A0A0E3QQI3 (UniProtKB entry) derived from the genus *Methanosarcina.*

### <Monoterpene synthase>

Monoterpene is terpene having 10 carbon atoms, consisting of two isoprene units. The monoterpene include acyclic monoterpene and cyclic monoterpene. Examples of the acyclic monoterpene include geraniol, myrcene, citral, linalool, and nerol. Examples of the cyclic monoterpene include limonene, α-phellandrene, β-phellandrene, menthol, thymol, α-pinene, β-pinene, carene, carvone, cineol, and camphor.

The monoterpene synthase is a general name of enzymes that convert geranyl diphosphate (GPP) or neryl diphosphate (NPP) into monoterpene. In a synthesis pathway of monoterpene, GPP or NPP is synthesized from isopentenyl diphosphate (IPP) by the action of the GPP synthase (GPPS) or NPP synthase (NPPS). Subsequently, monoterpene is synthesized from GPP or NPP by the action of the monoterpene synthase.

In the preferable embodiment, the monoterpene synthase is cyclic monoterpene synthase. Further preferably, the cyclic monoterpene synthase is phellandrene synthase, and, specifically, it is α-phellandrene synthase or β-phellandrene synthase.

As the α-phellandrene synthase, any enzymes can be used as long as they have activity to generate α-phellandrene from GPP or NPP as a substrate. Examples of the α-phellandrene synthase include G5CV35 and E5GAG2 (UniProtKB entry), and GN65-37361 (SolCyc GeneID), but not particularly limited thereto.

As the β-phellandrene synthase, any enzymes can be used as long they have activity to generate β-phellandrene from GPP or NPP as a substrate. Examples of the β-phellandrene synthase include Q9M7D1, C1K5M3, Q1XBU4, R9QMW3, R9QMR4, R9QMW7, E9N3U9, C0PTH8, F2XFA5, F2XFA1, F2XFA4, and A0A0B0P314 (UniProtKB entry), but not particularly limited thereto.

The monoterpene synthase used in the present invention may be not only a naturally occurring and isolated monoterpene synthase but also a modified product thereof. For example, the monoterpene synthase may be proteins that are partial fragments or amino acid substitution variants of the existing monoterpene synthase and that have monoterpene synthase activity.

For example, the phellandrene synthase (one example of the monoterpene synthase) used in the present invention includes at least the following protein (b-1) to (b-3):
(b-1) a protein consisting of an amino acid sequence of SEQ ID NO: 2,
(b-2) a protein consisting of an amino acid sequence in which 1 to 20 amino acids are deleted, substituted, or added in the amino acid sequence of SEQ ID NO: 2, and having α-phellandrene synthase activity, and
(b-3) a protein consisting of an amino acid sequence having identity of 90% or more with the amino acid sequence of SEQ ID NO: 2, and having α-phellandrene synthase activity.

Note here that the identity of the amino acid sequence in (b-3) is more preferably 92% or more, further more preferably 95% or more, and particularly preferably 98% or more.

Besides, the phellandrene synthase (one example of the monoterpene synthase) used in the present invention includes at least the following protein (c-1) to (c-3):
(c-1) a protein consisting of an amino acid sequence of SEQ ID NO: 3,
(c-2) a protein consisting of an amino acid sequence in which 1 to 20 amino acids are deleted, substituted, or added in the amino acid sequence of SEQ ID NO: 3, and having β-phellandrene synthase activity, and
(c-3) a protein consisting of an amino acid sequence having identity of 90% or more with the amino acid sequence of SEQ ID NO: 3, and having β-phellandrene synthase activity.

Note here that the identity of the amino acid sequence in (c-3) is more preferably 92% or more, further more preferably 95% or more, and particularly preferably 98% or more.

The preferable embodiment further includes a gene encoding isopentenyl diphosphate isomerase (IDI), in addition to the monoterpene synthase gene, as a foreign gene. Introduction of the IDI gene enhances the conversion from IPP to DMAPP, and can enhance the GPP synthesis ability or NPP synthesis ability. As a result, it is possible to enhance the monoterpene synthesis ability.

The preferable embodiment further includes a gene encoding GPP synthase (GPPS) or a gene encoding NPP synthase (NPPS), in addition to the monoterpene synthase gene, as a foreign gene. Introduction of these genes can enhance the synthesis ability of monoterpene from GPP or NPP. Examples of the GPPS include S4S927, S4S8D9, D8LHY4, H6VLF6, H6VLF3, D8RV97, Q6V4K1, Q8LKJ3, Q8LKJ2, Q8LKJ1, Q9FSW8, H6VLF7, V5REB1, and Q58GE8 (UniProtKB entry). Examples of the NPPS include NDPS1 derived from *Solanum lycopersicum* (Schilmiller AL et al., PNAS 2009, 106 (26), 10865-10870). Note here that in the case of GPPS, GPPS derived from archaea may be used, and, it is particularly preferable to use, for example, MSBRM_0487, MA_0606, MM_1767, MSBRW_3310, and Mbar_A1417 (KEGG entry) derived from the genus *Methanosarcina.*

### <Sesquiterpene synthase>

Sesquiterpene is terpene having 15 carbon atoms, consisting of three isoprene units. The sesquiterpene includes acyclic sesquiterpene, monocyclic sesquiterpene, bicyclic sesquiterpene, and tricyclic sesquiterpene. Examples of the acyclic sesquiterpene include farnesene and farnesol. Examples of the monocyclic sesquiterpene include zingiberene, Humulene, and abscisic acid. Examples of the bicyclic sesquiterpene include Caryophyllene, Eudesman, Eremophilan, Valeran, Cadinan, Cadinene, Guajan, Driman, Cedrol, and Nootkatone. Examples of the tricyclic sesquiterpene include Illudan, Prezizaan, Marasman, Cedran, Thujopsan, and Hirsutan.

The sesquiterpene synthase is a general name of enzymes that convert farnesyl diphosphate (FPP) into sesquiterpene. In a synthesis pathway of sesquiterpene, GPP is synthesized from IPP by the action of GPP synthase. Subsequently, FPP is synthesized from GPP by the action of FPP synthase. Subsequently, sesquiterpene is synthesized from FPP by the action of the sesquiterpene synthase.

In the preferable embodiment, the sesquiterpene synthase is cyclic sesquiterpene synthase. In another preferable embodiment, the sesquiterpene synthase is farnesene synthase.

As the farnesene synthase, any enzymes can be used as long as they have activity to generate farnesene from farnesyl diphosphate (FPP) as a substrate. Examples of the farnesene synthase include Q84LB2, B9RXW0, B2KSJ6, and Q84KL5 (UniProtKB entry) for synthesizing an α-form of farnesene ((3E, 6E)-alpha-farnesene), and Q9FXY7, O48935, Q2NM15, C7E5V9, C7E5V7, Q94JS8, C7E5W0, and C7E5V8 (UniProtKB entry), for synthesizing a β-form of the farnesene ((E)-beta-famesene), but the examples are not particularly limited thereto.

The sesquiterpene synthase used in the present invention may be not only a naturally occurring and isolated sesquiterpene synthase but also a modified product thereof. For example, the sesquiterpene synthase may be proteins that are partial fragments or amino acid substitution variants of the existing sesquiterpene synthase and that have sesquiterpene synthase activity.

For example, the farnesene synthase (one example of the sesquiterpene synthase) used in the present invention includes at least the following protein (d-1) to (d-3):
(d-1) a protein consisting of an amino acid sequence of SEQ ID NO: 4,
(d-2) a protein consisting of an amino acid sequence in which 1 to 20 amino acids are deleted, substituted, or added in the amino acid sequence of SEQ ID NO: 4, and having farnesene synthase activity, and
(d-3) a protein consisting of an amino acid sequence having identity of 90% or more with the amino acid sequence of SEQ ID NO: 4, and having farnesene synthase activity.

Note here that the identity of the amino acid sequence in (d-3) is more preferably 92% or more, further more preferably 95% or more, and particularly preferably 98% or more.

The preferable embodiment further includes a gene encoding IDI as a foreign gene, in addition to the sesquiterpene synthase gene. Introduction of the IDI gene can enhance the GPP synthesis ability. As a result, it is possible to enhance the FPP synthesis ability, and enhance the sesquiterpene synthesis ability.

The preferable embodiment further includes a gene encoding GPP synthase (GPPS) and/or a gene encoding FPP synthase (FPPS), in addition to the sesquiterpene synthase gene and the like, as a foreign gene. Introduction of these genes enhances synthesis ability of GPP and/or FPP, and, as a result, can enhance the synthesis ability of sesquiterpene. Examples of the GPPS include those listed above. Examples of the FPPS include P08524, P09152, P49349, P14324, P05369, and 0014230 (UniProtKB entry). Note here that FPPS derived from archaea may be used. It is particularly preferable to use, for example, MSBRM_0487, MA_0606, MM_1767, MSBRW_3310, and Mbar_A1417 (KEGG entry), derived from the genus *Methanosarcina.* Any one of GPPS gene and FPPS gene may be introduced, or both of them may be introduced.

### <Diterpene synthase>

Diterpene is terpene having 20 carbon atoms, consisting of four isoprene units. The diterpene include acyclic diterpene, monocyclic diterpene, bicyclic diterpene, and tricyclic diterpene. Examples of the acyclic diterpene include α-tocopherol, retinol, and phytol. Examples of the cyclic diterpene include Abietane, Abietic acid, Neoabietic acid, Levomaric acid, Sapietic acid, Atisane, Beyerane, Gibbane, Gibberellic acid, Kaurane, Steviol, Labdane, Picrasane, Pimarane, Podocarpane, Rosane, Taxane, retinal, retinoic acid, and retinol.

The diterpene synthase is a general name of enzymes that convert geranylgeranyl diphosphate (GGPP) into diterpene. In a synthesis pathway of diterpene, GPP is synthesized from IPP by the action of the GPP synthase. Subsequently, FPP is synthesized from GPP by the action of the FPP synthase. Subsequently, GGPP is synthesized from FPP by the action of the GGPP synthase (GGPPS). Subsequently, diterpene is synthesized from GGPP by the action of the diterpene synthase.

As the diterpene synthase, any enzymes can be used as long as they have activity to generate diterpene from GGPP. Examples the diterpene synthase include Q38710, P9WJ61, G9MAN7, M4HY05, H8ZM70, M1VDX3, A2PZA5, Q675L5, Q0E088, P9WJ60, Q6Z5J6, and M4HYP3 (UniProtKB entry), but not particularly limited thereto.

The diterpene synthase used in the present invention may be not only a naturally occurring and isolated diterpene synthase but also a modified product thereof. For example, the diterpene synthase may be proteins that are partial fragments or amino acid substitution variants of the existing diterpene synthase and that have diterpene synthase activity.

The preferable embodiment further includes a gene encoding IDI, in addition to the diterpene synthase gene, as a foreign gene. Introduction of the IDI gene can enhance GPP synthesis ability. As a result, the FPP synthesis ability and GPP synthesis ability are strengthened, and the diterpene synthesis ability can be enhanced.

The preferable embodiment further includes at least one gene selected from the group consisting of a gene encoding GPP synthase (GPPS), a gene encoding FPP synthase (FPPS), and a gene encoding GGPP synthase (GGPPS), in addition to the diterpene synthase gene, as a foreign gene. Introduction of these genes enhances the synthesis ability of GPP, FPP, or GGPP. As a result, the diterpene synthesis ability can be enhanced. Examples of the GPPS or the FPPS include those listed above. Examples of the GGPPS include Q12051, Q84J75, P34802, P80042, Q94ID7, Q9SLG2, Q9C446, Q54BK1, Q9LUE1, Q92236, Q39108, 095749, Q12051, Q9P885, and P24322 (UniProtKB entry). Note here that GGPPS derived from archaea may be used, and, for example, it is particularly preferable to use MSBRM_0487, MA_0606, MM_1767, MSBRW_3310, and Mbar_A1417, derived from the genus *Methanosarcina* (KEGG entry).

Any one of the GPPS gene, the FPPS gene, and the GGPPS gene may be introduced, or two or more thereof may be introduced.

The preferable embodiment further includes a gene encoding copalyl diphosphate synthase (CPPS), in addition to the diterpene synthase gene, as a foreign gene. Copalyl diphosphate (CPP) is a GGPP derivative having 20 carbon atoms. When the CPP synthase gene is introduced, a substrate of the diterpene synthase may be CPP. Examples of the CPPS include G8HZG6, O22667, A0A0N71618, and Q0Q2G7 (UniProtKB entry).

### <Squalene synthase>

Triterpene is terpene having 30 carbon atoms, consisting of six isoprene units. In general, squalene (C30) as acyclic triterpene is generated by dimerization of FPP (C15) (through catalysis of squalene synthase), 2,3-Oxidosqualene (2,3-epoxy-2,3-dihydroaqualene) is generated from squalene, and 200 types or more of triterpene skeletons can be biosynthesized through cyclization of 2,3-Oxidosqualene. However, since the generation of 2,3-Oxidosqualene from squalene has an oxygen requirement property, triterpene that can be produced by the recombinant cell that is an anaerobic archaeon of the present invention is mainly Hopene, Hopanol, and Hopanoid compounds as the derivative thereof, which are generated by the cyclization of squalene.

As described above, squalene synthase (SS) (EC 2.5.1.21) has action of dimerizing FPP. When the Hopanoid compound is synthesized, at least Squalene/Hopene cyclase (EC 5.4.99.17) gene, or Squalene/Hopanol cyclase (EC 4.2.1.129) gene, in addition to squalene synthase gene, may be introduced. In general, the Squalene/Hopene cyclase also has Squalene/Hopanol cyclization enzymatic activity. Examples of the squalene synthase (SS) include P53799, P36596, P29704, P37268, P52020, Q9HGZ6, Q9Y753, Q9SDW9, and P78589 (UniProtKB entry). Examples of the Squalene/Hopene (Squalene/Hopanol) cyclase include P33247, P33990, P54924, and P55348 (UniProtKB entry).

Further introduction of the IDI gene in addition to the SS gene can enhance the squalene synthesis ability. Furthermore, in addition, introduction of the geranyl diphosphate synthase (GPPS) gene and/or the farnesyl diphosphate synthase (FPPS) gene can enhance the synthesis ability of squalene. Examples of the GPPS and FPPS are those listed above.

The squalene synthase used in the present invention may be not only a naturally occurring and isolated squalene synthase but also a modified product thereof. For example, the squalene synthase may be proteins that are partial fragments or amino acid substitution variants of the existing squalene synthase and that have squalene synthase activity.

### <Phytoene synthase>

Tetraterpene is terpene having 40 carbon atoms, consisting of eight isoprene units, and mainly includes a compound group called carotenoid. Tetraterpene include many acyclic tetraterpenes or cyclic tetraterpenes. The acyclic tetraterpenes include phytoene, lycopene, and neurosporene. The monocyclic tetraterpene includes γ-carotene. The bicyclic tetraterpene includes α-carotene, β-carotene, astaxanthin, antheraxanthin, canthaxanthin, capsanthin, β-cryptoxanthin, lutein, myxoxanthophyll, zeaxanthin, fucoxanthin, rhodoxanthin, neoxanthin, and flavoxanthin.

Phytoene synthase (PYS) (EC 2.5.1.32) has action of dimerizing geranylgeranyl diphosphate (GGPP). Examples of the PYS include Q7Z859, Q9P854, P37272, Q67GH9, D5KXJ0, P21683, Q9UUQ6, P08196, B2ATB0, Q2U4X9, A2QM49, P37271, P37273, P49085, P54975, P9WHP3, P54977, P22872, and P17056 (UniProtKB entry).

Further introduction of the IDI gene in addition to the PSY gene can enhance the phytoene synthesis ability. Furthermore, introduction of at least one gene selected from the group consisting of a GPP synthase gene, an FPP synthase gene, and a GGPP synthase gene can enhance the synthesis ability of phytoene. Examples of the GPPS, FPPS, and GGPPS are those listed above.

The phytoene synthase used in the present invention may be not only a naturally occurring and isolated phytoene synthase but also a modified product thereof. For example, the phytoene synthase may be proteins that are partial fragments or amino acid substitution variants of existing phytoene synthase and that have phytoene synthase activity.

As described above, the recombinant cell of the present invention includes an isoprene synthase gene, a monoterpene synthase gene, a sesquiterpene synthase gene, a diterpene synthase gene, a squalene synthase gene, or a phytoene synthase gene as a foreign gene, and further optionally includes an IDI gene, a GPPS gene, an NPPS gene, a GGPPS gene, a CPPS gene, an SS gene, and the like.

The above-described foreign genes employed in the present invention may be heterologous genes or homologous genes with respect to the archaeal host cell.

As already described, a membrane lipid component of archaea has an isoprene skeleton. Therefore, by an archaeal mevalonate pathway (MVA pathway) shown in Fig. 1, archaea synthesize a larger amount of IPP that is a unit structure of isoprene or an isoprenoid compound as compared with the other organisms. For producing a much larger amount of isoprene or terpene, a measures for further enhancing the IPP synthesis ability may be applied. For example, for producing a much larger amount of isoprene or terpene without affecting proliferation of the recombinant cell, an MVA pathway gene as the foreign gene may be further introduced. The MVA pathway gene to be introduced may not be particularly limited as long as it normally functions in the recombinant cell. For example, an MVA pathway gene having the same origin as the host (endogenous), an MVA pathway gene derived from the other archaea, an MVA pathway gene derived from organisms other than archaea can be used.

Furthermore, instead of introducing the MVA pathway gene from the outside, a function of an endogenous MVA pathway may be improved. For example, it is possible to increase the expression level of the endogenous MVA pathway gene by promoter modification.

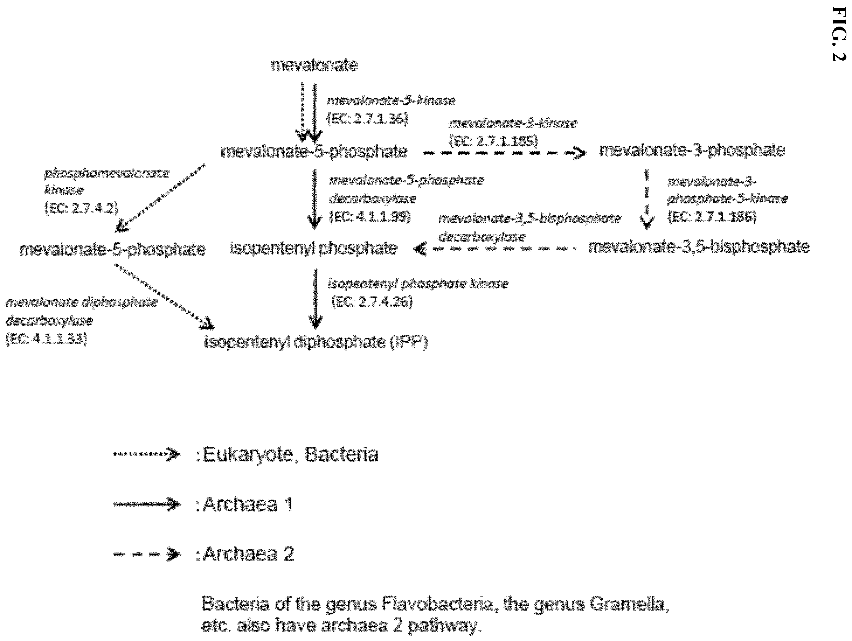

Fig. 1 shows enzymes constituting a representative MVA pathway derived from archaea, but as shown in Fig. 2, in eukaryote and prokaryote, there are various biosynthetic pathways from mevalonate to IPP. The MVA pathway genes to be introduced are genes encoding a part or whole of the enzymes shown in Fig. 1 or 2. Similarly, enzymes of the endogenous MVA pathway whose function is improved are a part or whole of these enzymes.

The enzymes shown in Fig. 1 or 2 are specifically:
acetoacetyl-CoAthiolase (EC:2.3.1.9),
HMG-CoA synthase (EC: 2.3.3.10),
HMG-CoAreductase (EC: 1.1.1.34; 1.1.1.88),
mevalonate-5-kinase (EC: 2.7.1.36),
mevalonate-5-phosphate decarboxylase (EC: 4.1.1.99),
isopentenyl phosphate kinase (EC: 2.7.4.26),
isopentenyl diphosphate isomerase (EC: 5.3.3.2),
phosphomevalonate kinase (EC: 2.7.4.2),
mevalonate diphosphate decarboxylase (EC: 4.1.1.33),
mevalonate-3-kinase (EC: 2.7.1.185),
mevalonate-3-phosphate-5-kinase (EC: 2.7.1.186), and
mevalonate-3,5-bisphosphate decarboxylase.

Among them, one or more enzymes selected from the group consisting of acetoacetyl-CoA thiolase, HMG-CoA synthase, HMG-CoA reductase, mevalonate-5-kinase, mevalonate-5-phosphate decarboxylase, and isopentenyl phosphate kinase are particularly preferable.

Furthermore, origin of the MVA pathway gene to be introduced is not particularly limited. Preferably, the MVA pathway gene is derived from archaea, and further preferably, derived from the genus *Methanosarcina,* the genus *Methanococcus,* the genus *Methanothermococcus,* the genus *Methanothermobacter,* the genus *Methanothrix,* the genus *Thermococcus,* the genus *Thermofilum,* the genus *Thermoplasma,* or the genus *Archaeoglobus.*

The recombinant cell of the present invention belongs to anaerobic archaea, and more specifically, is an anaerobic archaeon into which a foreign gene is introduced.

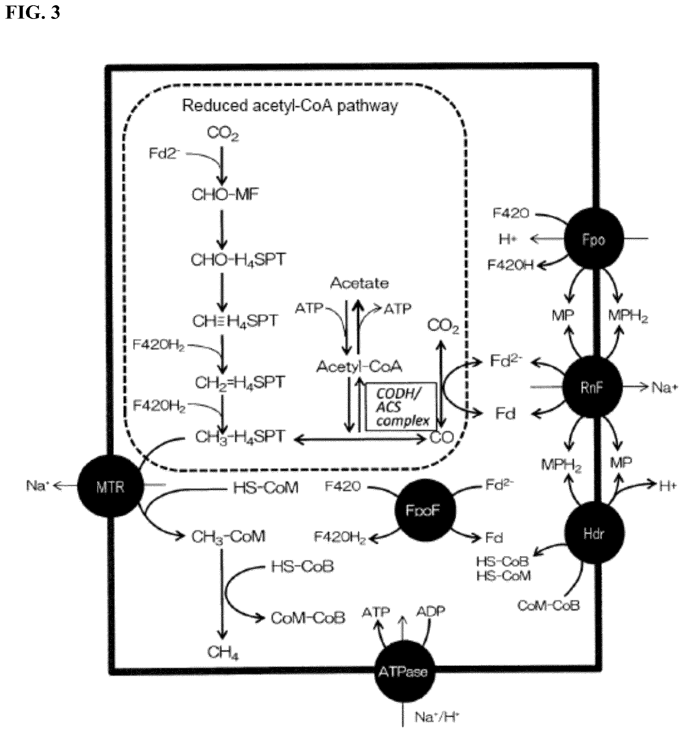

In general, the archaea can proliferate using at least one selected from carbon monoxide, carbon dioxide, methane, methanol, and acetic acid as a sole carbon source. They have a reduced acetyl-CoA pathway as shown in Fig. 3, and their basic metabolism includes synthesizing acetyl-CoA from methyltetrahydropterin, carbon monoxide, and CoA, and generating acetic acid and methane. A CODH/ACS (carbon monoxide dehydrogenase/acetyl-CoA synthase) complex has an important function in anabolism and catabolism of carbon monoxide and carbon dioxide in the reduced acetyl-CoA pathway.

Examples of archaea usable in the present invention include archaea belonging to the genus *Methanobacterium,* the genus *Methanobrevibacter,* the genus *Methanocalculus,* the genus *Methanococcus,* the genus *Methanosarcina,* the genus *Methanosphaera,* the genus *Methanothermobacter,* the genus *Methanothrix,* the genus *Methanoculleus,* the genus *Methanofollis,* the genus *Methanogenium,* the genus *Methanospirillium,* the genus *Methanosaeta,* the genus *Thermococcus,* the genus *Thermofilum,* and the genus *Archaeoglobus.* Among them, the genus Methanosarcina, the genus Methanococcus, the genus *Methanothermococcus,* the genus *Methanothermobacter,* the genus *Methanothrix,* the genus *Thermococcus,* the genus *Thermofilum,* or the genus *Archaeoglobus* are preferable. Furthermore, from the viewpoint of assimilation of carbon monoxide and carbon dioxide, the genus *Methanosarcina,* the genus *Methanococcus,* or the genus *Methanothermococcus* is particularly preferable. Specific examples thereof include *Methanosarcina barkeri, Methanosarcina mazei, Methanosarcina acetivorans, Methanococcus voltae, Methanococcus vannielii, Methanococcus maripaludis,* and *Methanothermococcus (Methanococcus) thermolithotrophicus.*

On the other hand, carbon monoxide dehydrogenase (CO dehydrogenase, CODH) is an enzyme that is important to the carbon monoxide metabolism, and has an activity to generate carbon dioxide and proton from carbon monoxide. This activity belongs to EC 1.2 (Oxidoreductase), and specifically belongs to EC1.2.7.4 (old EC1.2.99.2), and the like. A reaction to generate carbon dioxide and proton from carbon monoxide and water (water-gas shift reaction) is a representative example of this enzyme reaction.

In a preferable embodiment, the recombinant cell includes carbon monoxide dehydrogenase. In other words, preferably, the recombinant cell of the present invention includes the above-described enzymatic activity from the viewpoint of application to syngas fermentation. The carbon monoxide dehydrogenase may be one which a host naturally has or one expressed by a foreign gene. When a host has no or low CODH activity, it is useful to introduce a CODH gene as a foreign gene. In this case, the origin of the CODH includes, for example of bacteria, the genus *Rhodopseudomonas,* the genus *Rhodospirillum,* the genus *Rubrivivax,* the genus *Rhodocyclus,* the genus *Clostridium,* the genus *Peptostreptococcus,* the genus *Acetobacterium,* the genus *Moorella,* and the genus *Carboxydothermus,* which are carbon monoxide assimilation bacteria. Meanwhile, for example of archaea, the origin of the CODH includes the genus *Thermococcus,* the genus *Thermofilum,* the genus *Archeoglobus,* the genus *Methsanosarcina,* the genus *Methanococcus,* the genus *Methanothermococcus,* and the genus *Methanothermobacter.*

Since the recombinant cell of the present invention usually has the reduced acetyl-CoA pathway shown in Fig. 3, it can grow using carbon dioxide as a sole carbon source, and hydrogen as an energy source. Ability to proliferate by assimilating carbon dioxide and hydrogen can be applied to conversion from industrial exhaust gas, such as waste combustion gas or industrial steel and iron exhaust gas, into valuable products.

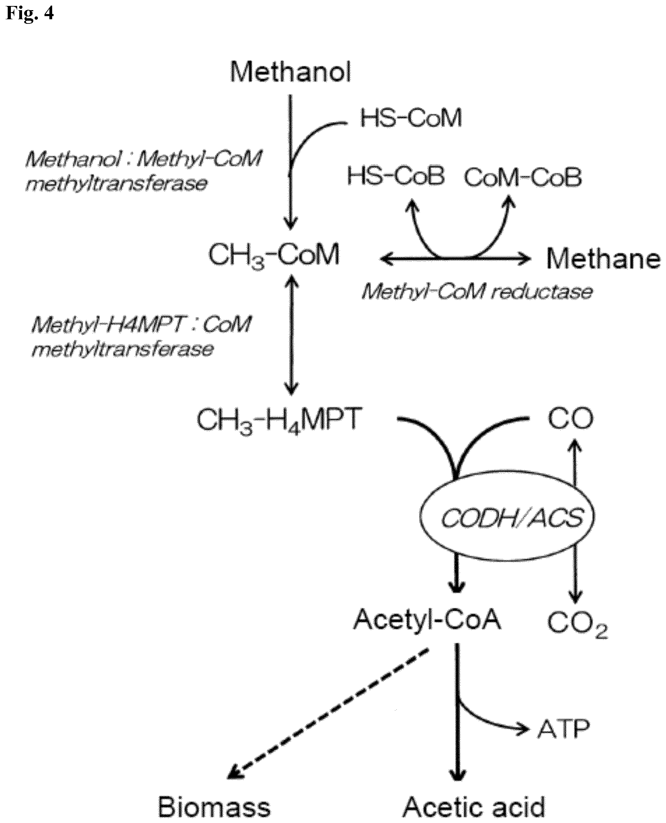

In a preferable embodiment, the recombinant cell can grow using methanol as a sole carbon source. In other words, the recombinant cells of the present invention include recombinant cells including methanol:coenzyme M methyltransferase (EC: 2.1.1.246). Since they generate methyl CoM from methanol and CoM, they can grow in a medium including methanol as a sole carbon source (Fig. 4). Methanol is produced abundantly at a low cost from natural gas, coal, waste combustion gas, and the like. Accordingly, the recombinant cell of the present invention, having methanol assimilating ability, is applicable to conversion from methanol into valuable products.

In a preferable embodiment, the recombinant cell can grow using methane as a sole carbon source. In other words, the recombinant cells of the present invention include recombinant cells including methyl coenzyme M reductase (Methyl-CoM reductase, EC2.8.4.1). Since they generate methyl CoM from methane, they can grow in a medium containing methane as a sole carbon source (Fig. 4). The recombinant cell of the present invention, having methane assimilating ability, is applicable to conversion from natural gas and biomass processed and produced methane into valuable products.

The method of introducing a gene (nucleic acid) into archaea is not particularly limited, and may be appropriately selected depending on the type of archaea as the host cell and the like. For example, it is possible to use a vector that can be introduced into the host cell and can express the introduced gene. As such a vector, it is possible to use a vector that can self-replicate in a host cell or can be introduced into chromosome and that contains a promoter at the position allowing transcription of the incorporated gene. For example, it is preferable to construct a series of constitutions including a promoter, a ribosome binding sequence, the above-described foreign gene(s) and a transcription termination sequence, in the host cell, by using the vector.

A case where the host cell is the genus *Methanosarcina* is described. For example, a shuttle vector with *E. coli* based on a plasmid pC2A that is included in the *Methanosarcina acetivorans* (DSM2834) can be used (Sowers K.R. et al., J. Bacteriol. 1988, 170, 4979-4982; Metcalf W.W. et al., PNAS 1997, 94, 2626-2631). Examples of introduction and deletion of gene by homologous recombination are disclosed (Rother M., et al., J. Bacteriol 2005, 187, 5552-5559; Conway D.M., J. Mol. Biol. 1996, 262, 12-20). These techniques can be used also in the present invention.

When the host cell is the genus *Methanococcus or* the genus *Methanothermococcus,* a shuttle vector can be constructed from a plasmid pURB500, pURB900, and the like, included in *Methanococcus maripaludis, Methanococcus st.* AG83, and the like (Lange M. et al., FEMS Microbiol. 2001, 25, 553-571).

As a promoter for expressing a foreign gene, a constitutive expression promoter can be used. The constitutive expression promoter is not particularly limited. However, since most of archaea have methane formation potential, for example, a Methyl Co-Enzyme M reductase promoter (Pmcr) can be used (Buan N. et al., Methods in Enzymology 2011, 494, 23-42). On the other hand, as the inducible expression system, a technique using a control system of expression of tetracycline-resistant gene derived from Tn10 transposon (Guess A.M. et al., Archaea 2008, 2, 193-203), and the like can be used.

For introducing a plurality of foreign genes into the host by using a vector, the genes may be incorporated into one vector, or incorporated in different vectors. When a plurality of genes is incorporated into one vector, these genes may be expressed under a common promoter, or expressed under different promoters. To express a plurality of enzymes in a form of a fusion protein, a fused gene in which a plurality of genes is linked may be introduced.

The transformation method is not particularly limited, and, for the genus *Methanosarcina*, a liposome method (Metcalf WW. et al., PNAS 1997, 94, 2626-2631) is useful. Furthermore, for the genus *Methanococcus* or the genus *Methanothermococcus*, a PEG method (Gernhardt P. et al., Mol. Gen. Genet. 1990, 221, 273-279) is effective.

In addition to the above-described foreign gene introduction, when mutation or cell fusion is further carried out, a strain in which productivity of isoprene and terpene is especially improved can be bred.

The method for producing isoprene or terpene of the present invention includes: bringing at least one carbon source selected from the group consisting of carbon monoxide, carbon dioxide, methane, methanol, and acetic acid into contact with the above-described recombinant cell; and allowing the recombinant cell to produce isoprene or terpene having 10, 15, 20, 30, or 40 carbon atoms from the carbon source. Typically, the recombinant cell is cultured using at least one carbon source selected from the group consisting of carbon monoxide, carbon dioxide, methane, methanol, and acetic acid, and isoprene or terpene having 10, 15, 20, 30, or 40 carbon atoms is obtained from the cultured product.

Since the recombinant cell of the present invention usually includes a reduced acetyl-CoA pathway, further, a methanol assimilation pathway, a reversible acetic acid synthesis pathway, and reversible methane production pathway, isoprene or terpene can be produced using at least one carbon source selected from the group consisting of carbon monoxide, carbon dioxide, methane, methanol, and acetic acid. The C1 compound or acetic acid may be used singly or in combination of two or more thereof.

When a main carbon source and an energy source are a gas component, culturing under a pressurized condition at about 0.2 to 0.3 MPa (absolute pressure), preferably, enhances the assimilation property of the gas component. Furthermore, for improving initial proliferation and attained cell density, small amounts of organic substances such as vitamins, yeast extract, corn steep liquor, and Bacto Tryptone, may be added.

Isoprene or terpene may be produced without culturing. In other words, isoprene or terpene can be produced by bringing the above-mentioned C1 compound or acetic acid into contact with the recombinant cell regardless of whether or not cell division (cell proliferation) occurs. For example, the above-mentioned C1 compound or acetic acid is continuously supplied to the immobilized recombinant cell, so that the isoprene or terpene can be continuously produced. Also in this aspect, the C1 compound or acetic acid as a carbon source thereof may be used singly or in combination of two or more thereof. Furthermore, it is preferable that hydrogen (H2) as an energy source is brought into contact concurrently.

In a preferable embodiment, the recombinant cell is cultured in a culture tank, a mixed gas including carbon monoxide, carbon dioxide, and hydrogen is supplied to the culture tank. Preferably, a carbon monoxide concentration in the mixed gas is 20% or more. Preferably, a carbon dioxide concentration in the mixed gas is 5% or more. Preferably, a hydrogen sulfide concentration in the mixed gas is 10 ppm or less.

In a case where the recombinant cell of the present invention is cultured using the mixed gas, addition of methanol and/or acetic acid in a culture medium may stabilize culturing. For example, the recombinant cell of the present invention includes cells allowing both chemoautotrophic growth (CO/CO₂/H₂ assimilation) and heterotrophic growth (methanol assimilation). In this case, efficient and stable culture using the following combinations of a carbon source and an energy source can be carried out.
CO/CO₂/H₂/methanol
CO/H₂/methanol
CO/methanol
CO₂/H₂/methanol
CO₂/methanol
CO/CO₂/H₂/acetic acid
CO/H₂/acetic acid
CO/acetic acid
CO₂/H₂/acetic acid
CO₂/acetic acid

The gas components (CO, CO₂, and H₂) and the fluid components (methanol and acetic acid) mentioned above may be always allowed to coexist, or used separately in each stage of culture. For example, cells may be allowed to proliferate with only methanol until a stationary state, and thereafter, may be changed to proliferation with only the gas component, or a combination of the gas components and methanol. When methanol is allowed to coexist in fermentation of the syngas (mixed gas of CO, CO₂, and H₂), syngas assimilation in long-term continuous culture can be stabilized. Reduction of contamination by methanol can be expected.

The present invention is applicable to recycling of waste combustion exhaust gas, steel industry exhaust gas, and the like. In other words, a mixed gas mainly including carbon monoxide (CO), carbon dioxide (CO₂), and hydrogen (H₂) as main carbon and energy component in the above-described exhaust gas is supplied to the recombinant cell. In other words, a recombinant cell is cultured using the mixed gas as a carbon source, or a mixed gas is brought into contact with the recombinant cell to produce isoprene or terpene from carbon monoxide or carbon dioxide in the mixed gas. Also in this case, hydrogen is used as an energy source. The composition of the main component in the above-mentioned industrial exhaust gas is, for example, CO/CO₂/H₂ = 30-50/10-40/25-45% for waste combustion waste gas, and for example, CO/CO₂/H₂ = 50-70/10-40/2-10% for steel industry waste gas.

The above-mentioned composition of gas may be changed corresponding to the gas assimilating property of archaea to be used. For example, carbon monoxide in the above-mentioned mixed gas can be easily converted into carbon dioxide and hydrogen in the presence of water vapor by, for example, a water gas shift reaction so that the concentration of carbon dioxide and hydrogen can be increased.

Furthermore, hydrogen sulfide is present in the industrial waste gas, and, usually, exposure to 1000 ppm or more may dramatically deteriorate proliferation of archaea. Therefore, before the waste gas is supplied to the culture tank, the hydrogen sulfide concentration is reduced preferably to 100 ppm or less, and further preferably 10 ppm or less, by a wet limestone-gypsum process, a hydrodesulfurization process, and the like.

In a preferable embodiment, a cell concentration in the culture solution in the proliferation stationary phase is 1 g dry cells/liter or more, and supplying of a culture medium and discharging of a culture solution are continuously carried out. In other words, the culture method is continuous culture method and cell density is kept high.

The continuous culture means that after the cell density reaches a certain level, supply of a fresh culture medium and gas, and discharge of a culture solution (including a cell) are carried out continuously so as to keep the cell density and the production rate of a target constant, and to carry out culture over a long period of time (for example, one month or more). Thus, as compared with batch production, productivity of isoprene or terpene can be increased especially, and furthermore, isoprene or terpene can be produced at a lower cost. The cell density in the above-mentioned continuous culture is usually 1 g dry cells/liter or more, preferably, 5 g dry cells/liter, and further preferably, 10 g dry cells/liter.

The produced isoprene or terpene can be collected from the outside of the cells, that is, from a culture solution or a vapor phase fraction.

In the following, the present invention will be described more specifically by way of Examples. However, the present invention is not limited to these Examples.

### EXAMPLES

### [Example 1]

### Preparation of isoprene synthesis gene introduction vector for producing isoprene in archaea

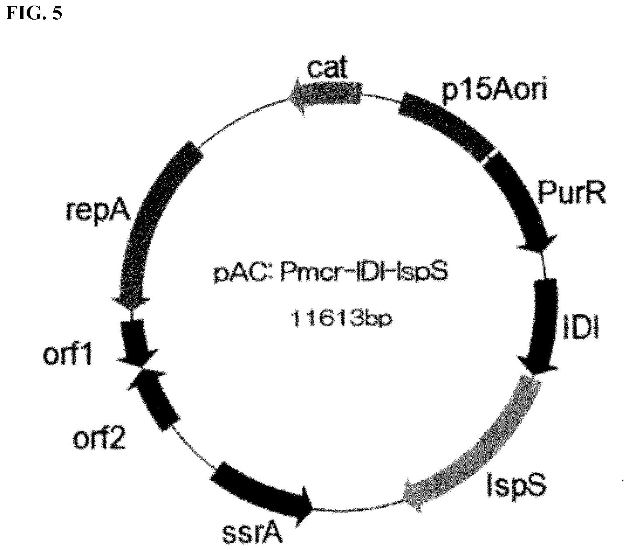

In this Example, a vector pAC:Pmcr-IDI-IspS for synthesizing isoprene in archaea shown in Fig. 5 was prepared. This vector is a shuttle vector having a replication region of an endogenous plasmid in *Methanosarcina acetivorans*, and a replication region p15A in *E. coli,* and can constitutively express an IDI (isopentenyl diphosphate isomerase) gene derived from *Saccharomyces cerevisiae*, and an IspS (isoprene synthase) gene derived from *Poplus canescens* by a promoter PmcrB (methyl coenzyme M reductase) derived from *Methanococcus voltae.*

A plasmid pACYC184 (manufactured by NIPPON GENE CO., LTD.) was cleaved by HindIII and BclI to obtain fragments including a chloramphenicol-resistant gene (Cat) and replication region pl5A, and a linker sequences consisting of SEQ ID NO: 5 and SEQ ID NO: 6 are introduced so as to obtain plasmid pAMCS. A puromycin-resistant gene (PuR, manufactured by Genewiz) of SEQ ID NO: 7 was introduced into a SphI-AscI cleavage site of pAMCS to construct pAY:PuR. A gene fragment including an IDI-IspS gene of SEQ ID NO: 8 (manufactured by Genewiz) was introduced into a PacI-NotI cleavage site of this plasmid vector to construct a plasmid pAY:Pmcr-IDI-IspS.

On the other hand, an endogenous plasmid pC2A (GenBank No. U78295) was prepared from cultured cells of *M. acetivorans* (DSM2834) according to the method by Sowers KR et al. (1988 J. Bacterial., 170 (10), 4979-4982). pC2A linearized by SpeI and pAY:Pmcr-IDI-IspS linearized by SpeI were fused to construct a shuttle vector pAC:Pmcr-IDI-IspS (SEQ ID NO: 9, Fig. 5) for constitutively expressing the genes for isoprene synthesis in archaea.

pAY:PuR and pC2A were fused by the similar procedure so as to prepare a shuttle vector pAC:PuR (SEQ ID NO: 10) for control.

### [Example 2]

### Transformation of Methanosarcina barkeri, and production of isoprene from various carbon sources

Single cell-forming culture of *Methanosarcina barkeri* (DSM800) and preparation of a competent cell were carried out according to the method of Metcalf WW et al. (PNAS 1997, 94, 1997). In other words, an ATCC2825 modified culture medium (10 g of magnesium chloride hexahydrate, 0.76 g of potassium chloride, and 24 g of sodium chloride per 1 L) (pH6.8) was cultured under strict anaerobic conditions until the logarithmic growth phase (OD600 = 0.3-0.6). Cells were collected in an anaerobic chamber (manufactured by Coy) by centrifugation. The collected cells were suspended in 0.85 M saccharose aqueous solution to obtain a competent cell suspension (about 10⁹ cells/mL).

Fifteen microliters (µL) of DOTAP liposome (manufactured by Boehringer Mannheim), 100 µL of 20 mM HEPES-KOH (pH7.4), and 2 µg of pAC:Pmcr-IDI-IspS (in 50 µL of HEPES-KOH (pH7.4)) were mixed, and the mixture was incubated for 15 minutes. One microliter (mL) of the competent cell suspension was added thereto, and the mixture was further incubated for four hours. This mixture solution (1.165 mL) was recovery cultured in 10 mL of the ATCC2825 modified culture medium at 35°C for 12 to 16 hours, and then the cells were collected. The collected cells were suspended in 2 mL of ATCC2825 modified culture medium (45°C) including 0.5% agarose, and the suspension was seeded in ATCC2825 modification agar culture medium including 1.4% agarose and puromycin (2 µg/mL). Culture in the agar culture medium was carried out in an anaerobic jar, in which a vapor phase had been substituted with a mixed gas having N₂/CO₂/H₂S = 80/20/0.1% at 35°C for 10 to 14 days.

Growing colony was cultured at 35°C for three days using 200 µL of ATCC2825 modified culture medium (wherein 500 mg of yeast extract, 500 mg of Casitone, and 5 g of sodium chloride per liter were included, and methanol was not included) and a predetermined carbon source. Cells in a whole culture solution were subjected to shaking culture at 35°C for 7 to 10 days under five types of conditions shown in Table 1. In experiments 1 to 4, various gases were filled at a pressure of 2.5 atmospheric pressure (absolute pressure). In experiments 5 and 6, N₂ was used as the vapor phase, and gases were filled at a pressure of 1.5 atmospheric pressure (absolute pressure). Culture was carried out in a 20 mL-vial for HS-20 sealed with a butyl rubber plug (vapor phase: 15 mL, and culture solution: 5 mL). In each experiment, three clones each was cultured (N = 3). After the culture was completed, a vapor phase was subjected to a head-space GCMS analysis (GCMS-QP2010 Ultra / HS-20, manufactured by SHIMADZU CORPORATION), and the isoprene concentration was measured.

The similar operation was carried out using pAC:PuR instead of pAC:Pmcr-IDI-IspS as a control.

The results are shown in Table 1. When pAC:Pmcr-IDI-IspS (Fig. 5) was introduced, in any case using any carbon sources of CO₂/H₂ (Experiment 1), CO/CO₂/H₂ (Experiment 2), and CO/H₂/N₂ (Experiment 3), significant isoprene production was observed. Furthermore, also when methanol (Experiment 4) and acetic acid (Experiment 5) were used as a carbon source, significant isoprene production was observed. A production amount of isoprene exceeded 1 mg/g dry cell in all of these cases. On the other hand, when a main carbon source is not included (Experiment 6), only a small amount of isoprene was produced. Note here that when pAC:PuR was introduced, isoprene was hardly detected.

From the above description, production of isoprene derived from all of carbons, i.e., CO, CO₂, methanol, and acetic acid by recombinant archaea was observed.

It was shown that the production amount of isoprene (more than 1 mg per 1 g dry cell) was clearly higher than a previously reported production amount of isoprene by a syngas assimilating *Clostridium* bacterium (see, for example, Patent Documents 1 and 3), and that isoprene production in archaea was efficient. Furthermore, it was shown that since isoprene was produced using CO/CO₂/H₂, and CO₂/H₂ as a carbon source, syngas such as waste combustion gas was able to be industrially used as a carbon source.

Furthermore, in conventional techniques using bacteria as a host, including the investigation example in the genus *Clostridium* descried above, it had been necessary to introduce an MVA pathway gene constructed by six or more types of foreign enzyme genes. That is, also from the viewpoint of easiness in gene manipulation, an endogenous MVA pathway gene of archaea can be used. In other words, it was shown that the present invention, which does not require a foreign MVA pathway gene, was effective.

As described above, it was also shown that the present invention was applicable to production of isoprene from waste combustion gas, methanol, acetic acid, and the like, being manufactured at a low cost and abundantly.

### [Table 1]

**TABLE 1**

| Experiment | Carbon source | Isoprene production (mg/g dry cell) | |
|---|---|---|---|
| | | pAC:Pmcr-IDI-lspS | pAC:PuR |
| 1 | CO₂/H₂ = 20/80 | 9.8 | < 0.01 |
| 2 | CO/CO₂/H₂=15/30/55 | 6.8 | < 0.01 |
| 3 | CO/H₂/N₂= 15/50/35 | 4.3 | < 0.01 |
| 4 | 250mM CH₃OH | 10.8 | < 0.01 |
| 5 | 15mM CH₃COONa | 8.6 | < 0.01 |
| 6 | None | < 0.1 | < 0.01 |

### [Example 3]

### Preparation of vector for production of farnesene in archaea

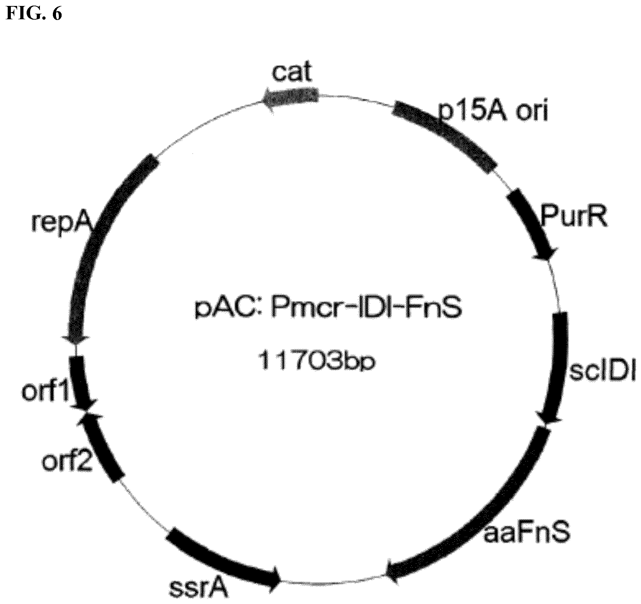

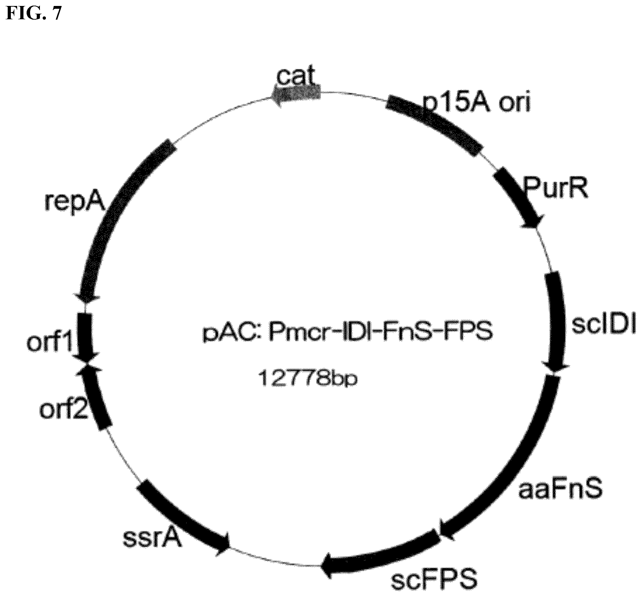

In this Example, vectors pAC:Pmcr-IDI-FnS (Fig. 6) and pAC:Pmcr-IDI-FnS-FPS (Fig. 7) for synthesizing farnesene in archaea were prepared. The pAC:Pmcr-IDI-FnS contains IDI derived from *Saccharomyces cerevisiae* and Farnesene synthase (FnS) gene derived from *Artemisia annua.* The pAC:Pmcr-IDI-FnS-FPS contains the above-mentioned IDI gene, farnesyl diphosphate synthase (FPS) gene derived from *Saccharomycess cerevisiae*, and the above-mentioned FnS gene.

The pAC:Pmcr-IDI-IspS (Fig. 5) prepared in Example 1 was cleaved by MluI-NotI to delete an IDI-IspS region, and a linear fragment was obtained. This linear fragment and a gene fragment containing IDI-FnS (SEQ ID NO: 11) were fused to construct pAC:Pmcr-IDI-FnS (SEQ ID NO: 13, Fig. 6). Furthermore, this linear fragment and a gene fragment containing IDI-FnS-FPS gene (SEQ ID NO: 12) (manufactured by Genewiz) were fused to construct pAC:Pmcr-IDI-FnS-FPS (SEQ ID NO: 14, Fig. 7). In Figs. 6 and 7, scIDI represents IDI derived from *Saccharomyces cerevisiae,* aaFnS represents FnS derived from *Artemisia annua*, and scFPS represents farnesyl diphosphate synthase derived from *Saccharomycess cerevisiae.*

### [Example 4]

### Production of farnesene from various carbon sources by recombinant Methanosarcina barkeri

By transformation method by liposome method similar to Example 2, pAC:Pmcr-IDI-FnS or pAC:Pmcr-IDI-FnS-FPS were introduced into *Methanosarcina barkeri* (DSM800) to obtain transformants for each. Subsequently, these transformants were cultured in the same conditions as in Example 2. However, for extracting and absorbing farnesene to be produced, 10% (v/v) dodecane was overlaid on a culture solution. After culture was completed, a dodecane layer was collected from each culture solution by centrifugation. The collected dodecane layer was 10-folded diluted with n-hexane, farnesene concentration was measured by GCMS (GCMS-QP2010 Ultra, manufactured by SHIMADZU CORPORATION) analysis.

The similar operation was carried out as a control using pAC:PuR (Example 1) instead of pAC:Pmcr-IDI-FnS or pAC:Pmcr-IDI-FnS-FPS.

The results are shown in Table 2. When pAC:Pmcr-IDI-FnS (Fig. 6) or pAC:Pmcr-IDI-FnS-FPS (Fig. 7) was introduced, in any case using any carbon/energy source of CO₂/H₂ (Experiment 7), CO/CO₂/H₂ (Experiment 8), and CO/H₂/N₂ (Experiment 9), significant farnesene production was observed. Furthermore, also when methanol (Experiment 10) or acetic acid (Experiment 11) was used as a carbon source, significant farnesene production was observed. These production amounts of the farnesene exceeded 1 mg/g dry cell.

On the other hand, even when pAC:Pmcr-IDI-FnS (Fig. 6) or pAC:Pmcr-IDI-FnS-FPS (Fig. 7) was introduced, if a main carbon source was not present (Experiment 12), only a slight amount of farnesene was produced. It was shown that since farnesene was able to be produced using CO/CO₂/H₂, CO₂/H₂, and CO/H₂ as a carbon source, the present invention was applicable to production of farnesene from syngas such as waste combustion gas.

Note here that when pAC:PuR was introduced, in all cases, farnesene was hardly detected.

As described above, it was also shown that the present invention was applicable to production of farnesene from waste combustion gas, methanol, acetic acid, and the like, being manufactured at a low cost and abundantly.

### [Table 2]

**TABLE 2**

| Experiment | Carbon source | Farnesene production (mg/g dry cell) | | |
|---|---|---|---|---|
| | | pAC:Pmcr-IDI-FnS | pAC:Pmcr-IDI-FnS-FPS | pAC:PuR |
| 7 | CO₂/H₂ = 20/80 | 7.8 | 9.9 | < 0.01 |
| 8 | CO/CO₂/H₂=15/30/55 | 5.8 | 6.6 | < 0.01 |
| 9 | CO/H₂/N₂= 15/50/35 | 3.3 | 5.1 | < 0.01 |
| 10 | 250mM CH₃OH | 7.7 | 10.4 | < 0.01 |
| 11 | 15mM CH₃COONa | 5.6 | 6.7 | < 0.01 |
| 12 | None | < 0.1 | < 0.1 | < 0.01 |

### [Example 5]

### Influence on foreign isopentenyl diphosphate isomerase (IDI) gene in production of isoprene by recombinant Methanosarcina barkeri

A vector pAC:Pmcr-IspS (SEQ ID NO: 15) was prepared. This vector corresponds to one in which an IDI gene is deleted from pAC:Pmcr-IDI-IspS (Fig. 5).

pAC:Pmcr-IspS or pAC:Pmcr-IDI-IspS was introduced into *Methanosarcina barkeri* (DSM800) by the same method as in Example 2 to obtain each transformant. Each transformant was cultured in the same conditions as in Example 2, and the concentration of isoprene in the vapor phase was measured. CO₂/H₂ (Experiment 13) or methanol (Experiment 14) was used as a carbon source for culture.

The results are shown in Table 3. With any carbon sources, the production amount of isoprene was higher when the IDI gene was introduced. This showed that when the IDI was allowed to act concurrently, the production amount of isoprene was increased.

### [Table 3]

**TABLE 3**

| Experiment | Carbon source | Isoprene production (mg/g dry cell) | |
|---|---|---|---|
| | | pAC:Pmcr-IDI-lspS | pAC:Pmcr-lspS |
| 13 | CO₂/H₂ = 20/80 | 10.2 | 6.4 |
| 14 | 250mM CH₃OH | 13.4 | 8.3 |
| 15 | None | < 0.1 | < 0.1 |

### [Example 6]

### Enhancement of isoprene production by introduction of MVA pathway enzyme gene

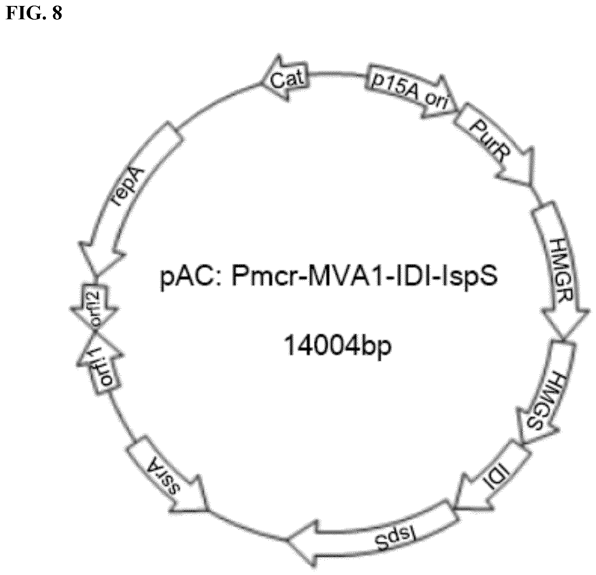

A vector pAC:Pmcr-MVA1-IDI-IspS (SEQ ID NO: 16, Fig. 8) was prepared. This vector further includes HMG-CoA synthase (KEGG entry: MA_4041) gene derived from *Methanosarcina acetivotans* and an HMG-CoA reductase (MA_3073) gene in addition to an IDI gene derived from *Saccharomyces cerevisiae* (Example 1) and an IspS gene derived from *Poplus canescens* (Example 1).

pAC:Pmcr-MVA1-IDI-IspS or pAC:Pmcr-IDI-IspS (Example 1) was introduced into *Methanosarcina barkeri* (DSM800) by the same method as in Example 2 to obtain each transformant. Each transformant was cultured in the same conditions as in Example 2, and the concentration of isoprene in the vapor phase was measured. CO₂/H₂ (Experiment 16) or methanol (Experiment 17) was used as a carbon source for culturing.

The results are shown in Table 4. With any carbon sources, the production amount of isoprene was higher in the transformant in which pAC:Pmcr-MVA1-IDI-IspS had been introduced. This showed that only reinforcing a part of enzymes constructing an MVA pathway increased the production amount of isoprene.

### [Table 4]

**TABLE 4**

| Experiment | Carbon source | Isoprene production (mg/g dry cell) | |
|---|---|---|---|
| | | pAC:Pmcr-MVA1-IDI-IspS | pAC:Pmcr-IDI-IspS |
| 16 | CO₂/H₂ = 20/80 | 13.2 | 9.9 |
| 17 | 250mM CH₃OH | 17.3 | 12.8 |
| 18 | None | < 0.1 | < 0.1 |

## Claims

1. A recombinant cell being an anaerobic archaeon, comprising: a gene encoding isoprene synthase, a gene encoding monoterpene synthase, a gene encoding sesquiterpene synthase, a gene encoding diterpene synthase, a gene encoding squalene synthase, or a gene encoding phytoene synthase as a first foreign gene,
wherein the first foreign gene is expressed, and the recombinant cell produces isoprene or terpene having 10, 15, 20, 30, or 40 carbon atoms.

2. The recombinant cell according to claim 1, growing using at least one selected from the group consisting of carbon monoxide, carbon dioxide, methane, methanol, and acetic acid as a sole carbon source.

3. The recombinant cell according to claim 1 or 2, comprising carbon monoxide dehydrogenase.

4. The recombinant cell according to any one of claims 1 to 3, having a function of synthesizing acetyl-CoA from methyltetrahydropterin, carbon monoxide, and CoA.

5. The recombinant cell according to any one of claims 1 to 4, having a methane formation potential.

6. The recombinant cell according to any one of claims 1 to 5, growing using carbon dioxide as a sole carbon source and hydrogen as an energy source.

7. The recombinant cell according to any one of claims 1 to 6, growing using methanol as a sole carbon source.

8. The recombinant cell according to any one of claims 1 to 7, growing using methane as a sole carbon source.

9. The recombinant cell according to any one of claims 1 to 8, belonging to the genus *Methanosarcina,* the genus *Methanococcus,* the genus *Methanothermococcus,* the genus *Methanothermobacter,* the genus *Methanothrix,* the genus *Thermococcus,* the genus *Thermofilum,* or the genus *Archaeoglobus.*

10. The recombinant cell according to any one of claims 1 to 8, belonging to the genus *Methanosarcina,* the genus *Methanococcus,* or the genus *Methanothermococcus.*

11. The recombinant cell according to claim 10, being *Methanosarcina barkeri, Methanosarcina mazei, Methanosarcina acetivorans, Methanococcus voltae, Methanococcus vannielii, Methanococcus maripaludis,* or *Methanothermococcus (Methanococcus) thermolithotrophicus.*

12. The recombinant cell according to any one of claims 1 to 11, further comprising a gene encoding carbon monoxide dehydrogenase as a second foreign gene.

13. The recombinant cell according to any one of claims 1 to 12, wherein the first foreign gene is a gene encoding isoprene synthase, the recombinant cell produces isoprene, and the isoprene synthase is derived from a plant.

14. The recombinant cell according to claim 13, wherein the isoprene synthase is a protein of the following (a-1), (a-2), or (a-3):
(a-1) a protein consisting of an amino acid sequence of SEQ ID NO: 1,
(a-2) a protein consisting of an amino acid sequence in which 1 to 20 amino acids are deleted, substituted, or added in the amino acid sequence of SEQ ID NO: 1, and having isoprene synthase activity, and
(a-3) a protein consisting of an amino acid sequence having identity of 90% or more with the amino acid sequence of SEQ ID NO: 1, and having isoprene synthase activity.

15. The recombinant cell according to claim 13 or 14, further comprising a gene encoding isopentenyl diphosphate isomerase as a third foreign gene.

16. The recombinant cell according to any one of claims 1 to 12, wherein the first foreign gene is a gene encoding sesquiterpene synthase, the recombinant cell produces terpene having 15 carbon atoms, and the sesquiterpene synthase is farnesene synthase.

17. The recombinant cell according to any one of claims 1 to 12, wherein the first foreign gene is a gene encoding sesquiterpene synthase, the recombinant cell produces terpene having 15 carbon atoms, and the sesquiterpene synthase is cyclic sesquiterpene synthase.

18. The recombinant cell according to claim 16, wherein the farnesene synthase is a protein of the following (d-1), (d-2), or (d-3):
(d-1) a protein consisting of an amino acid sequence of SEQ ID NO: 4,
(d-2) a protein consisting of an amino acid sequence in which 1 to 20 amino acids are deleted, substituted, or added in the amino acid sequence of SEQ ID NO: 4, and having farnesene synthase activity, and
(d-3) a protein consisting of an amino acid sequence having identity of 90% or more with the amino acid sequence of SEQ ID NO: 4, and having farnesene synthase activity.

19. The recombinant cell according to any one of claims 16 to 18, further comprising a gene encoding isopentenyl diphosphate isomerase as a fourth foreign gene.

20. The recombinant cell according to any one of claims 16 to 19, further comprising at least one gene selected from the group consisting of a gene encoding geranyl diphosphate synthase and a gene encoding farnesyl diphosphate synthase as a fifth foreign gene.

21. The recombinant cell according to any one of claims 1 to 12, wherein the first foreign gene is a gene encoding monoterpene synthase, the recombinant cell produces terpene having 10 carbon atoms, and the monoterpene synthase is cyclic monoterpene synthase.

22. The recombinant cell according to claim 21, further comprising a gene encoding isopentenyl diphosphate isomerase as a sixth foreign gene.

23. The recombinant cell according to claim 21 or 22, further comprising a gene encoding geranyl diphosphate synthase as a seventh foreign gene.

24. The recombinant cell according to claim 21 or 22, further comprising a gene encoding neryl diphosphate synthase as an eighth foreign gene.

25. The recombinant cell according to any one of claims 21 to 24, wherein the cyclic monoterpene synthase is phellandrene synthase.

26. The recombinant cell according to claim 25, wherein the phellandrene synthase is a protein of the following (b-1), (b-2), or (b-3):
(b-1) a protein consisting of an amino acid sequence of SEQ ID NO: 2;
(b-2) a protein consisting of an amino acid sequence in which 1 to 20 amino acids are deleted, substituted, or added in the amino acid sequence of SEQ ID NO: 2, and having α-phellandrene synthase activity; and
(b-3) a protein consisting of an amino acid sequence having identity of 90% or more with the amino acid sequence of SEQ ID NO: 2, and having α-phellandrene synthase activity.

27. The recombinant cell according to claim 25, wherein the phellandrene synthase is protein of the following (c-1), (c-2), or (c-3):
(c-1) a protein consisting of an amino acid sequence of SEQ ID NO: 3;
(c-2) a protein consisting of an amino acid sequence in which 1 to 20 amino acids are deleted, substituted, or added in the amino acid sequence of SEQ ID NO: 3, and having β-phellandrene synthase activity; and
(c-3) a protein consisting of an amino acid sequence having identity of 90% or more with the amino acid sequence of SEQ ID NO: 3, and having β-phellandrene synthase activity.

28. The recombinant cell according to any one of claims 1 to 12, wherein the first foreign gene is a gene encoding diterpene synthase, the recombinant cell produces terpene having 20 carbon atoms, and the diterpene synthase is cyclic diterpene synthase.

29. The recombinant cell according to claim 28, further comprising a gene encoding copalyl diphosphate synthase as a ninth foreign gene.

30. The recombinant cell according to claim 28 or 29, further comprising a gene encoding isopentenyl diphosphate isomerase as a tenth foreign gene.

31. The recombinant cell according to any one of claims 28 to 30, further comprising at least one gene selected from the group consisting of a gene encoding geranyl diphosphate synthase, a gene encoding farnesyl diphosphate synthase, and a gene encoding geranylgeranyl diphosphate synthase as an eleventh foreign gene.

32. The recombinant cell according to any one of claims 1 to 12,
wherein the first foreign gene is a gene encoding squalene synthase, the recombinant cell produces terpene having 30 carbon atoms,
the recombinant cell further comprising a gene encoding isopentenyl diphosphate isomerase as a twelfth foreign gene.

33. The recombinant cell according to claim 32, further comprising at least one gene selected from the group consisting of a gene encoding geranyl diphosphate synthase and a gene encoding farnesyl diphosphate synthase as a thirteenth foreign gene.

34. The recombinant cell according to any one of claims 1 to 12,
wherein the first foreign gene is a gene encoding phytoene synthase, and the recombinant cell produces terpene having 40 carbon atoms,
the recombinant cell further comprising a gene encoding isopentenyl diphosphate isomerase as a fourteenth foreign gene.

35. The recombinant cell according to claim 34, further comprising at least one gene selected from the group consisting of a gene encoding geranyl diphosphate synthase, a gene encoding farnesyl diphosphate synthase, and geranylgeranyl diphosphate synthase as a fifteenth foreign gene.

36. The recombinant cell according to any one of claims 1 to 35, further comprising at least one gene selected from the group consisting of acetoacetyl-CoA thiolase, HMG-CoA synthase, HMG-CoA reductase, mevalonate-5-kinase, mevalonate-5-phosphate decarboxylase, and isopentenyl phosphate kinase as a sixteenth foreign gene.

37. The recombinant cell according to any one of claims 1 to 36, wherein the first foreign gene is expressed under a constitutive expression promoter.

38. A method for producing isoprene or terpene, the method comprising bringing at least one carbon source selected from the group consisting of carbon monoxide, carbon dioxide, methane, methanol, and acetic acid into contact with the recombinant cell according to any one of claims 1 to 37, thereby allowing the recombinant cell to produce isoprene or terpene having 10, 15, 20, 30, or 40 carbon atoms from the carbon source.

39. The method according to claim 38, the method comprising culturing the recombinant cell using at least one carbon source selected from the group consisting of carbon monoxide, carbon dioxide, methane, methanol, and acetic acid, and obtaining isoprene or terpene having 10, 15, 20, 30, or 40 carbon atoms from the cultured product.

40. The method according to claim 38 or 39,
wherein a cell concentration of a culture solution of the recombinant cell in a proliferation stationary phase is 1 g or more of dry cells per liter,
the method comprises continuously supplying a culture medium and discharging a culture solution.

41. The method according to any one of claims 38 to 40, wherein the recombinant cell is cultured in a culture tank, and a mixed gas consisting of carbon monoxide, carbon dioxide, and hydrogen is supplied to the culture tank.

42. The method according to claim 41, wherein a carbon monoxide concentration in the mixed gas is 20% or more.

43. The method according to claim 41 or 42, wherein a carbon dioxide concentration in the mixed gas is 5% or more.

44. The method according to any one of claims 41 to 43, wherein a hydrogen sulfide concentration in the mixed gas is 10 ppm or less.

45. The method according to any one of claims 41 to 44, the method further comprising adding methanol and/or acetic acid in a culture solution.
